# EUROPEAN PATENT APPLICATION

(11) **EP 4 669 085 A1**
(43) Date of publication of application: **24.12.2025**
(21) Application number: 23926818.8
(22) Date of filing: 16.03.2023
(51) Int. Cl.: H10K 85/50, H10K 30/50, C07C 211/00

(54) **PEROVSKITE PRECURSOR SOLUTION, PEROVSKITE THIN FILM, PEROVSKITE CELL, AND ELECTRIC DEVICE**

(71) Applicant: Contemporary Amperex Technology (Hong Kong) Limited, Hong Kong (HK)
(72) Inventor: LIN, Xiangling, Ningde, Fujian 352100 (CN); SU, Shuojian, Ningde, Fujian 352100 (CN); LI, Hanfang, Ningde, Fujian 352100 (CN); LIANG, Weifeng, Ningde, Fujian 352100 (CN); LIU, Zhaohui, Ningde, Fujian 352100 (CN); ZHANG, Fan, Ningde, Fujian 352100 (CN); CHEN, Changsong, Ningde, Fujian 352100 (CN); CHEN, Guodong, Ningde, Fujian 352100 (CN); GUO, Yongsheng, Ningde, Fujian 352100 (CN)
(74) Representative: Gong, Jinping
(86) International application number: PCT/CN2023/081962
(87) International publication number: WO 2024/187465

(57) **Abstract**

Provided is a perovskite precursor solution, a perovskite thin film, a perovskite cell, and an electrical device. The perovskite precursor solution comprises a perovskite precursor material, a solvent, and an additive; the additive contains, in its structure, a weakly reducing group which has reducibility on a 0 valence corresponding to a monovalent anion in the perovskite-type metal halide in the solution and is inert to a divalent cation in the perovskite-type metal halide; and the additive is a hydrogen halide salt.

## Description

### TECHNICAL FIELD

The present application relates to the technical field of solar cells, in particular, to a perovskite precursor solution, a perovskite thin film, a perovskite cell, and an electrical device.

### BACKGROUND

The statement here merely provides the background information related to the present application and do not necessarily constitute the prior art.

A perovskite solar cell, which is a device converting solar energy into electric energy using a photoelectric conversion mechanism of a perovskite-type crystal material and is a current third generation solar cell, has various advantages such as high photoelectric conversion efficiency, a simple manufacturing process, a low production cost, etc., and the perovskite solar cell is intensively studied recently. However, its commercial large-scale application is still limited due to technical problems, and one of the problems which needs to be solved is how to prolong the service life of the perovskite cell.

### SUMMARY

Given the above problem, the present application provides a perovskite precursor solution, a perovskite thin film, a perovskite cell, and an electrical device, and the perovskite precursor solution has a good stability, the prepared perovskite thin film has few defects, and the perovskite cell can be improved for the service life.

In a first aspect, the present application provides a perovskite precursor solution comprising a perovskite precursor material, a solvent, and an additive, wherein the perovskite precursor material comprises a perovskite-type metal halide; the additive contains, in its structure, a weakly reducing group which has reducibility on a 0 valence corresponding to a monovalent anion in the perovskite-type metal halide and is inert to a divalent cation in the perovskite-type metal halide; and the additive is a hydrogen halide salt.

By adding, to the perovskite precursor solution, an additive (noted as a first additive), a hydrogen halide salt of an organic compound, containing a weakly reducing group, for metal halide moiety BX₂ in main component ABX₃ in the perovskite precursor solution and the perovskite thin film, first, the weakly reducing group can increase storage time of the perovskite precursor solution by re-reducing X₂ (X has a valence of 0) to X⁻, that is, the weakly reducing group has reducibility on a 0 valence corresponding to a monovalent anion in the perovskite-type metal halide; second, the weak reducibility of the weakly reducing group does not lead to reduce B²⁺ to B⁰, that is, the weakly reducing group is inert to a divalent cation in the perovskite-type metal halide; third, the additive can participate in crystallization reaction and is uniformly dispersed in the perovskite thin film and can act as a sacrificial agent, so that a service life of a perovskite device can be prolonged; and fourth, formation of B⁰, and X₂ can be suppressed to reduce defects at an interface to increase performance of the perovskite device; and the additive can be better dissolved in the perovskite precursor solution and distributed more uniformly in the perovskite thin film by forming a hydrogen halide salt. Here, the perovskite precursor solution is generally a colloidal solution, BX₂ in the solution is often present in a state of being complexed with a solvent and distributed in a system, and the weak reducing group related in the present application easily forms a hydrogen bond with the perovskite colloidal solution and can be uniformly distributed in the colloidal solution; and when crystallization reaction occurs, such an additive may enable a stronger surface tension of a lower surface (a coating surface) when the solvent is quenched, and may form a crystal nucleus site, so that perovskite spreads at such a site to promote crystallization. Further, for perovskite ABX₃, taking FAPbI₃ as an example, due to susceptibility to oxygen, moisture, light, etc., a following equilibrium formula exists: FAPbI₃ 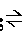 FAI·pBI₂ 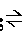 FAI + PbI₂ 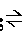 FAI + Pb⁰+ I₂, where I₂ easily sublimates to escape from the system, shifting the whole equilibrium towards the right side of the equilibrium formula to cause degradation of perovskite; when the system has weak reducibility, I₂ having a 0 valence can be reduced to I⁻, so that the whole reaction shifts towards the right, so as to inhibit degradation of perovskite, and the weak reducing group itself does not react with Pb²⁺(exhibiting that the group is inert to Pb²⁺); and after the weakly reducing group is consumed, a corresponding product does not convert to its starting material, and thus the weakly reducing group is a consumable group, and is a sacrificial agent.

In some embodiments, the weakly reducing group comprises one or more of the group of: -NHNH-, -S-S-, -NHNH₂, sulfino, phosphinico, hydroxyphenyl, and hydroxynaphthyl; and
optionally, the weakly reducing group is selected from the group of: -NHNH-, -S-S-, -NHNH₂, sulfino, phosphinico, hydroxyphenyl, and hydroxynaphthyl.

In some embodiments, in one molecule of the first additive, the number of the weakly reducing groups is 1 or more;
optionally, in one molecule of the additive, the number of the weak reducing group is an integer selected from 1~10; further optionally, the number of the weak reducing group is 1, 2, 3, 4 or 5; further optionally, the number of the weak reducing group is 1, 2 or 3; and further optionally, the number of the weak reducing group is 1 or 2; and
optionally, in one molecule of the additive, the type of the weak reducing group is one or more; and further optionally, the type of the weak reducing group is 1, 2, 3 or more.

In some embodiments, in one molecule of the first additive, the number of halogen acid molecules is 1 or more;
optionally, in one molecule of the additive, the number of halogen acid molecules is an integer selected from 1~10; further optionally, the number of halogen acid molecules is 1, 2, 3, 4, or 5; and even further optionally, the number of halogen acid molecules is 1 or 2.

In some embodiments, the halogen in the hydrogen halide salt includes one or more of F, Cl, Br, and I;
optionally, any one halogen in the hydrogen halide salt is independently F, Cl, Br or I; and
further optionally, the hydrogen halide salt is a hydrochloride salt.

In some embodiments, the perovskite precursor solution satisfies one or more of the following features:
the molecular weight of the additive is less than 1000 Da; optionally, the molecular weight of the additive is less than or equal to 500 Da; further optionally, the molecular weight of the additive is less than or equal to 400 Da; further optionally, the molecular weight of the additive is less than or equal to 350 Da; further optionally, the molecular weight of the additive is less than or equal to 300 Da; and further optionally, the molecular weight of the additive is 150~300 Da;
the number of the carbon atoms in the additive is 2~40; further optionally, the number of the carbon atoms in the additive is 2~25; even further optionally, the number of the carbon atoms in the additive is 2~20; even further optionally, the number of the carbon atoms in the additive is 2~18; even further optionally, the number of the carbon atoms in the additive is 2~15; even further optionally, the number of the carbon atoms in the additive is 2~10; even further optionally, the number of the carbon atoms in the additive is 2~8; and even further optionally, the number of the carbon atoms in the additive is 2~6; and
the number of non-hydrogen atoms is 8~40 in the additive, further optionally the number of non-hydrogen atoms is 10~30 in the additive, further optionally the number of non-hydrogen atoms is 10~25 in the additive, further optionally the number of non-hydrogen atoms is 10~20 in the additive, and further optionally the number of non-hydrogen atoms is 10~18 in the additive.

The aforementioned additive (a first additive) can be finely adjusted in terms of the type and amount of the weak reducing group, the type and amount of the halogen acid molecule, and the molecular weight (wherein the molecular weight can be indirectly adjusted by adjusting the number of carbon atoms or the number of non-hydrogen atoms), so that the additive can better interact with the perovskite precursor material, so as to better reduce defects of the perovskite thin film, and to better improve the service life of the perovskite cell.

In some embodiments, the additive has a structure represented by formula (1);

L(Z₁-R₁₁)ₚ₁·nHZ (1)

in formula (1), p1 is 0 or a positive integer; Z is halogen in the hydrogen halide salt; and n is the number of hydrohalic acid molecules in the hydrogen halide salt;
any one R₁₁ is independently the weakly reducing group and has a valence of 1, optionally any one R₁₁ is independently -NHNH₂, sulfino, phosphinico, hydroxyphenyl or hydroxynaphthyl, and further optionally any one R₁₁ is independently -NHNH₂, sulfino or phosphinico;
when p1 is 0, the additive is a hydrohaliden salt of an organic compound containing at least one of -NHNH-, and -S-S-;
when p1 is a positive integer, L is alkyl, heteroalkyl, cycloalkyl, heterocycloalkyl, aryl, heteroaryl, substituted alkyl, substituted heteroalkyl, substituted cycloalkyl, substituted heterocycloalkyl, substituted aryl, or substituted heteroaryl in a valence of p1 and when L contains a heteroatom, any one heteroatom in L is independently a non-carbon atom or a non-hydrogen atom, and is further independently any one selected from N, S, P, O, and B;
L and all Z₁ contain a total of p2 divalent groups selected from -NH-NH-, and -S-S-, p2 being 0 or a positive integer; and (p1+p2)≥1
Z₁ is a chemical bond, carbonyl, -CH₂-, -CH(Q₁)- or -(Q₂)C(Q₃)-, wherein Q₁, Q₂, and Q₃are each independently selected from groups in the group of: alkyl, heteroalkyl, cycloalkyl, heterocycloalkyl, aryl, heteroaryl, substituted alkyl, substituted heteroalkyl, substituted cycloalkyl, substituted heterocycloalkyl, substituted aryl, and substituted heteroaryl; and when Z contains a heteroatom, any one heteroatom in Z₁is independently a non-carbon, or non-hydrogen atom, and further independently selected from any of N, S, P, O, and B; and optionally, Z₁ is -CH₂-, -CH(Q₁)- or -(Q₂)C(Q₃)-;
any one R₁₁ is independently directly linked to a carbon atom in Z₁ or L; and
in formula (1), alkyl, heteroalkyl, cycloalkyl, heterocycloalkyl, aryl, or heteroaryl in any one of the substituted alkyl, the substituted heteroalkyl, the substituted heterocycloalkyl, the substituted aryl, or the substituted heteroaryl contained in L, Z₁, Q₁, Q₂, or Q₃ is independently substituted with 1 or more groups selected from group G1 of: alkyl, heteroalkyl, cycloalkyl, heterocycloalkyl, aryl, heteroaryl, -CN, hydrazino, sulfino, phosphinico, -OH, -NH₂, -COOH, sulfo, phosphono, boronate, and halogen.

In some embodiments, the additive satisfies any one or more of the following features:
L is a chain structure; optionally, L is a C₂₋₂₀ chain structure; further optionally, L is a C₂₋₁₈ chain structure; even further optionally, L is a C₂₋₁₅ chain structure; even further optionally, L is a C₂₋₁₂ chain structure; even further optionally, L is a C₂₋₁₀ chain structure; and even further optionally, L is a C₂₋₈ chain structure;
L is a ring-containing structure, and the number of ring atoms in L is 3~25; optionally 3~20; further optionally 3~18; further optionally 5~18; even further optionally 5~15; even further optionally 5~12; even further optionally 5, 6, 10, 11 or 12; and even further optionally 6, 10, 11 or 12;
the number of carbon atoms in L is 2~30; optionally 2~25; further optionally 2~20; even further optionally 2~18; even further optionally 2~15; even further optionally 2~12; even further optionally 2~10; and even further optionally 2~8;
the number of non-hydrogen atoms in L is from 2~35; further optionally the number of non-hydrogen atoms in L is 2~30; even further optionally the number of non-hydrogen atoms in the additive is 2~25; even further optionally the number of non-hydrogen atoms in the additive is 2~20; and even further optionally the number of non-hydrogen atoms in the additive is 2~18;
L is a ring-containing structure, and the ring in L is an aromatic ring, an aliphatic ring, or a combination thereof; optionally, the ring in L comprises an aromatic ring; further optionally, the number of carbon atoms in L is 3~40; even further optionally, the number of carbon atoms in L is 3~30; even further optionally, the number of carbon atoms in L is 3~25; even further optionally 3~20; even further optionally 3~18; even further optionally 5~18; even further optionally 5~15; even further optionally 5~12; even further optionally 5, 6, 10, 11, or 12; and even further optionally 6, 10, 11 or 12;
L is a ring-containing structure containing 2~40 carbon atoms; and optionally, the number of carbon atoms in L is 3~30, further optionally 3~25, even further optionally 3~20, further optionally 3~18, and further optionally 4~18;
L is an aliphatic structure; and optionally, the number of carbon atoms in L is 2~2; further optionally 2~20, further optionally 2~18; even further optionally 2~15; further optionally 2~12; even further optionally 2~10, and even further optionally 2~8;
L is an aromatic structure; optionally, L comprises one or more of aryl, heteroaryl, substituted aryl and substituted heteroaryl; further optionally, L comprises one or more of C₆₋₂₀ aryl, C₄₋₂₀ heteroaryl, substituted C₆₋₂₀ aryl and substituted C₄₋₂₀ heteroaryl; and even further optionally, L comprises one or more of C₆₋₁₀ aryl, C₄₋₁₀ heteroaryl, substituted C₆₋₁₀ aryl, and substituted C₄₋₁₀ heteroaryl;
p1 is 0, and the additive is a hydrogen halide salt of a C₂₋₄₀ compound containing at least one of -NHNH-, and -S-S-;
p1 is a positive integer selected from 1~10; optionally p1 is 1, 2, 3, 4 or 5; and optionally p1 is 1 or 2;
p1 is a positive integer, L is C₂₋₁₈ alkyl, C₂₋₁₈ heteroalkyl, C₃₋₂₀ cycloalkyl, C₃₋₂₀ heterocycloalkyl, C₆₋₂₀ aryl, C₃₋₂₀ heteroaryl, substituted C₂₋₁₈ alkyl, substituted C₂₋₁₈ heteroalkyl, substituted C₃₋₂₀ cycloalkyl, substituted C₃₋₂₀ heterocycloalkyl, substituted C₆₋₂₀ aryl or substituted C₃₋₂₀ heteroaryl having a valance of p1; optionally, L is C₂₋₁₂ alkyl, C₂₋₁₂ heteroalkyl, C₃₋₁₂ cycloalkyl, C₃₋₁₂ heterocycloalkyl, C₆₋₁₅ aryl, C₃₋₁₅ heteroaryl, substituted C₂₋₁₂ alkyl, substituted C₂₋₁₂ heteroalkyl, substituted C₃₋₁₂ cycloalkyl, substituted C₃₋₁₂ heterocycloalkyl, substituted C₆₋₁₅ aryl or substituted C₃₋₁₅ heteroaryl having a valance of p1; further optionally, L is C₂₋₁₀ alkyl, C₂₋₁₀ heteroalkyl, C₅₋₁₀ cycloalkyl, C₄₋₁₀ heterocycloalkyl, C₆₋₁₀ aryl, C₃₋₁₀ heteroaryl, substituted C₂₋₁₀ alkyl, substituted C₂₋₁₀ heteroalkyl, substituted C₅₋₁₀ cycloalkyl, substituted C₄₋₁₀ heterocycloalkyl, substituted C₆₋₁₀ aryl or substituted C₃₋₁₀ heteroaryl having a valance of p1 and even further optionally, L is a linear chain structure; wherein when L contains a heteroatom, any one heteroatom in L is independently a non-carbon and non-hydrogen atom, and further independently selected from any one of N, S, P, O, and B;
Q₁, Q₂, and Q₃are each independently selected from the group of: C₁₋₆ alkyl, C₁₋₆ heteroalkyl, C₃₋₈ cycloalkyl, C₃₋₈ heterocycloalkyl, C₆₋₁₂ aryl, C₃₋₁₁ heteroaryl, substituted C₁₋₆ alkyl, substituted C₁₋₆ heteroalkyl, substituted C₃₋₈ cycloalkyl, substituted C₃₋₈ heterocycloalkyl, substituted C₆₋₁₂ aryl, and substituted C₃₋₁₁ heteroaryl; optionally, Q;₁, Q₂, and Q₃ are each independently selected from the group of: C₁₋₃ alkyl, C₁₋₃ heteroalkyl, C₃₋₆ cycloalkyl, C₃₋₆ heterocycloalkyl, C₆₋₁₀ aryl, C₃₋₉ heteroaryl, substituted C₁₋₃ alkyl, substituted C₁₋₃ heteroalkyl, substituted C₃₋₆ cycloalkyl, substituted C₃₋₆ heterocycloalkyl, substituted C₆₋₁₀ aryl, and substituted C₃₋₉ heteroaryl; and further optionally, Q₁, Q₂, and Q₃ are each independently selected from the group of: methyl, methoxy, cyclopropyl, cyclobutyl, cyclopentyl, cyclohexyl, piperidinyl (optionally 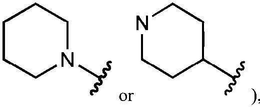 piperazinyl (optionally 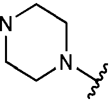 ), phenyl, naphthyl, pyridyl, pyrimidinyl, pyrazinyl, pyridazinyl, and N_{1~6} azanaphthyl, and a substituted form of any one of the aforementioned groups; wherein when₁, Q₂ or Q₃ is a substituted form of any one of the aforementioned groups, it is independently substituted by 1 or more (optionally 1 or 2~5, further optionally 1, 2 or 3) groups selected from group G1^{a} of: methyl, cyclopentyl, phenyl, benzyl, methylphenyl, -Ph-Ph, N_{1~2} aza heterocycloalkyl (optionally monoaza C₃₋₈ cycloalkyl or diaza C₃₋₈ cycloalkyl, further optionally monoaza cyclobutyl, piperidinyl or piperazinyl, even further optionally monoaza cyclobutyl, and even further optionally 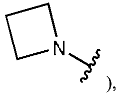 -CN, hydrazino, sulfino, phosphinico, -OH, -NH₂, -COOH, sulfo, phosphono, boronate, and halogen (optionally one or more of F, Cl, Br, and I);
the group G1 comprises alkyl (optionally C₁₋₈ alkyl, further optionally C₁₋₆ alkyl, even further optionally C₁₋₄ alkyl, even further optionally C₁₋₃ alkyl, and even further optionally methyl), heteroalkyl (optionally alkoxy or secondary amino, further optionally C₁₋₈ alkoxy or C₁₋₈ alkylamino, even further optionally C₁₋₆ alkoxy or C₁₋₆ alkylamino, even further optionally C₁₋₄ alkoxy or C₁₋₄ alkylamino, even further optionally C₁₋₃ alkoxy or C₁₋₃ alkylamino, even further optionally -NHCH₃ or -NHCH₂CH₃, and even further optionally -NHCH₃), cycloalkyl (optionally C₃₋₈ cycloalkyl, further optionally C₃₋₆ cycloalkyl, and even further optionally cyclopropyl, cyclobutyl, cyclopentyl or cyclohexyl), heterocycloalkyl (optionally 3~8-membered heterocycloalkyl having 1 or 2 N atoms as ring atoms, further optionally 3~6-membered heterocycloalkyl having 1 or 2 N atoms as ring atoms, further optionally 3~4-membered heterocycloalkyl having 1 or 2 N atoms as ring atoms, further optionally piperidinyl, piperazinyl or monoaza cyclobutyl, and further optionally 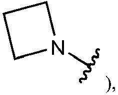 aryl (optionally C₆₋₁₂ aryl, further optionally C₆₋₁₀ aryl, and even further optionally phenyl, naphthyl or biphenyl), heteroaryl (6~12-membered aryl containing 1~6 ring nitrogen atoms, further optionally 6~12-membered aryl containing 1~4 ring nitrogen atoms, even further optionally 6~12-membered aryl containing 1~3 ring nitrogen atoms, further optionally 6~12-membered aryl containing 1 or 2 ring nitrogen atoms, even further optionally N₁₋₂azaphenyl, even further optionally monoazaphenyl or diazazaphenyl, and even further optionally pyridyl, pyrimidyl, pyrazyl or pyridazyl), -CN, hydrazino, sulfino, phosphinico, -OH, -NH₂, -COOH, sulfo, phosphono, boronate, and halogen (optionally one or more of F, Cl, Br and I); and
Z₁ is a chemical bond, -CH₂-, -CH(Q₁)- or -(Q₂)C(Q₃)-.

In some embodiments, the additive satisfies any one or more of the following features:
L is an aliphatic structure and Z₁ is a chemical bond or carbonyl (Zi optionally is a chemical bond);
L is an aromatic structure and Z₁ is not a chemical bond;
L is phenyl, naphthyl, cyclopentyl, cyclohexyl, n-octyl, n-nonyl, n-decyl, n-undecyl, n-dodecyl, n-tridecyl, n-tetradecyl, n-pentadecyl, n-hexadecyl, heptadecyl, n-octadecyl, pyridyl, piperidinyl, piperazinyl, N-methylpiperidinyl, methylphenyl, dimethylphenyl, biphenyl, naphthyl-substituted phenyl, aminophenyl, C₂₋₁₈ alkyl with 1 or more sulfo substituents (optionally ethyl with a monosulfo substituent, and further optionally 2-sulfoethyl), C₂₋₁₈ alkyl substituted with 1 or more NH₂(optionally C₂₋₁₈ alkyl substituted with one -NH₂, further optionally aminobutyl, and even further optionally 4-aminobutyl), C₂₋₁₈ alkyl substituted with 1 or more -(O=)P(OH)₂ (optionally C₂₋₁₂ alkyl substituted with 1 -(O=)P(OH)₂, further optionally phosphonopropyl, and even further optionally 3-phosphonopropyl), C₂₋₁₈ alkyl substituted by 1 or more -(O=)PR₀₂(OH) (optionally C₂₋₁₂ alkyl substituted with 1-(O=)PR₀₂(OH), optionally C₂₋₈ alkyl substituted with 1 -(O=)PR₀₂(OH), even further optionally (CH₃)(OH)(R₀₂)P(=O)-C_{2~8} alkylene-, even further optionally (CH₃)(OH)(R₀₂)P(=O)-(CH₂)_{2~8}-, and even further optionally (CH₃)(OH)(R₀₂)P(=O)-(CH₂)₃-), and R₀₂ is alkyl (optionally C₁₋₆ alkyl, further optionally C₁₋₃ alkyl, and even further optionally methyl), C₂₋₁₈ alkyl having 1 or more -(O=)PR₀₂(OH) and 1 or more NH₂- substituents (optionally C₂₋₁₈ alkyl having 1 -(O=)PR₀₂(OH) and 1 NH₂ substituent, further optionally C₂₋₁₂ alkyl having 1 -(O=)PR₀₂(OH) and 1 NH₂ substituent, even further optionally C₂₋₈ alkyl having 1 -(O=)PR₀₂(OH) and 1 NH₂₋ substituent, and even further optionally (CH₃)(OH)(R₀₂)P(=O)-CH₂CH₂CH(NH₂)-), sulfophenyl, carboxyphenyl, phosphonophenyl, and boratophenyl;
Z₁ is any one of divalent groups of: methylene, -CH(CH₃)-, -CH(benzyl)-, -CH(cyclopentyl)-, -CH(-Ph-Ph)-, - CH(phenyl), -CH(-Ph-CH₃)-, and 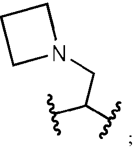
the group G1 is group G2 comprising groups of: alkyl (optionally C₁₋₈ alkyl, further optionally C₁₋₆ alkyl, even further optionally C₁₋₄ alkyl, even further optionally C₁₋₃ alkyl, and even further optionally methyl), heteroalkyl (optionally alkoxy or secondary amino, further optionally C₁₋₈ alkoxy or C₁₋₈ alkylamino, even further optionally C₁₋₆ alkoxy or C₁₋₆ alkylamino, even further optionally C₁₋₄ alkoxy or C₁₋₄ alkylamino, even further optionally C₁₋₃ alkoxy or C₁₋₃ alkylamino, even further optionally -NHCH₃ or -NHCH₂CH₃, and even further optionally -NHCH₃), cycloalkyl (optionally C₃₋₈ cycloalkyl, further optionally C₃₋₆ cycloalkyl, and even further optionally cyclopropyl, cyclobutyl, cyclopentyl or cyclohexyl), heterocycloalkyl (optionally 3~8-membered heterocycloalkyl having 1 or 2 N atoms as ring atoms, further optionally 3~6-membered heterocycloalkyl having 1 or 2 N atoms as ring atoms, even further optionally 3~4-membered heterocycloalkyl having 1 or 2 N atoms as ring atoms, still further optionally piperidinyl, piperazinyl or monoazetidinyl, and further optionally 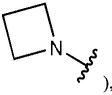 aryl (optionally C₆₋₁₂ aryl, further optionally C₆₋₁₀ aryl, and still optionally phenyl, naphthyl or biphenyl), and heteroaryl (6~12-membered aryl containing 1~6 ring nitrogen atoms, further optionally 6~12-membered aryl containing 1~4 ring nitrogen atoms, even further optionally 6~12-membered aryl containing 1~3 ring nitrogen atoms, even further optionally 6~12-membered aryl containing 1 or 2 ring nitrogen atoms, even further optionally N₁₋₂ azaphenyl, even further optionally monoazaphenyl or diazazaphenyl, and even further optionally pyridyl, pyrimidyl, pyrazyl or pyridazyl); optionally, the group G1 is the group G1^{b} comprising groups of: methyl, cyclopentyl, cyclopentylmethyl, phenyl, benzyl, biphenyl, methylphenyl, piperidinyl (optionally 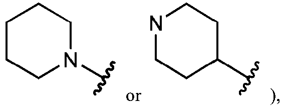 piperazinyl (optionally 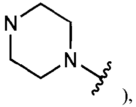 and monoaza cyclobutyl (optionally 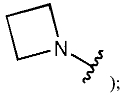 and optionally, the group G1 comprises methyl, cyclopentyl, phenyl, benzyl, biphenyl, methylphenyl, piperidinyl (optionally 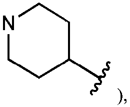 and monoaza cyclobutyl (optionally

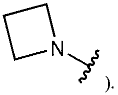

The weakly reducing group (which may be denoted as R₀₁) in the aforementioned additive related in the present application may be a monovalent group R₁₁, such as -NHNH₂, sulfino, phosphinico, hydroxyphenyl or hydroxynaphthyl, or may be a divalent group, such as -NH-NH-, -S-S-, or the like, and R₀₁ having two different valences may be any one described above, or a combination of the two. Here, monovalent R₀₁ is present at an end group, and is subjected to relatively small steric hindrance when it interact with other components in the precursor solution. Divalent R₀₁ also has an effect of a linking group, and the strength of the effect exerted by R₀₁ can be adjusted by flexibly adjusting its position in a molecule to adjust steric hindrance. The additive contains, in addition to the weakly reducing group R₀₁ and the halogen acid molecule HZ, a main moiety L(Z₁-)ₚ₁ which is an aromatic or aliphatic group and can produce a certain surface tension on a lower surface during quenching of a solvent, to promote to form crystal nuclei and to perform crystallization reaction. Z₁ may be absent; and Z₁ may also be a spacer group having a length of 1 spacer atom, and in this case, a structural property of Z₁ may be used to adjust weak reducibility of connected R₁₁. For example, when L is a benzene ring and R₁₁ is hydrazino-NHNH₂, Z₁ is -CH₂-, -CH(Q₁)- or -(Q₂)C(Q₃)- (further such as -CH₂-), facilitating to increase the stability of the perovskite precursor solution to prolong a useful period of a precursor.

In some embodiments, p1 is a positive integer, R₁₁ is -NHNH₂, and optionally, at least one Z₁ is not a chemical bond.

In some embodiments, p1 is a positive integer, L is an aromatic group, Z₁ is directly linked to the aromatic ring in L, and corresponding R₁₁ is -NHNH₂, and Z₁ is not a chemical bond; and optionally, R₁₁ is -CH₂-, -CH(Q₁)- or -(Q₂)C(Q₃)-; and
optionally, Q₁, Q₂, and Q₃ are each independently selected from the group of: alkyl, aralkyl, heteroaralkyl, substituted alkyl, substituted aralkyl, and substituted heteroaralkyl, wherein Q₁, Q₂, or Q₃, when is any one of the substituted forms described above, is independently substituted with 1 or more groups selected from the group G1.

In some embodiments, L is aryl, heteroaryl, substituted aryl, or substituted heteroaryl; and
optionally, L is any one of C₆₋₂₀ aryl, C₄₋₂₀ heteroaryl, substituted C₆₋₂₀ aryl, and substituted C₄₋₂₀ heteroaryl; and further optionally, L is any one of C₆₋₁₀ aryl, C₄₋₁₀ heteroaryl, substituted C₆₋₁₀ aryl, and substituted C₄₋₁₀ heteroaryl;
wherein L, when it is any one of the above substituted forms, is independently substituted with 1 or more groups selected from the group G1.

Improvement of the stability of the perovskite precursor solution is facilitated to prolong the useful period of the precursor in a case where a spacer group (e.g., alkylene or substituted alkylene, further such as -CH₂-, -CH(Q₁)- or -(Q₂)C(Q₃)-, and even further such as -CH₂-) is between hydrazino and a benzene ring with respect to a case where hydrazino is directly linked to an aromatic ring (e.g., a benzene ring). Taking a benzylhydrazine hydrochloride salt and a phenylhydrazine hydrochloride salt as examples, N in hydrazino has a valence of -1 and tends to lose an electron, and therefore, when N in hydrazino in the phenylhydrazine hydrochloride salt is directly linked to a benzene ring, a strong electron-withdrawing ability of the benzene ring aggravates a process of losing electrons from N in hydrazino, so that the phenylhydrazine hydrochloride salt exists as a sacrificial agent in the perovskite for a relatively short period of time; and in the benzylhydrazine hydrochloride salt, by separating hydrazino from a benzene ring by a methylene structure, the hydrazino is directly linked to a carbon atom of methylene, and the methylene tends to donate an electrons to transfer an electron to the hydrazino, so that the stability of the benzylhydrazine hydrochloride salt is increased, facilitating to increase the stability of the perovskite precursor solution to prolong the useful period of the precursor.

In addition, when a spacer group is present between hydrazino and an aromatic ring in L, it is facilitated to promote crystallization reaction, and lattice distortion can also be avoided. The spacer group may increase steric hindrance, so that it is difficult for the first additive to enter ABX₃ lattices in perovskite, and thus lattice distortion can also be avoided, and since the first additive is dissolved in the perovskite precursor solution due to the like-dissolves-like rule, it can be uniformly dispersed in the perovskite thin film to induce, to some extent, nucleus formation during crystallization.

When the spacer group is present between the hydrazino and the aromatic ring in L, a lifetime of a perovskite device can also be prolonged. Degradation of perovskite is mainly caused by degradation of A⁺ and BX₂ in a perovskite layer, a hydrochloride salt can suppress degradation of a site-A cation, and in this case, an aromatic hydrazino compound containing a spacer group can suppress degradation of a halogen anion, and also has a better stability.

L may contain an aromatic ring or be an aliphatic chain, and may produce different steric-hindrance and electron-donating abilities, leading to different levels of reaction. Given an identical number of carbon atoms, an aromatic ring-containing structure is preferred to an aliphatic chain, on one hand, when the aromatic ring-containing structure is contained, it may not be involved in perovskite crystal lattices, and on the other hand, the aliphatic chain is more hydrophobic than the aromatic ring, and possibly degrades crystal quality.

In some embodiments, the additive has a structure of any one of formula (11), formula (12), formula (13), formula (14), and formula (15):

Ar(-Z₂₁-R₁₁)ₚ₁·nHCl (11)

in formula (11), p1 is a positive integer, and Z₂₁ is -CH₂-, -CH(Q₁)- or -(Q₂)C(Q₃)-; Ar(-R₁₁)ₚ₁·nHCl (12)
in formula (12), p1 is a positive integer, and R₁₁ is sulfino, and phosphinico; 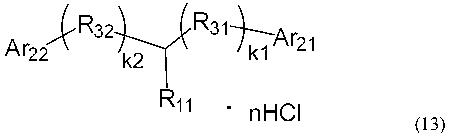
in formula (13), k1, and k2 are each independently 0 or 1; R₃₁ and R₃₂ are each independently alkylene; optionally, R₃₁ and R₃₂ are each independently C₂₋₂₀ alkylene, further optionally, R₃₁ and R₃₂ are each independently C₂₋₁₆ alkylene, even further optionally, R₃₁ and R₃₂ are each independently C₂₋₁₂ alkylene, even further optionally, R₃₁, and R₃₂ are each independently C₂₋₁₀ alkylene, even further optionally, R₃₁, and R₃₂ are each independently C₂₋₈ alkylene, even further optionally, R₃₁, and R₃₂ are each independently C₂₋₆ alkylene, and even further optionally, R₃₁, and R₃₂ are each independently C₂₋₄ alkylene; 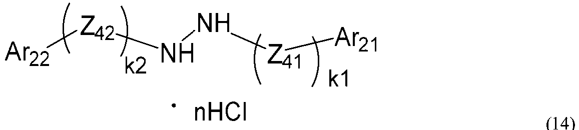 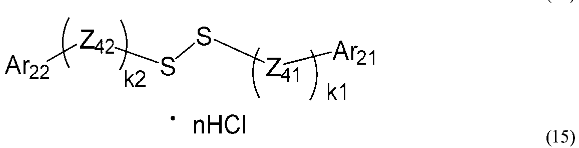
in formula (14) and formula (15), k1 and k2 are each independently 0 or 1; Z₄₁ and Z₄₂ are each independently alkylene; optionally, Z₄₁ and Z₄₂ are each independently C₂₋₁₅ alkylene, further optionally, Z₄₁ and Z₄₂ are each independently C₁₋₁₂ alkylene, even further optionally, Z₄₁ and Z₄₂ are each independently C₁₋₁₀ alkylene, even further optionally, Z₄₁ and Z₄₂ are each independently C₁₋₈ alkylene, even further optionally, Z₄₁ and Z₄₂ are each independently C₁₋₆ alkylene, even further optionally, Z₄₁ and Z₄₂ are each independently C₁₋₄ alkylene, and even further optionally, Z₄₁ and Z₄₂ are each independently methylene, ethylene, propylene or butylene;
wherein Ar, Ar₂₁, and Ar₂₂ are each independently an aromatic group; optionally the number of ring atoms of Ar, Ar₂₁, and Ar₂₂ are each independently 5~15, further optionally 5~12, further optionally 5~10 or 12, and further optionally 5, 6, 10 or 12; and further optionally Ar, Ar₂₁, and Ar₂₂ are each independently aryl, heteroaryl, substituted aryl or substituted heteroaryl, wherein aryl or heteroaryl in the substituted aryl or the substituted heteroaryl is independently substituted with 1 or more groups selected from the group G1; and when containing a heteroatom, any one heteroatom contained in any one of Ar, Ar₂₁, and Ar₂₂ is independently a non-carbon, or non-hydrogen atom, and further independently any one selected from N, S, P, O, and B.

When the first additive contains an aromatic ring, it may not be involved in perovskite lattices, and on the other hand, generally has hydrophobicity weaker than that of an aliphatic chain, so that crystal quality can be increased.

In some embodiments, the additive contains a second functional group W₁₁, and W₁₁ may be any one selected from the group of: -COOH, sulfo, phosphono, and boronate; and

in one molecule of the additive, the number j of the second functional group W₁₁ is 1 or more; optionally j is 1, 2 or 3; and further optionally j is 1 or 2.

By introducing a weakly acidic group (such as, but not limited to, one or more of -COOH, sulfo, phosphono, and boronate, etc.) into the additive, addition-elimination reaction of A (A is an organic cation) in ABX₃ can be suppressed, to prolong a service life of a perovskite device. In addition, when it is consumed in terms of acidity, it itself may be supplemented to a perovskite system to enhance a stability of a perovskite structure. Electron transport may also be enhanced when the weak acid group is coordinated with a B-site cation. A trace amount of an acid radical anion contained in W₁₁ may promote seed crystal formation and enhance seed crystal quantitative formation, and, when perovskite is annealed, such a substance is easy to exist from a lattice system thereof, so that perovskite is first subjected to a stable α-phase transition.

Taking W₁₁ as -COOH as an example, -COOH may be weakly dissociated into H⁺ and COO⁻, H⁺ may inhibit degradation of A⁺ cations, and COO⁻ has a strong ability to coordinate with B, thereby enhancing bulk stability of the perovskite and promoting long-term stability of the perovskite. As noted, introduction of a weakly acidic group does not substitute for salification of halogen acid molecules, salification of a halogen acid (for example, salification of hydrochloric acid) can significantly improve solubility; and salification with only a weakly acidic group and without halogen acid molecule may cause that the first additive can not achieve intended function or achieve a poor effect possibly because it can not be involved in the perovskite thin film due to a pre-filtration step or the like, or it is not unevenly distributed in the perovskite thin film.

In some embodiments, the additive has any one of the following structures:

(W₁₁)ⱼL₀(R₀₂)ₘ₁·nHCl (2)

in formula (2), j is a positive integer, and m1 is a positive integer; any one R₀₂ is independently the weak reducing group; Lois hydrocarbyl or heterohydrocarbyl with a valence of m1+j, the heterohydrocarbyl containing 0, 1, or more substituents selected from the group of: -CN, -OH, halogen (optionally one or more of F, Cl, Br, and I), -NH₂, -SH, -CF₃, and -COOH; and optionally, any one R₀₂ is independently selected from the group of: a -NHNH-containing group, -NHNH₂, sulfino, and phosphinico;

   (W₁₁)ⱼ₁Lo ₁(Z₁-R₁₁)ₚ₁·nHCl (21)
in formula (21), p1, and j1 are each independently a positive integer, any one Z₁, and any one R₁₁ are each independently as defined as above; L₀₁(Z₁₋)ₚ₁ contains p2 the divalent weakly reducing groups, and j2 W₁₁, wherein p2, and j2 are each independently 0 or a positive integer; and L₀₁ is hydrocarbyl or heterohydrocarbyl having a valence of p1+j1, the heterohydrocarbyl containing 0, 1 or more substituents selected from the group of: -CN, -OH, and halogen (optionally one or more of F, Cl, Br and I), -NH₂, -SH, -CF₃, and -COOH;

   (W₁₁)ⱼⱼ₁Ar₀₁(-Z₃₁-R₁₁)ₚ₁·nHCl (22)
in formula (22), p1, j1, any one R₁₁, and any one W₁₁ are each independently as defined in formula (21); and Ar₀₁ is a divalent aromatic group; wherein the divalent aromatic group contains 0, 1, or more substituents selected from the group of: -CN, -OH, -NH₂, -SH, -CF₃, -COOH, and halogen; Z₃₁ is -CH₂-, -CH(Qi)- or -(Q₂)C(Q₃)-; and Q₁, Q₂, and Q₃ are each independently a group selected from group G3 of: alkyl (optionally C₁₋₆ alkyl, further optionally C₁₋₃ alkyl, and even further optionally methyl), and heteroalkyl (optionally alkoxy or alkylamino, further optionally C₁₋₆ alkoxy or C₁₋₆ alkylamino, even further optionally C₁₋₃ alkoxy or C₁₋₃ alkylamino, even further optionally methoxy, ethoxy, -NHCH₃ or -NHCH₂CH₃, even further optionally -NHCH₃ or -NHCH₂CH₃, and even further optionally -NHCH₃); wherein the heterohydrocarbyl contains 0, 1 or more substituents selected from the group of: -CN, - OH, halogen (optionally one or more of F, Cl, Br and I), -NH₂, -SH, -CF₃, and -COOH; 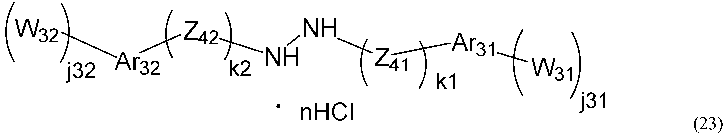 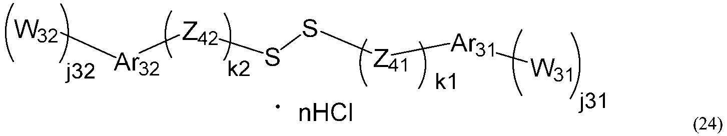
in formula (23), and formula (24), k1, k2, Z₄₁, and Z₄₂ are each independently as defined in formula (14); j31 and j32 are each independently 0 or a positive integer, with (j31 + j32) ≥1; and W₃₁ and W₃₂ are each independently any one group selected from the group of: -COOH, sulfo, phosphono, boronate, -NH ₂, and -SH;
wherein Ar₃₁ and A₃₂ are each independently a divalent aromatic group; wherein the divalent aromatic group contains 0, 1 or more substituents selected from the group of: -CN, -OH, amino, mercapto, and halogen (optionally one or more of F, Cl, Br, and I).

The aforementioned effect of the weakly acidic group can be more flexibly enabled by adjusting features, such as a type, a number, and a position of the weakly acidic group.

In some embodiments, the additive includes any any one or more of the following compounds: 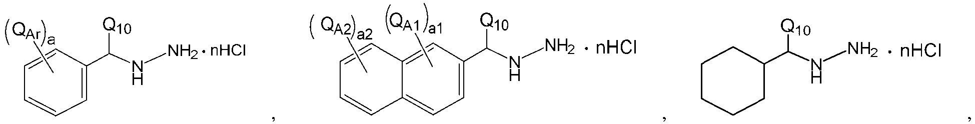 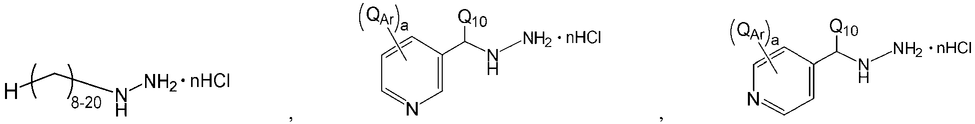 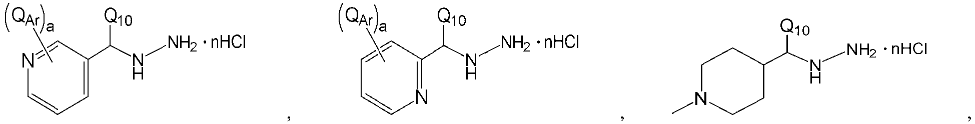 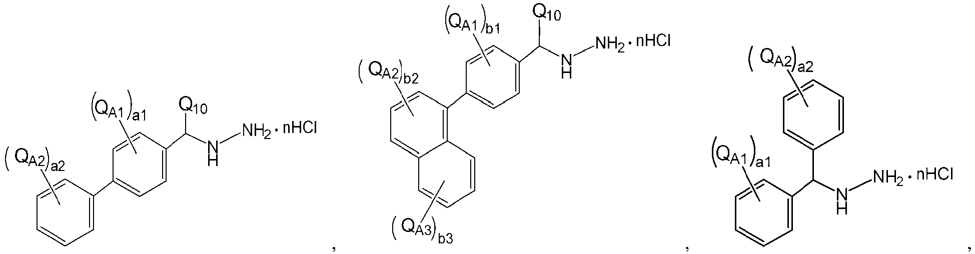 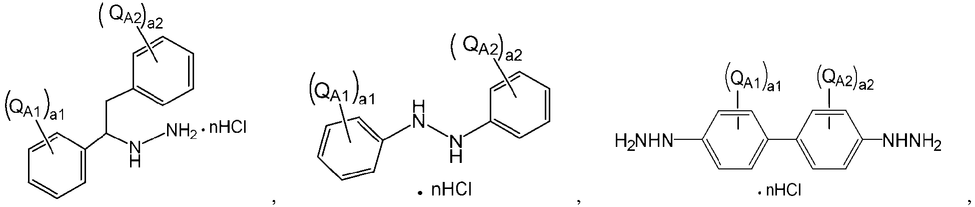 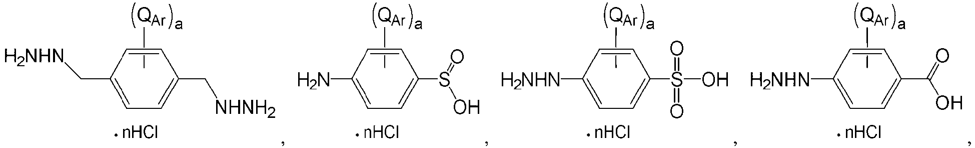 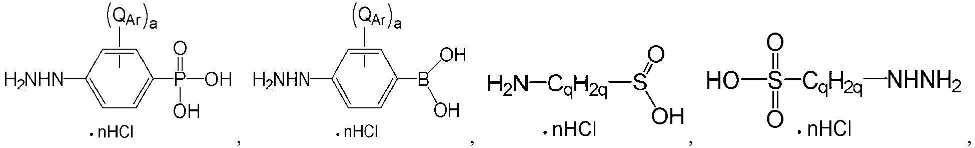 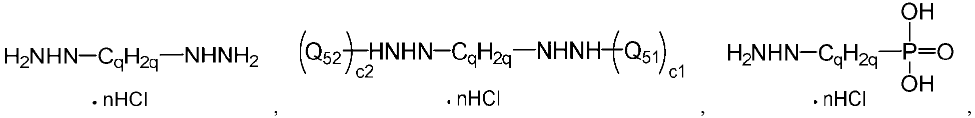 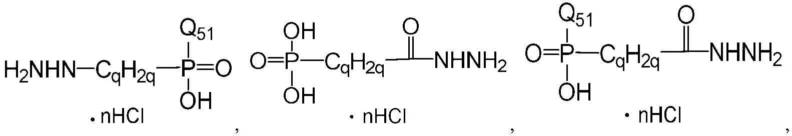 and a case where any one of the aforementioned compound salts is substituted with a substituent selected from group G1;
wherein n is a positive integer, optionally, n is an integer selected from 1~10; further optionally, n is 1, 2, 3, 4 or 5; and further optionally, n is 1 or 2;
Q_{Ar} is any one group selected from the group G1; and a is 0 or a positive integer (optionally is 0, 1 or 2);
Q₁₀ is H or Q ; optionally, Q₁₀ is H; and further optionally, Q₁₀ is Qi;
a1 and a2 are each 0 or a positive integer, with a1+a2≥1;
b1, b2, and b3 are each 0 or a positive integer, with b1+b2+b2≥1, optionally, b2+b3>1, and further optionally b1=0;
Q_{A1}, Q_{A2}, and Q_{A3}are each independently Q_{Ar};
q is an integer selected from 2~20, optionally an integer of 2~18, further optionally an integer of 2~16, even further optionally an integer of 2~12, even further optionally an integer of 2~10, even further optionally an integer of 2~8, and even further optionally an integer of 2, 3, 4, 5, 6 or 7;
c1 and c2 are each independently 0 or 1, optionally c1+c2≥1; and
Q₅₁ and Q₅₂ are each independently alkyl (optionally C₁₋₆ alkyl, further optionally C₁₋₃ alkyl, and even further optionally methyl).

In some embodiments, the additive includes any any one or more of the following compounds: 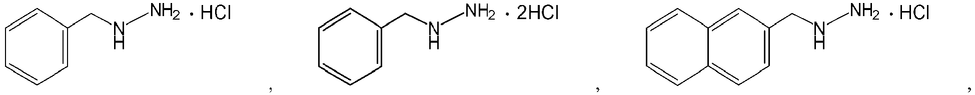 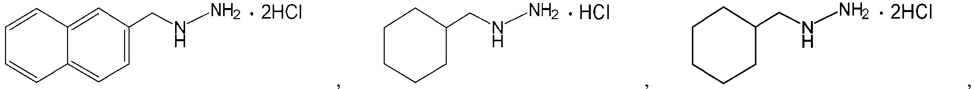 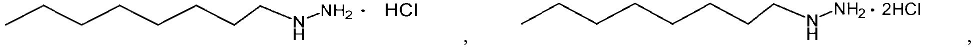 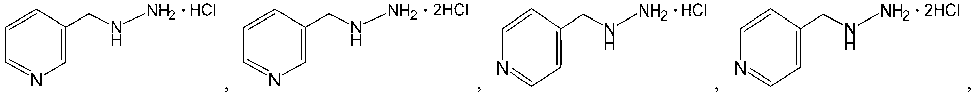 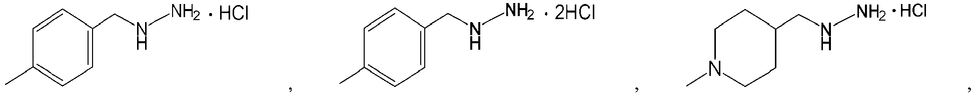 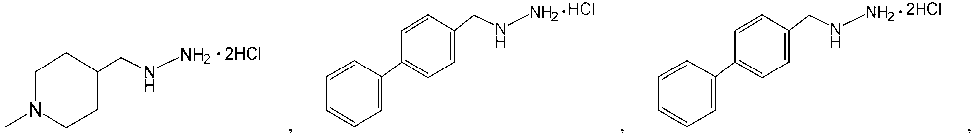 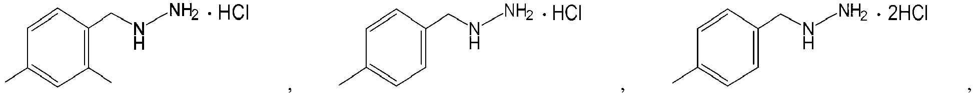 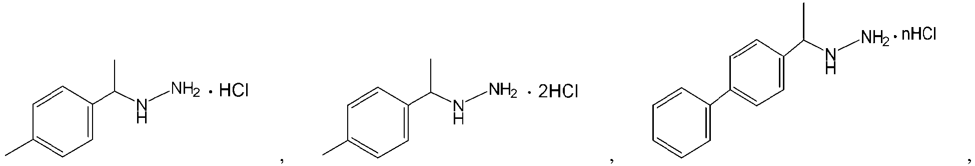 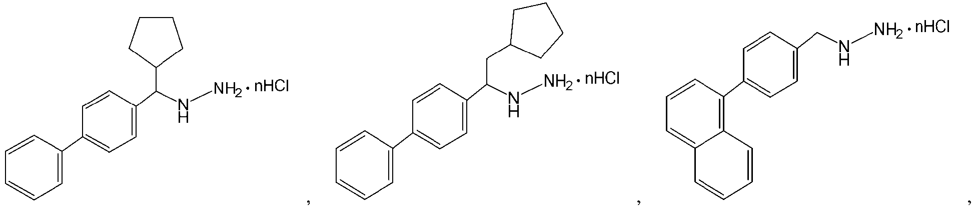 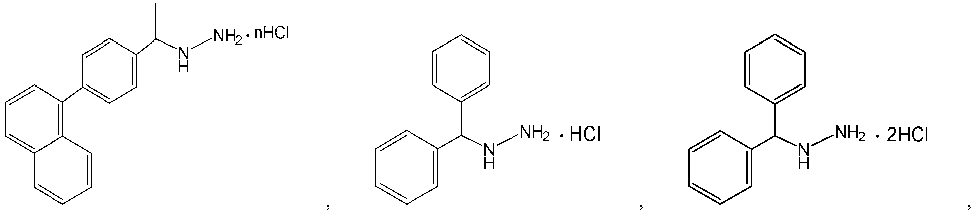 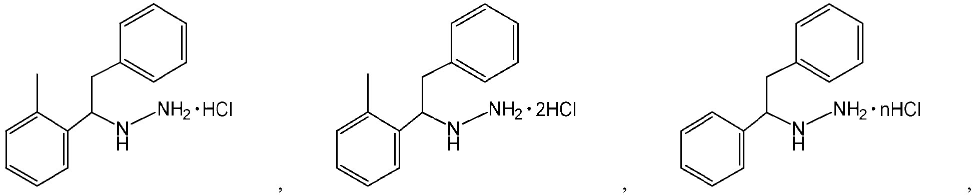 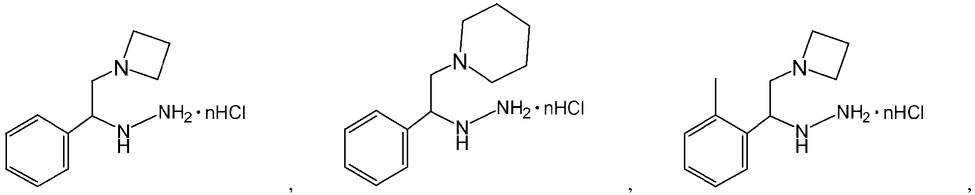 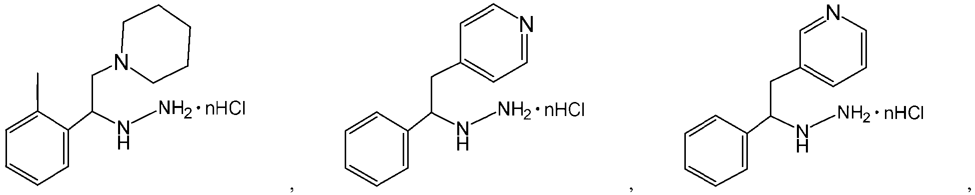 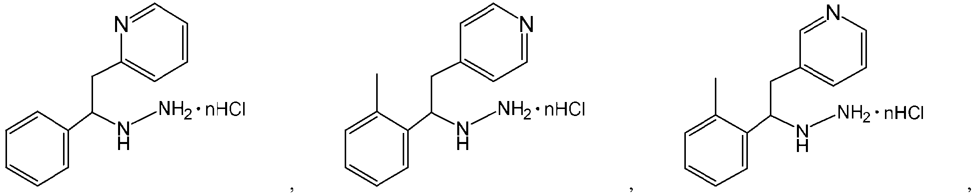 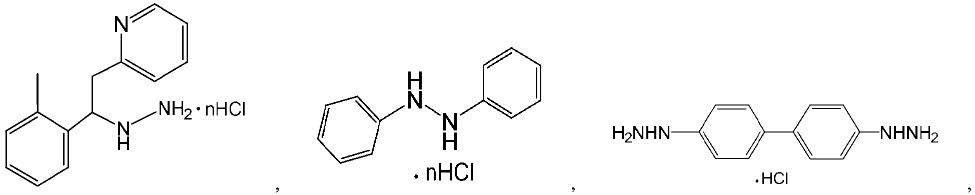 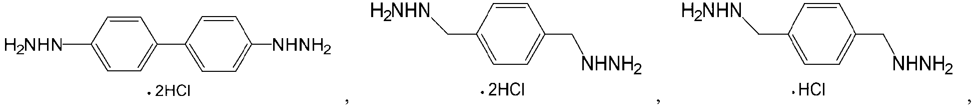 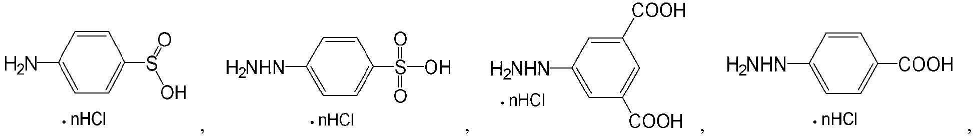 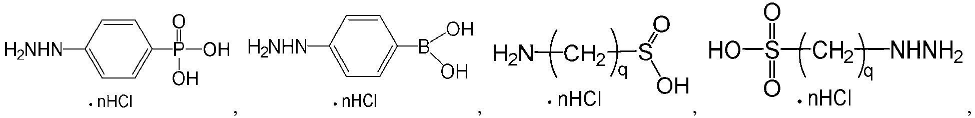 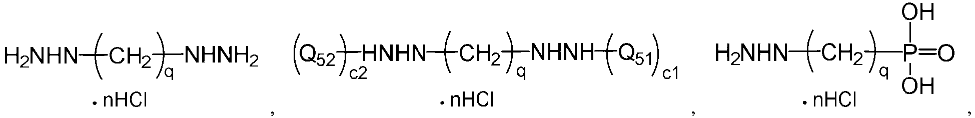 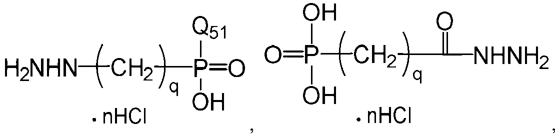 and a case where any one of the foregoing compound salts is substituted with a substituent selected from the group G1;
optionally, n is 1, 2, or 2;
optionally, n is 1; and
optionally, n is 2.

The additive may include any any one or more of the compounds described above, thereby effectively improving the quality of the perovskite thin film to reduce defects, and thereby effectively increasing the energy conversion efficiency of the perovskite cell. In addition, the perovskite cell can maintain relatively good energy conversion efficiency for a long period of time, and the cell has good stability.

In some embodiments, the perovskite precursor material comprises a perovskite-type metal halide represented by chemical formula ABX₃, where A is a monovalent cation, B is a divalent cation, and X is a monovalent anion;
optionally, A comprises one or more of Cs⁺, K⁺, Rb⁺, a monovalent amine cation, and a monovalent amidinate cation;
optionally, B comprises one or more of Pb²⁺, Sn²⁺, Fe²⁺, Mn²⁺, Ni²⁺, Ge²⁺, Co²⁺, and Sb²⁺; and
optionally, X comprises one or more of I⁻, Br⁻, and Cl⁻.

The first additive provided in the present application may improve different types of perovskite precursor material systems, increase quality of a corresponding perovskite thin film to reduce defects, and prolong a lifetime of a perovskite cell.

In some embodiments, the weight percent of the additive relative to B element in the perovskite precursor material is 0.01%-15%; and
optionally, the weight percent of the additive relative to B element in the perovskite precursor material is 0.01%~10%;
   and
optionally, the weight percent of the additive relative to B element in the perovskite precursor material is 0.1%~8%.

In some embodiments, the molar ratio of the additive to B in the perovskite-type metal halide is 0.001%~15%;
optionally, the molar ratio of the additive to B in the perovskite-type metal halide is 0.01~15%; and
optionally, the molar ratio of the additive to B in the perovskite-type metal halide is 2~6.5%.
   In some embodiments, the molar ratio of the weak reducing group to the B element in the perovskite-type metal halide is 0.01~15%; and
optionally, the molar ratio of the weak reducing group to the B element in the perovskite-type metal halide is 1~8%.

The first additive can function better by controlling its weak reducing capacity contributed to the perovskite precursor solution by adjusting one or more of the weight percentage of the first additive relative to the B element in the perovskite precursor material, the molar ratio of the first additive to the B element in the perovskite precursor material, the molar ratio of the weak reducing group to the B element in the perovskite-type metal halide, and the like.

In some embodiments, the perovskite precursor solution further contains an oxidation-state ion of a multi-valent metal element M.

In some embodiments, the multi-valent metallic element M comprises one or more elements of lanthanide elements, Fe, Co, Ni, Ti, Cr, Mn, Y, Rh, and Bi;
optionally, the lanthanide elements comprise one or more elements of Ce, Pr, Sm, Eu, Tb, and Yb;
optionally, the oxidation-state ion of the multi-valent metal element M comprises one or more of Ce⁴⁺, Pr⁴⁺, Sm³⁺, Eu^{3+,} Tb⁴⁺, Yb³⁺, Fₑ³⁺, Co³⁺, Ni³⁺, Ti⁴⁺, Cr³⁺, Mₙ⁴⁺, Y³, and Rh⁴⁺; and
optionally, the oxidation-state ion of the multi-valent metal element M is derived from an organic salt of the metal element M, and further optionally, the organic salt includes one or more of an acetylacetonate salt, a sulfonate salt, and a sulfate salt.

In some embodiments, the perovskite precursor solution contains one or more ion pairs of Ce³⁺-Ce⁴⁺, Pr⁴⁺-Pr³⁺, Sm³⁺-Sm²⁺, Eu³⁺-Eu²⁺, Tb⁴⁺-Tb³⁺, Yb³⁺-Yb²⁺, Fe³⁺-Fe²⁺, Co³⁺-Co²⁺, Ni³⁺-Ni²⁺, Ti⁴⁺-Ti^{x+}, Cr³⁺-Cr²⁺, Mn⁴⁺-Mn²⁺, Y³⁺-Y²⁺, Rh⁴⁺-Rh²⁺, and Bi³⁺-Bi²⁺; wherein Ti⁺ in ion pair Ti⁴⁺-Ti^{X+} has a positive charge valence less than 4.

In some embodiments, the perovskite precursor material includes the perovskite-type metal halide;
the molar ratio of the multi-valent metal element M to the B element in the perovskite-type metal halide is 0.001%~15%;
   and
optionally, the molar ratio of the multi-valent metal element M to the B element in the perovskite-type metal halide is 0.01%~15%.

By introducing the oxidation-state ion of the multi-valent metal cation into the perovskite precursor solution, the cation can react with B⁰ to form a reduction-state cation, and the oxidation-state ion of the multi-valent metal cation and the reduction-state cation can constitute a redox-balance pair which can cyclically inhibit degradation of BX₂ in the perovskite thin film, thereby prolonging the lifetime of the perovskite cell, and increasing energy conversion efficiency; and further, formation of B⁰, and X₂ can be inhibited to reduce defects at an interface, thereby further increasing performance of a perovskite device.

In a second aspect, the present application provides a perovskite thin film prepared by using the perovskite precursor solution described in the first aspect.

In a third aspect, the present application provides a perovskite cell including the perovskite thin film described in the second aspect.

In some embodiments, the perovskite cell is an inversed p-i-n cell or a normal n-i-p cell.

The perovskite thin film prepared by the perovskite precursor solution described in the first aspect of the present application has high quality with few defects, and the perovskite cell further prepared has high energy conversion efficiency, and also relatively good stability.

In a fourth aspect, the present application provides an electric device, comprising the perovskite cell described in the third aspect.

Details of one or a plurality of embodiments of the present application are set forth in the accompanying drawings and the description below. Other features, objectives, and advantages of the present application will be apparent from the specification, and the accompanying drawings, and the claims.

### DESCRIPTION OF DRAWINGS

In order to better describe and illustrate embodiments and/or examples of those applications disclosed herein, reference may be made to one or a plurality of accompanying drawings. Additional details or examples used to describe the accompanying drawings should not be considered as limiting the scope of any of the disclosed application, the currently described embodiments or examples, and best modes of these applications as currently understood. Moreover, the same reference numerals are used to denote the same components throughout the accompanying drawings. In the accompanying drawings:
FIG. 1 is a schematic diagram of a perovskite cell according to an embodiment of the present application, including a first electrode, a first transport layer, a perovskite layer, a second transport layer, and a second electrode;
FIG. 2 is a schematic diagram of a perovskite cell according to an embodiment of the present application, including a substrate layer, a first electrode, a first transport layer, a perovskite layer, a second transport layer, and a second electrode;
FIG. 3 is a schematic diagram of a perovskite cell according to an embodiment of the present application; and
FIG. 4 is a schematic diagram of an electric device using a perovskite cell as a power source according to an embodiment of the present application.

Description of reference number: 100 perovskite cell, 110 substrate layer, 120 first electrode, 130 first transport layer, 140 perovskite layer, 150 second transport layer, 160 second electrode, P1 first scoring area, P2 second scoring area, P3 third scoring area, 20 electrical device.

### DESCRIPTION OF EMBODIMENTS

Hereinafter, some embodiments of the perovskite precursor solution, perovskite thin film, perovskite cell, and electrical device disclosing the present application are described below in detail with reference to the figures as appropriate. However, an unnecessary detailed description may be omitted. For example, a detailed description of well-known matters and repeated descriptions of a substantially same structure may be omitted. This is to avoid the following descriptions from becoming unnecessarily redundant and to facilitate understanding by those skilled in the art. The accompanying drawings and the following descriptions are provided for those skilled in the art to fully understand this application, and are not intended to limit subject matters described in the claims.

The "range" disclosed in this application is limited in the form of a lower limit and an upper limit. A given range is limited by selecting a lower limit and an upper limit, which define the boundaries of the specific range. A range defined in this manner may include an end value or may not include an end value, and may be any combination, that is, any lower limit may be combined with any upper limit to form a range. For example, if the ranges of 60-120 and 80-110 are listed for a specific parameter, it is understood that the ranges of 60-110 and 80-120 are also expected. In addition, if the minimum range values of 1 and 2 are listed, and if the maximum range values of 3, 4, and 5 are listed, the following ranges may all be expected: 1-3, 1-4, 1-5, 2-3, 2-4, and 2-5. In this application, unless otherwise stated, a numerical range "a-b" represents a shorthand representation for a combination of any real numbers between a and b, where both a and b are real numbers. For example, the numerical range of "0-5" represents that all real numbers between "0-5" have been listed herein, and "0-5" is only a shortened representation of these numerical combinations. In addition, when a parameter is expressed as an integer ≥ 2, it is equivalent to disclosing that the parameter is an integer such as 2, 3, 4, 5, 6, 7, 8, 9, 10, 11, or 12. In addition, when a parameter is expressed as an integer sleeted from "2-10", it is equivalent to list integer 2, 3, 4, 5, 6, 7, 8, 9, 10, 11, or 10.

Unless otherwise specified, all embodiments and optional embodiments of this application may be combined with each other to form new technical solutions.

Unless otherwise specified, all steps in this application may be performed sequentially or randomly, preferably sequentially. For example, the method includes steps (a) and (b), which indicates that the method may include sequentially performed steps (a) and (b) or may include sequentially performed steps (b) and (a). For example, the mentioned method may further include step (c), which indicates that step (c) may be added to the method in any order, for example, the method may include steps (a), (b), and (c), may include steps (a), (c), and (b), may include steps (c), (a) and (b), or the like.

Unless otherwise specified, the terms "including", "containing", and "comprising" as used herein are meant to be open or closed. For example, the "including", "containing", and "comprising" may mean that other components not listed may be further included or contained, or only the listed components may be included or contained.

In this application, the term "or" is inclusive, unless specifically stated otherwise. For example, the phrase "A or B" means "A, B, or both A and B". Further, the condition "A or B" is satisfied by either A being true (or present) and B being false (or absent), A being false (or absent) and B being true (or present), or both A and B being true (or present).

In the present application, unless otherwise specified, A (e.g., B) means that B is a non-limiting example of A, and it may be understood that A is not limited to B.

Unless otherwise defined, "multiple", "a plurality of" and the like related in the present application mean a number of two or more. For example, "one or more" means one or greater or equal to two.

As used herein, "a combination thereof", "any combination thereof", "any combination mode thereof", and the like includes all suitable combination modes of any two or more of listed items.

Herein, "suitable" in "suitable combination mode", "suitable mode", "any suitable mode", and the like allows that the technical solution of the present application can be implemented.

Herein, "preferred", "better", "optimal", and "appropriate" are merely used to describe preferred embodiments or examples, and should not be construed to limit the scope of the present disclosure. If "preferred" occurs repeatedly in a technical solution, each "preferred" is independent, unless otherwise specified without inconsistency or mutual restriction.

In the present application, "further", "even further", "particularly", and the like are used for descriptive purposes to indicate differences in content, but should not be construed to limit the scope of the present application.

In the present application, in "first aspect", "second aspect", "third aspect", "fourth aspect", "first additive", "second additive" and the like, terms "first", "second", "third", "fourth" and the like are used for descriptive purposes only and are not to be understood as indicating or implying relative importance or quantity, nor as implicitly indicating the importance or quantity of the indicated technical feature. Also, "first", "second", "third", "fourth" and the like are used merely for the purpose of describing a non-exhaustive list, and it should be understood that they do not constitute a closed limitation on the quantity.

Herein, the term "room temperature" generally refers to 4°C~35°C, and may refer to 20°C ± 5°C. In some embodiments in the present application, room temperature refers to 20°C~30°C.

In the present application, when the unit of a data range is related, the unit of a left end point and the unit of a right end point are the same if the unit is attached only after the right end point. For example, 3~5 h or 3~5 h each means that the unit of the left end point "3" and the unit of the right end point "5" are both h (hour).

A weight of a relevant component mentioned in the description of the embodiment of the present application may not only indicate the content of each component, but also indicate a ratio relation among a weight of each component. Therefore, it is within the scope disclosed in the description of the embodiment of the present application to scale up or down the content of the relevant component according to the description of the embodiment of the present application. Further, the weight described in the description of the embodiments of the present application may be a mass unit known in the chemical industry such as µg, mg, g, kg, etc.

Herein, unless otherwise specified, "alkyl" refers to a monovalent residue generated by losing one hydrogen atom from saturated hydrocarbon containing a primary (positive) carbon atom, or a secondary carbon atom, or a tertiary carbon atom, or a quaternary carbon atom, or a combination thereof. A phrase containing the term, for example, "C₁~C₉ alkyl" means alkyl containing 1 to 9 carbon atoms, which, each time it appears, may independently be C₁ alkyl, C₂ alkyl, C₃ alkyl, C₄ alkyl, C₅ alkyl, C₆ alkyl, C₇ alkyl, C₈ alkyl or C₉ alkyl. Suitable examples include, but are not limited to, methyl (Me, -CH)₃, ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, - CH₂CH₂CH₂CH₂CH₃), 2-pentyl (-CH(CH3)CH2CH2CH3), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2, 3-dimethyl-2-butyl (-C(CH₃)₂CH(CH₃)₂), 3, 3-dimethyl-2-butyl (-CH(CH₃)C(CH₃)₃, and octyl (-(CH₂)₇CH₃).

Herein, unless otherwise specified, "heteroalkyl" refers to alkyl in which at least one carbon atom is substituted by a non-carbon atom which may be an N atom, an O atom, an S atom, a P atom, or the like. Description is made below taking O, N, and S as examples. For example, if a carbon atom, linked to an adjacent group, in an alkyl group is replaced by a non-carbon atom O, N, S, the resulting heteroalkyl group is alkoxy (e.g., -OCH₃, etc.), amino (e.g., -NHCH₃, -N(CH₃)₂, etc.), or thioalkyl (e.g., -SCH₃), respectively. If a carbon atom, that is not directly linked to an adjacent group, in an alkyl group is substituted by a non-carbon atom O, N, S, the resulting heteroalkyl groups are alkoxyalkyl (e.g., -CH₂CH₂-O-CH₃, etc.), alkylaminoalkyl (e.g., -CH₂NHCH₃, -CH₂N(CH₃)₂, etc.) or alkylthioalkyl (e.g., -CH₂-S-CH₃), respectively. If a terminal carbon atom of alkyl is substituted by a non-carbon atom, the resulting heteroalkyl group can be hydroxyalkyl (e.g., -CH₂CH₂-OH), aminoalkyl (e.g., -CH₂NH₂), or mercaptoamino (e.g., -CH₂CH₂-SH). A phrase comprising the term "heteroalkyl", e.g., "C₁~C₉ heteroalkyl" or "C₁₋₉ heteroalkyl" means heteroalkyl comprising 1 to 9 carbon atoms, which, each time it appears, independently be C₁ heteroalkyl, C₂ heteroalkyl, C₃ heteroalkyl, C₄ heteroalkyl, C₅ heteroalkyl, C₆ heteroalkyl, C₇ heteroalkyl, C₈ heteroalkyl or C₉ heteroalkyl.

Herein, unless otherwise specified, "cycloalkyl" and "non-aromatic cycloalkyl" have the same meaning, and refer to a monovalent residue generated from the loss of one ring hydrogen atom from non-aromatic hydrocarbon (saturated or unsaturated) comprising a ring-membering carbon atom, i.e., direct formation of a monovalent linking site point on the ring. Cycloalkyl derived from non-aromatic saturated hydrocarbon may be recorded as saturated cycloalkyl, and cycloalkyl derived from non-aromatic unsaturated hydrocarbon may be recorded as unsaturated cycloalkyl. The cycloalkyl may be monocycloalkyl, or spirocycloalkyl, or bridged cycloalkyl. A phrase containing the term, for example, "C₃~C₉ cycloalkyl" or "C₃~₉ cycloalkyl" means cycloalkyl containing 3 to 9 carbon atoms, which, each time it appears, may independently be C₃ cycloalkyl, C₄ cycloalkyl, C₅ cycloalkyl, C₆ cycloalkyl, C₇ cycloalkyl, C₈ cycloalkyl or C₉ cycloalkyl. Suitable examples include, but are not limited to, cyclopropyl 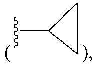 cyclobutyl ( 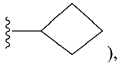 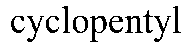 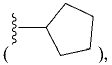 cyclohexyl 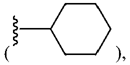 and cycloheptyl. In addition, "cycloalkyl" may also contain one or more double bonds, and representative examples of cycloalkyl containing a double bond include, but are not limited to, cyclopentenyl (including, but not limited to 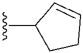 ), cyclohexenyl (including but not limited to 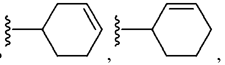 and 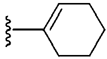 ), cyclohexadiene (including but not limited to 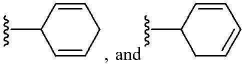 ), cyclopentadienyl (including but not limited to 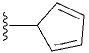 ), cyclobutadienyl (including but not limited to 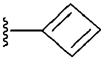 ), and the like.

Herein, unless otherwise specified, "heterocycloalkyl" refers to cycloalkyl in which at least one carbon atom substituted by a non-carbon atom which may be an N atom, an O atom, an S atom, a P atom, or the like, and may be a saturated or partially unsaturated ring. A phrase containing the term, for example, "C₄~C₉ heterocyclyl" means heterocyclyl containing 4 to 9 carbon atoms, which may independently be, each time it appears, C₄ heteroalkyl, C₅ heteroalkyl, C₆ heteroalkyl, C₇ heteroalkyl, C₈ heteroalkyl, or C₉ heteroalkyl. Suitable examples include, but are not limited to, dihydropyridyl, tetrahydropyridyl (piperidyl), tetrahydrothienyl, thiooxidated tetrahydrothienyl, tetrahydrofuranyl, tetrahydroquinolinyl, tetrahydroisoquinolinyl, indolinyl, and the like.

Herein, unless otherwise specified, "aryl" refers to aromatic alkyl derived from an aromatic cyclic hydrocarbon compound by the loss of one hydrogen atom, i.e., formation of a monovalent linking site directly on a ring, and may be monocyclic aryl, or fused ring aryl, or polycyclic aryl; and in a case where multiple rings are present, at least one of the rings is an aromatic ring system. For example, "C₆~C₁₀ aryl" means aryl containing 6 to 10 carbon atoms, which may independently be, each time it appears, C₆ aryl, C₈ aryl, C₉ aryl, or C₁₀ aryl. For another example, "C₆~C₂₀" aryl means aryl containing 6~20 carbon atoms which, each time it appears, may independently be, but not limited to C₆ arylaryl (e.g., phenyl), C₆ arylaryl (e.g., benzocyclobutenyl), C₈ aryl (e.g., phenylpropcyclobutenyl), C₉ aryl (e.g. Indenyl), C₁₀ aryl (e.g., naphthyl), C₁₂ aryl (e.g., acenaphthyl, biphenyl), C₁₃ aryl (e.g., fluorenyl), C₁₄ aryl (e.g., anthracenyl, phenanthrenyl), C₁₈ aryl (e.g., triphenylenyl) orC₂₀ aryl (such asperylenyl). Examples of suitable aromatic cyclic hydrocarbon compounds include, but are not limited to, benzene, benzocyclobutene, biphenyl, indene, naphthalene, acenaphthylene, fluorene, anthracene, phenanthrene, triphenylene, perylene, and derivatives thereof.

Herein, unless otherwise specified, "heteroaryl" is an aromatic heterocyclic radical, and may be a monovalent group in which at least one carbon atom is substituted by a non-carbon atom on the basis of aryl, or a monovalent group in which at least one carbon atom is substituted by a non-carbon atom on the basis of a cyclopentadienyl group, and the non-carbon atom may be, but is not limited to, an N atom, an O atom, an S atom, or the like. For example, "C₁~C₁₀ heteroaryl" means heteroaryl containing 1 to 10 carbon atoms, which, each time it appears, may independently be C₁ heteroaryl (e.g., tetrazolyl, etc.), C₂ heteroaryl (e.g., triazolyl, oxadiazolyl, etc.), C₃ heteroaryl (e.g., imidazolyl, etc.), C₄ heteroaryl (e.g., furyl, etc.), C₅ heteroaryl (e.g., pyridyl, etc.), C₆ heteroaryl, C₇ heteroaryl (e.g., benzimidazole, etc.), C₈ heteroaryl (e.g., indolyl, etc.), C₉ heteroaryl (e.g., quinolinyl, etc.), or C₁₀ heteroaryl (e.g., pyrrolodipyridyl). For another example, "C₃~C₂₀ heteroaryl" means heteroaryl comprising 3 to 20 carbon atoms, which, each time it appears, may independently be, but not limited to, C₂ heteroaryl, C₃ heteroaryl, C₄ heteroaryl, C₅ heteroaryl, C₆ heteroaryl, C₈ heteroaryl, C₉ heteroaryl, C₁₀ heteroaryl, C₁₂ heteroaryl, C₁₃ heteroaryl, C₁₄ heteroaryl, C₁₈ heteroaryl or C₂₀ heteroaryl. Suitable examples include, but are not limited to, heteroaryl derived from the following heteroaromatic rings (number of carbon atoms indicated in parentheses): furan(C₄), benzofuran(C₈), thiophene (C₄), benzothiophene (C₈), pyrrole (C₄), pyrazole (C₃), triazole (C₂), imidazole (C₃), oxazole (C₃), oxadiazole (C₂), thiazole (C₃), tetrazole (C₁), indole (C₈), carbazole (C₁₂), pyrroloimidazole (C₅), pyrrolopyrrole (C₆), thienopyrrole (C₆), thienothiophene (C₆), furanopyrrole (C₆), furanfuran (C₆), thienofuran(C₆), thienopyridine (C₇), furopyridine (C₇), benzoxazole (C₇), benzisoxazole (C₇), benzothiazole (C₇), benzisothiazole (C₇), benzimidazole (C₇), pyridine (C₅), pyrazine (C₄), pyridazine (C₄), pyrimidine (C₄), triazine (C₃), quinoline (C₉), isoquinoline (C₉), diazanaphthalene (C₈, such as o-diazanaphthalene), quinoxaline (C₈), phenanthridine (C₁₃), pidine (C₁₁), quinazoline (C₈), and quinazolinone (C₈).

Herein, unless otherwise specified, "alkylene" refers to an alkyl group having two monovalent group centers that is derived from alkane by removing its two hydrogen atoms (or derived from alkyl by losing its one hydrogen atom), and can be saturated branched alkyl or saturated straight-chain alkyl. For example, "C₁~C₉ alkylene" means that an alkyl moiety contains 1 to 9 carbon atoms, and, each time it appears, may independently be C₁ alkylene, C₂ alkylene, C₃ alkylene, C₄ alkylene, C₅ alkylene, C₆ alkylene, C₇ alkylene, C₈ alkylene or C₉ alkylene. Suitable examples include, but are not limited to, methylene (-CH₂-), 1, 1-ethyl (-CH(CH₃)-), 1, 2-ethyl (-CH₂CH₂-), 1, 1-propyl (-CH(CH₂CH₃)-), 1, 2-propyl (-CH₂CH(CH₃)-), 1, 3-propyl (-CH₂CH₂CH₂-), and 1, 4-butyl (-CH₂CH₂CH₂CH₂-)^{·}

Herein, unless otherwise specified, "halogen" or "halo" means F, Cl, Br Cl, Br, or 1.

Herein, unless otherwise specified, "amino" can be primary amino (-NH₂), secondary amino (> NH), tertiary amino (> N -), or quaternary amino (> N⁺ <).

Herein, unless otherwise specified, hydroxy is -OH, carboxy is -COOH, cyano is -CN, hydrazino is -NHNH₂, divalent hydrazino is -NHNH-, sulfino is -S(=O)OH, phosphinico is (*-)₂P(=O)OH, sulfo is -S(=O)₂OH, phosphono is (* -)P(=O)(OH)₂, and boronate is (*-)B(OH)₂. Here, * in the phosphinico denotes connections to a carbon atom or H and at least one connection to a carbon atom, * in the phosphono denotes connections to a carbon atom, and * in the boronate denotes connections to a carbon atom.

In the present application, a valence of an atom, a group or a compound residue participating in formation of a covalent bond refers to the number of linking sites at which the atom, group or compound residue participates in the formation of the covalent bond. For example, the valence of alkyl (-CH₃) is monovalent, and the valence of alkylene (-CH₂-) is divalent. For another example, the valence of a group such as -OH, -COOH, -CN, -NHNH₂ or -S(=O)OH is monovalent, and the valence of -NHNH- or -S-S- is divalent. The difference between "valence" and a charge valence of an ion may be understood by those skilled in the art.

In this application, "a charge valency of an ion" refers to the charge number of the ion which may be positively or negatively charged. For example, a trivalent iron ion (Fe³⁺) has a positive charge valence of 3, and an iodide ion (I⁻) has a negative charge valence of 1.

In a perovskite cell, main component ABX₃ of a perovskite precursor solution and a perovskite thin film is unstable, wherein halogenated metal moiety BX₂ of AX-BX₂ is easily degraded, for example, I⁻ in lead iodine-based perovskite is easily oxidized to I₂, and Pb²⁺ is easily reduced to Pb⁰, resulting in an increased defect density, and causing a shortened lifetime and reduced efficiency of a perovskite cell.

ABX₃ ↔ AX·BX₂

BX₂ → B⁰ + X2

When A is an organic cation, an addition-elimination reaction may also occur to accelerate decomposition reaction of BX₂.

AX ↔ A⁺ + X⁻ ↔ A↑+H⁺ + X⁻

4H⁺ + 4X⁻ + O₂ → 2H₂O + 2X₂

On that basis, in a first aspect, the present application provides a perovskite precursor solution comprising an additive containing a weakly reducing group (which may be denoted as R₀₁) and in the form of a hydrogen halide salt, and the additive may be denoted as "a first additive" herein. "First" in "a first additive" is used for descriptive purposes only and is not to be understood as indicating or implying relative importance or quantity, nor as implicitly indicating the importance or quantity of the indicated technical feature.

In the present application, unless otherwise specified, "a weakly reducing group" used herein, which may be noted as R₀₁, refers to a group having the following reducibility: the weakly reducing group has reducibility on a 0 valence corresponding to a monovalent anion in the perovskite-type metal halide and is inert to a divalent cation in the perovskite-type metal halide. In the perovskite precursor solution, the weak reducibility can enable that 0-valence X₂ is reduced to negative ion X⁻, but, the weak reducibility can not enable that divalent cation B²⁺ is reduced to 0-valence B⁰. That is, it has reducibility on 0-valence X, while it is inert to divalent cation B²⁺. The weak reducing group may be a monovalent group or a polyvalent group, wherein the polyvalent weak reducing group may be divalent. Examples of the divalent weakly reducing groups include, but are not limited to, -NHNH-, and -S-S-. Examples of the monovalent weak reducing groups include, but are not limited to, -NHNH₂, sulfino, phosphinico, hydroxyphenyl, and hydroxynaphthyl. The weak reducing group may include one or more of: -NHNH-, -S-S-, -NHNH₂, sulfino, phosphinico, hydroxyphenyl, and hydroxynaphthyl, further may include one or more of: -NHNH-, -S-S-, -NHNH₂, sulfino, and phosphinico, and further optionally may include one or more of: -NHNH₂, ssulfino, and phosphinico.

The concentrations of X⁻ and B²⁺ in the perovskite precursor solution may be detected before and after addition of an additive to a base solution of the perovskite precursor solution, and it can be determined whether the reducing property of the additive is within the scope of the "weak reducing property" of the present application based on the change of the concentrations of both ions. If the concentration of X increases and the concentration of B²⁺is substantially not changed after an additive is added to a base solution of a perovskite precursor solution, it can be considered whether the reducibility of the additive is within the scope of "the weak reducibility" of the present application. Here, the concentrations of X⁻ and B²⁺ can be determined by appropriately selecting a method capable of characterizing concentrations of both ions in the field of organic chemistry, and a selected method should be compatible with the perovskite precursor solution system of the present application. Taking X as iodine and X⁻ as I⁻ as an example, a starch potassium iodide reagent can be used to detect changes in the concentration of I⁻ ions.

In some embodiments, the weakly reducing group has reducibility on a 0 valence of the following monovalent anion in the perovskite-type metal halide: Br⁻, I⁻, or a combination thereof, i.e., the weakly reducing group has reducibility on Br, I, or a combination thereof in the perovskite precursor solution. In some of the embodiments, the weakly reducing group has reducibility on I in the perovskite precursor solution.

In some embodiments, the present application provides a perovskite precursor solution comprising a perovskite precursor material, a solvent, and an additive, wherein the perovskite precursor material comprises a perovskite-type metal halide; the additive (i.e., a first additive) contains, in its structure, a weakly reducing group which has reducibility on a 0 valence corresponding to a monovalent anion in the perovskite-type metal halide and is inert to a divalent cation in the perovskite-type metal halide; and the additive is a hydrogen halide salt.

In the present application, reference to "the additive" and "the first additive" refers to the aforesaid hydrogen halide salt additive having weak reducibility, unless otherwise specified.

By adding, to the perovskite precursor solution, an additive (noted as a first additive), a hydrogen halide salt of an organic compound, containing a weakly reducing group, for metal halide moiety BX₂ in main component ABX₃ in the perovskite precursor solution and the perovskite thin film, first, the weakly reducing group can increase storage time of the perovskite precursor solution by re-reducing X₂ (X has a valence of 0) to X⁻, that is, the weakly reducing group has reducibility on a 0 valence corresponding to a monovalent anion in the perovskite-type metal halide; second, the weak reducibility of the weakly reducing group does not lead to reduce B²⁺ to B⁰, that is, the weakly reducing group is inert to a divalent cation in the perovskite-type metal halide; third, the additive can participate in crystallization reaction and is uniformly dispersed in the perovskite thin film and can act as a sacrificial agent, so that a service life of a perovskite device can be prolonged; and fourth, formation of B⁰, and X₂ can be suppressed to reduce defects at an interface to increase performance of the perovskite device; and the additive can be better dissolved in the perovskite precursor solution and distributed more uniformly in the perovskite thin film by forming a hydrogen halide salt. Here, the perovskite precursor solution is generally a colloidal solution, BX₂ in the solution is often present in a state of being complexed with a solvent and distributed in a system, and the weak reducing group related in the present application easily forms a hydrogen bond with the perovskite colloidal solution and can be uniformly distributed in the colloidal solution; and when crystallization reaction occurs, such an additive may enable a stronger surface tension of a lower surface (a coating surface) when the solvent is quenched, and may form a crystal nucleus site, so that perovskite spreads at such a site to promote crystallization. Further, for perovskite ABX₃, taking FAPbl₃ as an example, due to susceptibility to oxygen, moisture, light, etc., a following equilibrium formula exists: FAPbI₃ 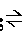 FAI·pBI₂ 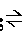 FAI + PbI₂ 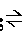 FAI + Pb⁰+ I₂, where I₂ easily sublimates to escape from the system, shifting the whole equilibrium towards the right side of the equilibrium formula to cause degradation of perovskite; when the system has weak reducibility, I₂ having a 0 valence can be reduced to I⁻, so that the whole reaction shifts towards the right, so as to inhibit degradation of perovskite, and the weak reducing group itself does not react with Pb²⁺ (exhibiting that the group is inert to Pb²⁺); and after the weakly reducing group is consumed, a corresponding product does not convert to its starting material, and thus the weakly reducing group is a consumable group, and is a sacrificial agent.

In some embodiments, the weakly reducing group comprises one or more of the group of: -NHNH-, -S-S-, -NHNH₂, sulfino, phosphinico, hydroxyphenyl, and hydroxynaphthyl; and
optionally, the weakly reducing group is selected from the group of: -NHNH-, -S-S-, -NHNH₂, sulfino, phosphinico, hydroxyphenyl, and hydroxynaphthyl.

In some embodiments, the weakly reducing group is selected from the group of: -NHNH-, -S-S-, -NHNH₂, sulfino, and phosphinico.

In some embodiments, the weakly reducing group comprises at least one of -NHNH-, and -S-S-.

In some embodiments, the weakly reducing group comprises at least one of -NHNH₂, sulfino, and phosphinico.

In some embodiments, the weakly reducing group comprises at least one of -NHNH-, and -NHNH₂.

In some embodiments, the weakly reducing group comprises a sulfino.

In some embodiments, the weakly reducing group comprises a sulfino.

In some embodiments, in one molecule of the first additive, the number of the weakly reducing group (which may be denoted as m) is one or more. In one molecule of the first additive, the number of the weak reducing group may be an integer selected from 1~10, further, the number of the weak reducing groups may be 1, 2, 3, 4 or 5; even further, the number of the weak reducing group may be 1, 2 or 3; and even further, the number of the weak reducing group may be 1 or 2. In some embodiments, m is 1. In some embodiments, m is 2.

When the number of the weakly reducing groups in the first additive molecule is more than 1, the types of the weakly reducing groups may be the same or different. In some embodiments, in one molecule of the first additive, there is one or more types of the weak reducing groups, for example, 1, 2, 3 or more, or 1, 2, 3, 4 or more. In some embodiments, in one molecule of the first additive, there are 1~5 types of the weak reducing groups, further 1~3. In some embodiments, in one molecule of the first additive, there is 1 type of the weak reducing group. In some embodiments, in one molecule of the first additive, there are 2 types of the weak reducing groups. In some embodiments, in one molecule of the first additive, there are 3 types of the weak reducing groups.

In some embodiments, in one molecule of the first additive, the number (which may be denoted as n) of halogen acid molecules is one or more. In one molecule of the first additive, the number of the halogen acid molecules may be an integer selected from 1~10; further, the number of the halogen acid molecules may be 1, 2, 3, 4 or 5; and even further, the number of the halogen acid molecules may be 1 or 2. In some embodiments, n is 1. In some embodiments, n is 2.

In some embodiments, the halogen in the hydrogen halide salt includes one or more of F, Cl, Br, and I;
optionally, any one halogen in the hydrogen halide salt is independently F, Cl, Br or I; and
further optionally, the hydrogen halide salt is a hydrochloride salt.

In some embodiments, the first additive is a small-molecule compound, and further is monodisperse. For a monodisperse small molecule compound, all molecules thereof have a molecular weight with a single value. Further, in some embodiments, the molecular weight of the first additive is less than 1000 Da, optionally the molecular weight of the first additive is less than or equal to 500 Da, further optionally the molecular weight of the first additive is less than or equal to 300 Da, and further optionally the molecular weight of the first additive is 100~300 Da, or the molecular weight may be any one molecular weight below or a range consisting of any two molecular weights selected from 100 Da, 150 Da, 200 Da, 250 Da, 300 Da, and the like, and for example, may also be selected from 150~300 Da .

In some embodiments, the number of the carbon atoms in the additive is 2~40; further optionally, the number of the carbon atoms in the first additive is 2~25; even further optionally, the number of the carbon atoms in the first additive is 2~20; even further optionally, the number of the carbon atoms in the first additive is 2~18; even further optionally, the number of the carbon atoms in the first additive is 2~15; even further optionally, the number of the carbon atoms in the first additive is 2~10; even further optionally, the number of the carbon atoms in the first additive is 2~8; and even further optionally, the number of the carbon atoms in the first additive is 2~6. Non-limiting examples of the number of carbon atoms in the first additive may be any one of: 2, 3, 4, 5, 6, 7, 8, 9, 10, 11, 12, 13, 14, 15, 16, 17, 18, 19, 20, 21, 22, 23, 24, 25, 30, and the like, and may be an interval consisting of any two of the above numbers, for example, 2~30, 3~30, 4~30, 6~30, 2~25, 3~25, 4~25, 6~25, 2~20, 3~20, 4~20, 6~20, 2~18, 3~18, 4~18, 6~18, and the like.

In the present application, unless otherwise specified, "the number of carbon atoms in the first additive" means the number of carbon atoms contained in one molecule of the first additive.

In some embodiments, the number of non-hydrogen atoms in the additive is 8~40; further optionally, the number of non-hydrogen atoms in the additive is 10~30; even further optionally, the number of non-hydrogen atoms in the additive is 10~25; even further optionally, the number of non-hydrogen atoms in the additive is 10~20; and further optionally, the number of non-hydrogen atoms in the additive is 10~18. Non-limiting examples of the number of non-hydrogen atoms in the first additive may be any one of 8, 9, 10, 11, 12, 13, 14, 15, 16, 17, 18, 19, 20, 21, 22, 23, 24, 25, 26, 28, 30, 35, and the like, and may be an interval consisting of any two of the above carbon atom numbers, for example, 8 ~ 30, 8~25, 8~20, 10~30, 10~25, 10~20, and the like.

In some embodiments, the first additive is a hydrogen halide salt of an organic compound containing at least one of - NHNH-, and -S-S-. The number of carbon atoms of the first additive may be as defined in any suitable embodiment herein. The number of non-hydrogen atoms of the first additive may be as defined in any suitable embodiment herein. For example, the first additive may be a hydrogen halide salt of a C₂₋₄₀ compound containing at least one of -NHNH- and -S-S-.

The aforementioned additive (a first additive) can be finely adjusted in terms of the type and amount of the weak reducing group, the type and amount of the halogen acid molecule, and the molecular weight, so that the additive can better interact with the perovskite precursor material, so as to better reduce defects of the perovskite thin film, and to better improve the service life of the perovskite cell. Here, the molecular weight of the first additive can be indirectly adjusted by adjusting the number of carbon atoms or the number of non-hydrogen atoms.

In some embodiments, the first additive is a hydrogen halide salt, its one molecule has n complexed HZ, and any one Z is independently a halogen atom. n is the number of halogen acid molecules in one molecule of the first additive, and reference may be made to the above definition. n may be an integer selected from 1~10, or 1, 2, 3, 4 or 5, or 1 or 2. In some embodiments, n=1. In some embodiments, n=2. In some embodiments, the first additive is a combination where n includes a plurality of different integers, corresponding to a combination of molecules of the first additive with different numbers of HZ. For example, n may be a combination of 1 and 2, in such a case, in the additive of the perovskite precursor solution, n is 1 in some molecules, and n is 2 in other molecules.

In some embodiments, in the first aspect, the perovskite precursor solution comprises a perovskite precursor material, a solvent and an additive; one molecule of the first additive contains m R₀₁, any one R₀₁ is independently a weakly reducing group; and the first additive is a hydrogen halide salt, its one molecule has n complexed HZ, any one Z is independently a halogen atom; wherein m and n are each independently a positive integer.

In some embodiments, in the first aspect, the perovskite precursor solution comprises the perovskite precursor material, solvent, and additive; one molecule of the first additive contains m R₀₁, any one R₀₁ is independently selected from any group of: - NHNH-, -S-S-, -NHNH₂, sulfino, phosphinico, hydroxyphenyl, and hydroxynaphthyl; and the first additive is a hydrogen halide salt, its one molecule has n complexed Hz, and any Z is independently a halogen atom, wherein m and n are each independently a positive integer.

In some embodiments, any one Z is independently F, Cl, Br, or I; and optionally, Z is Cl.

By adding the additive, a compound which is a hydrogen halide salt, containing the weakly reducing group (such as, but not limited to, one or more of -NHNH-, -S-S-, -NHNH₂, sulfino, phosphinico, hydroxyphenyl, hydroxynaphthyl, and the like) to the perovskite precursor solution, for the halogenated metal moiety BX₂ in the main component ABX₃ in the perovskite precursor solution and perovskite thin film, first, 0-valence X₂ can be reduced to X to increase the storage time of the perovskite precursor solution; second, its weak reducibility does not reduce B²⁺ to 0-valence B⁰; third, the additive can participate in crystallization reaction and be uniformly dispersed in the perovskite thin film, and can act as a sacrificial agent to extend the service life of the perovskite device; and fourth, formation of B⁰ and X₂ can be suppressed to reduce defects at an interface to improve the performance of the perovskite device. By forming a hydrogen halide salt (e.g., a hydrochloride salt salt, etc.), the additive can be more easily soluble in the perovskite precursor solution and more uniformly distributed in the perovskite thin film, and taking a chloride ion as an example, electron transport can also be enhanced, thereby improving the performance of the perovskite device.

In some embodiments, Z is chlorine. In this case, the first additive is a hydrochloride salt salt salt having a weakly reducing group. On one hand, a trace amount of Cl⁻ itself has an effect of promoting perovskite crystallization, with minimal side reaction, and does not participate in crystal lattices; and on the other hand, the salt is more easily soluble than other hydrogen halide salts. In addition, the hydrochloride salt is easily soluble in a perovskite precursor solution in a solvent system such as DMF (dimethylformamide), NMP (N-methylpyrrolidone), or DMSO (dimethylsulfoxide). The hydrochloride salt more easily participate in the perovskite system than a weak-acid salt, which more difficultlyparticipate in the perovskite system.

In some embodiments, the first additive has a structure represented by formula (1);

L(Z₁-R₁₁)ₚ₁·nHZ (1);

in formula (1), p1 is 0 or a positive integer; n is a positive integer; Z is halogen in the hydrogen halide salt; and n is the number of hydrohalic acid molecules in the hydrogen halide salt;
any one R₁₁ is independently the weakly reducing group and has a valence of 1(optionally any one R₁₁ is independently - NHNH₂, sulfino, phosphinico, hydroxyphenyl or hydroxynaphthyl; further optionally, any one R₁₁ is independently -NHNH₂, sulfino or phosphinico; and further optionally, any one R₁₁ is independently -NHNH₂ or sulfino);
when p1 is 0, the first additive is a hydrogen halide salt of an organic compound containing at least one of -NHNH-, and -S-S- (the molecular weight of the first additive may be directly or indirectly as defined above; further, the first additive may be a hydrogen halide salt of a C₂₋₄₀ compound containing at least one of -NHNH-, and -S-S-; and the number of carbon atoms in the first additive may be further as defined);
when p1 is a positive integer, L is alkyl, heteroalkyl, cycloalkyl, heterocycloalkyl, aryl, heteroaryl, substituted alkyl, substituted heteroalkyl, substituted cycloalkyl, substituted heterocycloalkyl, substituted aryl, or substituted heteroaryl in a valence of p1 and when L contains a heteroatom, any one heteroatom in L is independently a non-carbon atom or a non-hydrogen atom (and is further independently any one selected from N, S, P, O, and B);
L and all Z₁ contain a total of p2 divalent groups selected from -NH-NH-, and -S-S-, p2 being 0 or a positive integer; and (p1+p2)≥1;
Z₁ is a chemical bond, carbonyl, -CH₂-, -CH(Q₁)- or -(Q₂)C(Q₃)-, wherein Q₁, Q₂, and Q₃ are each independently selected from groups in the group of: alkyl, heteroalkyl, cycloalkyl, heterocycloalkyl, aryl, heteroaryl, substituted alkyl, substituted heteroalkyl, substituted cycloalkyl, substituted heterocycloalkyl, substituted aryl, and substituted heteroaryl; and when Z₁ contains a heteroatom, any one heteroatom in Z₁ is independently a non-carbon, or non-hydrogen atom (and may be further independently selected from any of N, S, P, O, and B); and optionally, Z₁ is -CH₂-, -CH(Q₁)- or -(Q₂)C(Q₃)-;
any one R₁₁ is independently directly linked to a carbon atom in Z₁ or L; and
in formula (1), alkyl, heteroalkyl, cycloalkyl, heterocycloalkyl, aryl, or heteroaryl in any one of the substituted alkyl, the substituted heteroalkyl, the substituted heterocycloalkyl, the substituted aryl, or the substituted heteroaryl contained in L, Z₁, Q₁, Q₂, or Q₃ is independently substituted with 1 or more groups selected from group G1 of: alkyl, heteroalkyl, cycloalkyl, heterocycloalkyl, aryl, heteroaryl, -CN, hydrazino, sulfino, phosphinico, -OH, -NH₂, -COOH, sulfo, phosphono, boronate, and halogen.

The weakly reducing group (which may be denoted as R₀₁) in the aforementioned additive related in the present application may be a monovalent group R₁₁, such as -NHNH₂, sulfino, phosphinico, hydroxyphenyl or hydroxynaphthyl, or may be a divalent group, such as -NH-NH-, -S-S-, or the like, and R₀₁ having two different valences may be any one described above, or a combination of the two. Here, monovalent R₀₁ is present at an end group, and is subjected to relatively small steric hindrance when it interact with other components in the precursor solution. Divalent R₀₁ also has an effect of a linking group, and the strength of the effect exerted by R₀₁ can be adjusted by flexibly adjusting its position in a molecule to adjust steric hindrance. The additive contains, in addition to the weakly reducing group R₀₁ and the halogen acid molecule HZ, a main moiety L(Z₁-)ₚ₁ which is an aromatic or aliphatic group and can produce a certain surface tension on a lower surface (the lower surface refers a coating surface) during quenching of a solvent, to promote to form crystal nuclei and to perform crystallization reaction. Z₁ may be absent; and Z₁ may also be a spacer group having a length of 1 spacer atom, and in this case, a structural property of Z₁ may be used to adjust weak reducibility of connected R₁₁. For example, when L is a benzene ring and R₁₁ is hydrazino-NHNH₂, Z₁ is -CH₂-, -CH(Q₁)- or -(Q₂)C(Q₃)- (further such as -CH₂-), facilitating to increase the stability of the perovskite precursor solution to prolong a useful period of a precursor.

In the present application, unless otherwise specified, R₁₁ is a monovalent weakly reducing group. Any one R₁₁ may independently be any of -NHNH₂, a sulfino, phosphinico, hydroxyphenyl, and hydroxynaphthyl; further may be any oen of -NHNH₂, sulfino, and phosphinico; and further may be -NHNH₂, or a sulfino. In some embodiments, R₁₁ may be hydrazino. In some embodiments, R₁₁ may be sulfino.

In some embodiments, Q₁, Q₂, and Q₃ are groups each independently selected from the group of: alkyl (optionally C₁₋₈ alkyl, further optionally C₁₋₆ alkyl, even further optionally C₁₋₄ alkyl, even further optionally C₁₋₃ alkyl, and even further optionally methyl), heteroalkyl (optionally alkoxy or secondary amino, further optionally C₁₋₈ alkoxy or C₁₋₈ alkylamino, even further optionally C₁₋₆ alkoxy or C₁₋₆ alkylamino, even further optionally C₁₋₄ alkoxy or C₁₋₄ alkylamino, even further optionally C₁₋₃ alkoxy or C₁₋₃ alkylamino, even further optionally -NHCH₃ or -NHCH₂CH₃, and even further optionally -NHCH₃), cycloalkyl (optionally C₃₋₈ cycloalkyl, further optionally C₃₋₆ cycloalkyl, and even further optionally cyclopropyl, cyclobutyl, cyclopentyl or cyclohexyl), heterocycloalkyl (optionally 3~8-membered heterocycloalkyl having 1 or 2 N atoms as ring atoms, further optionally 3~6-membered heterocycloalkyl having 1 or 2 N atoms as ring atoms, further optionally 3~4-membered heterocycloalkyl having 1 or 2 N atoms as ring atoms, further optionally piperidinyl, piperazinyl or monoaza cyclobutyl, and further optionally 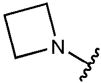 ), aryl (optionally C₆₋₁₂ aryl, further optionally C₆₋₁₀ aryl, and even further optionally phenyl, naphthyl or biphenyl), heteroaryl (6~12-membered aryl containing 1~6 ring nitrogen atoms, further optionally 6~12-membered aryl containing 1~4 ring nitrogen atoms, even further optionally 6~12-membered aryl containing 1~3 ring nitrogen atoms, further optionally 6~12-membered aryl containing 1 or 2 ring nitrogen atoms, even further optionally N₁₋₂ azaphenyl, even further optionally monoazaphenyl or diazazaphenyl, and even further optionally pyridyl, pyrimidyl, pyrazyl or pyridazyl) substituted alkyl (e.g., benzyl, 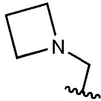 etc.), substituted heteroalkyl, substituted cycloalkyl, substituted heterocycloalkyl, substituted aryl, and substituted heteroaryl; and when Z₁ contains a heteroatom, any one of the heteroatoms in Z₁ is independently a non-carbon and non-hydrogen atom (which can further be independently selected from any one of N, S, P, O, and B).

In the present application, unless otherwise specified, the group G1 is a group consisting of one or more substituents which may include, but is not limited to, alkyl, heteroalkyl, cycloalkyl, heterocycloalkyl, aryl, heteroaryl, -CN, hydrazino, sulfinic, phosphinico , -OH, -NH₂, -COOH, sulfo, phosphono, boronate, and halogen.

In the present application, a "halogen" substituent, which may also be referred to as a halogen group, is considered to be within the scope of "group" in the present application.

In some embodiments, the group G1 comprises alkyl (optionally C₁₋₈ alkyl, further optionally C₁₋₆ alkyl, even further optionally C₁₋₄ alkyl, even further optionally C₁₋₃ alkyl, and even further optionally methyl), heteroalkyl (optionally alkoxy or secondary amino, further optionally C₁₋₈ alkoxy or C₁₋₈ alkylamino, even further optionally C₁₋₆ alkoxy or C₁₋₆ alkylamino, even further optionally C₁₋₄ alkoxy or C₁₋₄ alkylamino, even further optionally C₁₋₃ alkoxy or Ci-3 alkylamino, even further optionally -NHCH₃ or -NHCH₂CH₃, and even further optionally -NHCH₃), cycloalkyl (optionally C₃₋₈ cycloalkyl, further optionally C₃₋₆ cycloalkyl, and even further optionally cyclopropyl, cyclobutyl, cyclopentyl or cyclohexyl), heterocycloalkyl (optionally 3~8-membered heterocycloalkyl having 1 or 2 N atoms as ring atoms, further optionally 3~6-membered heterocycloalkyl having 1 or 2 N atoms as ring atoms, further optionally 3~4-membered heterocycloalkyl having 1 or 2 N atoms as ring atoms, further optionally piperidinyl, piperazinyl or monoaza cyclobutyl, and further optionally 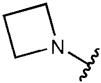 ), aryl (optionally C₆₋₁₂ aryl, further optionally C₆₋₁₀ aryl, and even further optionally phenyl, naphthyl or biphenyl), heteroaryl (6~12-membered aryl containing 1~6 ring nitrogen atoms, further optionally 6~12-membered aryl containing 1~4 ring nitrogen atoms, even further optionally 6~12-membered aryl containing 1~3 ring nitrogen atoms, further optionally 6~12-membered aryl containing 1 or 2 ring nitrogen atoms, even further optionally N₁₋₂ azaphenyl, even further optionally monoazaphenyl or diazazaphenyl, and even further optionally pyridyl, pyrimidyl, pyrazyl or pyridazyl), -CN, hydrazino, sulfino, phosphinico, -OH, -NH₂, -COOH, sulfo, phosphono, boronate, and halogen (optionally one or more of F, Cl, Br and I).

In some embodiments, the group G1 is group G2 comprising groups of: alkyl (optionally C₁₋₈ alkyl, further optionally C₁₋₆ alkyl, even further optionally C₁₋₄ alkyl, even further optionally C₁₋₃ alkyl, and even further optionally methyl), heteroalkyl (optionally alkoxy or secondary amino, further optionally C₁₋₈ alkoxy or C₁₋₈ alkylamino, even further optionally C₁₋₆ alkoxy or C₁₋₆ alkylamino, even further optionally C₁₋₄ alkoxy or C₁₋₄ alkylamino, even further optionally C₁₋₃ alkoxy or C₁₋₃ alkylamino, even further optionally - NHCH₃ or -NHCH₂CH₃, and even further optionally -NHCH₃), cycloalkyl (optionally C₃₋₈ cycloalkyl, further optionally C₃₋₆ cycloalkyl, and even further optionally cyclopropyl, cyclobutyl, cyclopentyl or cyclohexyl), heterocycloalkyl (optionally 3~8-membered heterocycloalkyl having 1 or 2 N atoms as ring atoms, further optionally 3~6-membered heterocycloalkyl having 1 or 2 N atoms as ring atoms, even further optionally 3~4-membered heterocycloalkyl having 1 or 2 N atoms as ring atoms, still further optionally piperidinyl, piperazinyl or monoazetidinyl, and further optionally 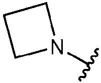 ), aryl (optionally C₆₋₁₂ aryl, further optionally C₆₋₁₀ aryl, and still optionally phenyl, naphthyl or biphenyl), and heteroaryl (6~12-membered aryl containing 1~6 ring nitrogen atoms, further optionally 6~12-membered aryl containing 1~4 ring nitrogen atoms, even further optionally 6~12-membered aryl containing 1~3 ring nitrogen atoms, even further optionally 6~12-membered aryl containing 1 or 2 ring nitrogen atoms, even further optionally N₁₋₂ azaphenyl, even further optionally monoazaphenyl or diazazaphenyl, and even further optionally pyridyl, pyrimidyl, pyrazyl or pyridazyl); optionally, the group G1 is the group G1^{b} comprising groups of: methyl, cyclopentyl, cyclopentylmethyl, phenyl, benzyl, biphenyl, methylphenyl, piperidinyl (optionally 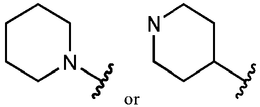 ), piperazinyl (optionally 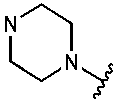 ), and monoaza cyclobutyl (optionally 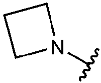 ); and optionally, the group G1 comprises methyl, cyclopentyl, phenyl, benzyl, biphenyl, methylphenyl, piperidinyl (optionally 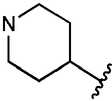 ), and monoaza cyclobutyl (optionally 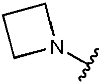 ).

In some embodiments, Q₁, Q₂, and Q₃ are each independently selected from the group of: C₁₋₆ alkyl, C₁₋₆ heteroalkyl, C₃₋₈ cycloalkyl, C₃₋₈ heterocycloalkyl, C₆₋₁₂ aryl, C₃₋₁₁ heteroaryl, substituted C₁₋₆ alkyl, substituted C₁₋₆ heteroalkyl, substituted C₃₋₈ cycloalkyl, substituted C₃₋₈ heterocycloalkyl, substituted C₆₋₁₂ aryl, and substituted C₃₋₁₁ heteroaryl; optionally, Q;₁, Q₂, and Q₃ are each independently selected from the group of: C₁₋₃ alkyl, C₁₋₃ heteroalkyl, C₃₋₆ cycloalkyl, C₃₋₆ heterocycloalkyl, C₆₋₁₀ aryl, C₃₋₉ heteroaryl, substituted C₁₋₃ alkyl, substituted C₁₋₃ heteroalkyl, substituted C₃₋₆ cycloalkyl, substituted C₃₋₆ heterocycloalkyl, substituted C₆₋₁₀ aryl, and substituted C₃₋₉ heteroaryl; and further optionally, Q₁, Q₂, and Q₃ are each independently selected from the group of: methyl, methoxy, cyclopropyl, cyclobutyl, cyclopentyl, cyclohexyl, piperidinyl (optionally 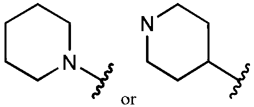 ), piperazinyl (optionally 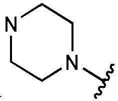 ), phenyl, naphthyl, pyridyl, pyrimidinyl, pyrazinyl, pyridazinyl, and N_{1~6} azanaphthyl, and a substituted form of any one of the foregoing groups; wherein, n₁, Q₂or Q₃ is a substituted form of any one of the foregoing groups, it may be independently substituted by 1 or more (optionally 1 or 2~5, further optionally 1, 2 or 3) groups selected from group G1^{a} of: methyl, cyclopentyl, phenyl, benzyl, methylphenyl, -Ph-Ph, N_{1~2} aza heterocycloalkyl (optionally monoaza C₃₋₈ cycloalkyl or diaza C₃₋₈ cycloalkyl, further optionally monoaza cyclobutyl, piperidinyl or piperazinyl, even further optionally monoaza cyclobutyl, and even further optionally 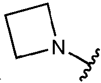 ), -CN, hydrazino, sulfino, phosphinico, -OH, -NH₂, -COOH, sulfo, phosphono, boronate, and halogen (optionally one or more of F, Cl, Br, and I).

In some embodiments, Z₁ is a chemical bond, carbonyl, or methylene (i.e., -CH₂-) or -CH(Q₁)-.

In some embodiments, Z₁) is a chemical bond, carbonyl, or methylene (i.e., -CH₂-).

In some embodiments, Z₁ is -CH₂-, -CH(Q₁)- or -(Q₂)C(Q₃)-.

In some embodiments, Z₁ is methylene.

In some embodiments, Z₁ is -CH(Q₁)- or -(Q₂)C(Q₃)-. Preferably, Q₁, Q₂ and Q₃ are substituents with small steric hindrance, so as not to affect perovskite crystallization. The substituent with small steric hindrance may have, for example, a ring structure having a ring number ≤3 (which may be 1, 2 or 3) and a chain structure having an atom number ≤12 (optionally, ≤10, further optionally, ≤8, and even further optionally, ≤6; which may be, for example, 1, 2, 3, 4, 5, 6, 7, 8, 9, 10, 11 or 12, or an interval consisting of any two integers above, for example, 1~10, 1~8, 1~6, etc.).

In some embodiments, Z₁ is -CH(Q₁)-.

In some embodiments, ZiR₁₁ is -C(=O)NHNH₂. According to the intermediate bridge theory, it may be relatively advantageous to prolong stable existence time of functional group -NHNH₂, and in addition, in a special case (for example, heating via light), it may be that coordination with B-site cations of a perovskite layer is performed to enhance stability.

In some embodiments, Z₁ is linked to an aromatic ring in L, and Z₁ may be or may not be carbonyl. In some of the embodiments, Z₁ is not carbonyl. In some of the embodiments, Z₁ is carbonyl. In some embodiments, R₁₁ is -NHNH₂.

In some embodiments, Z₁ is a chemical bond, -CH₂-, -CH(Q₁)- or -(Q₂)C(Q₃)-.

In some embodiments, Z₁ is a chemical bond.

In some embodiments, Z₁ is -CH₂-.

In some embodiments, Z₁ is -CH(Q₁); and further, Q₁ may be any one of methyl, phenyl, cyclopentyl, benzyl, -Ph-CH₃, 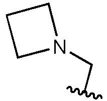 or the like.

In the present application, regarding -Ph-, the two linking sites may be of ortho-, meta- or para-position. In some embodiments, -Ph- is 1, 4-phenylene.

In some embodiments, Z₁ is any one of divalent groups of: methylene, -CH(CH₃)-, -CH(benzyl)-, -CH(cyclopentyl)-, - CH(-Ph-Ph)-, -CH(phenyl), -CH(-Ph-CH ₃)- and

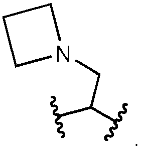

In the present application, L may correspond to a compound molecule or may be a part of a compound structure. When L is a compound molecule, p1 is 0, and an overall valence of L is 0, and in this case, L contains a polyvalent weak reducing group (e.g., a divalent weak reducing group). When L is a part of a compound structure, L may be a group consisting of multiple atoms and has p1 sites connectable to -Z₁-R₁₁.

L in the present application contains carbon atoms unless otherwise specified.

In some embodiments, L may be a chain structure or a ring-containing structure.

In the present application, unless otherwise specified, the "chain structure" does not include a ring structure, and may be linear or branched. A linear structure is, for example, (-CH₂-)ₙ, and a branched structure is, for example, -CH₂CH₂-CH(CH₃)-. The branched structure includes a main chain and a branched chain grafted to the main chain, and a branched chain portion includes a non-hydrogen atom and may include one or more of a carbon atom and a hetero atom.

In the present application, a ring in "a ring-containing structure" may be an aromatic ring, an aliphatic ring, or a combination thereof. Examples of the aromatic ring include a benzene ring, a naphthalene ring, a pyridine ring, a biphenyl ring, and the like. Examples of the aliphatic ring include a hexyl ring, a pentyl ring, a monoazabutyl ring, and the like.

In some embodiments, L may be an aliphatic structure or an aromatic structure.

In the present application, the meaning of "aliphatic structure" and "aromatic structure" may be understood by those skilled in the art. The "aromatic structure" may contain one or more of aryl and heteroaryl. The "aliphatic structure" does not contain any aromatic structure, and contains neither aryl nor heteroaryl.

In some embodiments, L is a chain structure; and further, the number of carbon atoms in L may be as previously defined. For example, L may be a C₂₋₂₀ chain structure; further optionally, L is a C₂₋₁₈ chain structure; even further optionally, L is a C₂₋₁₅ chain structure; even further optionally, L is a C₂₋₁₂ chain structure; even further optionally, L is a C₂₋₁₀ chain structure; and even further optionally, L is a C₂₋₈ chain structure.

In some embodiments, L is a ring-containing structure, further, the number of ring-membering atoms in L may be 3~25; optionally 3~20; further optionally 3~18; further optionally 5~18; further optionally 5~15; further optionally 5~12; further optionally 5, 6, 10, 11 or 12, and further optionally 6, 10, 11 or 12. The number of ring-membering atoms in L may be any one of: 3, 4, 5, 6, 7, 8, 9, 10, 11, 12, 13, 14, 15, 16, 17, 18, 19, 20, 21, 22, 23, 24, 25, and the like, and may be an interval consisting of any two ring atmo numbers selected from the above numbers, for example, 6~25, 6~20, 6~18, 6~15, 6~12, 6~10, and the like.

In the present application, unless otherwise specified, "ring-membering atom" refers to a non-hydrogen atom constituting a ring skeleton. For example, the number of non-hydrogen atoms is 6 for benzene, 10 for naphthalene, 6 for toluene, 12 for biphenyl, 6 for pyridine, 6 for hexane ring, 5 for pentane ring, 6 for piperidine, 6 for piperazine, and 6 for benzyl.

In the present application, "non-hydrogen atom" may be a carbon atom or a heteroatom. "Heteroatom" is a type of an atom other than carbon and hydrogen atoms, and is also referred to as "non-carbon and non-hydrogen atom". Non-limiting examples of heteroatoms may include N, S, P, O, B, and the like.

In some embodiments, the number of carbon atoms in L is 2~40, and may be any one of : 2, 3, 4, 5, 6, 7, 8, 9, 10, 11, 12, 13, 14, 15, 16, 17, 18, 19, 20, 21, 22, 23, 24, 25, 26, 27, 28, 29, 30, and the like, and may be an interval consisting of any two numbers selected from the above number, for example, 2~40, 2~35, 3~40, 3~35, 4~40, 4~35, 6~40, 6~35, 8~40, 8~35, and the like.

In some embodiments, the number of carbon atoms in L is 2~30, and may be any one of: 2, 3, 4, 5, 6, 7, 8, 9, 10, 11, 12, 13, 14, 15, 16, 17, 18, 19, 20, 21, 22, 23, 24, 25, 26, 27, 28, 29, 30, and the like, and may be an interval consisting of any two numbers selected from the above number, for example, 2~25, 2~20, 2~18, 2~15, 2~12, 2~10, 2~8, 3~30, 3~25, 3~20, 3~18, 3~15, 3~12, 3~10, 4~30, 4~25, 4~20, 4~18, 4~15, 4~12, 4~10, 6~30, 6~25, 6~20, 6~18, 6~15, 6~12, 6~10, and the like.

In some embodiments, L is a ring-containing structure, and further, the number of carbon atoms in L may be as defined in any one of the embodiments above. For example, L may be a ring-containing structure containing 2~40 carbon atoms; and optionally, the number of carbon atoms in L is 3~30; further optionally 3~25; even further optionally 3~20; further optionally 3~18; and further optionally 4~18.

In some embodiments, the number of non-hydrogen atoms in L is 2~35, and may be any one of: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, and the like, and may be an interval consisting of any two numbers selected from the above number, for example, 2~30, 2~25, 2~20, 2~18, 2~15, 2~12, 2~10, 2~8, 4~35, 4~30, 4~25, 4~20, 4~18, 4~15, 4~12, 4~10, 6~35, 6~30, 6~25, 6~20, 6~18, 6~15, 6~12, 6~10, and the like.

In some embodiments, L is a ring-containing structure, and further, the ring in L may be an aromatic ring, an aliphatic ring, or a combination thereof.

In some embodiments, the ring in L comprises an aromatic ring, and further, the number of carbon atoms in L may be as previously defined. For example, the number may be 3~40; further optionally, the number of carbon atoms in L is 3~30; even further optionally, the number of carbon atoms in L is 3~25; even further optionally 3~20; even further optionally 3~18; even further optionally 5~18; even further optionally 5~15; even further optionally 5~12; even further optionally 5, 6, 10, 11, or 12; and even further optionally 6, 10, 11 or 12.

In some embodiments, the ring in L comprises an aliphatic ring, and further, the number of carbon atoms in L may be as previously defined, and may be for example, 2~40; optionally, the number of carbon atoms in L is 3~40; even further optionally, the number of carbon atoms in L is 3~30; even further optionally, the number of carbon atoms in L is 3~25; even further optionally 3~20; even further optionally 3~18; even further optionally 5~18; even further optionally 5~15; even further optionally 5~12; even further optionally 5, 6, 10, 11, or 12; and even further optionally 6, 10, 11 or 12;

In some embodiments, L is an aliphatic structure, and further, the number of carbon atoms in L may be as previously defined. For example, the number of carbon atoms in L may be 2~25; more preferably 2~20; even more preferably 2~18; even more preferably 2~15; even more preferably 2~12; even more preferably 2~10 and even more preferably 2~8.

In some embodiments, L is an aromatic structure, and further, the number of carbon atoms in L may be as previously defined. For example, L may comprise one or more of aryl, heteroaryl, substituted aryl and substituted heteroaryl; optionally, L comprises one or more of C₆₋₂₀ aryl, C₄₋₂₀ heteroaryl, substituted C₆₋₂₀ aryl and substituted C₄₋₂₀ heteroaryl; and further optionally, L comprises one or more of C₆₋₁₀ aryl, C₄₋₁₀ heteroaryl, substituted C₆₋₁₀ aryl, and substituted C₄₋₁₀ heteroaryl.

When p1=0, the structure of the first additive is L·ₙHZ, and in this case, the number of monovalent weakly reducing group R₁₁ is 0, and L contains a polyvalent weakly reducing group, such as a divalent weakly reducing group. Further, for example, when p1=0, L may contain one or more divalent groups selected from the group of: -NH-NH-, and -S-S-. It may be understood that when L contains no -NH-NH- and only contain, a weakly reducing group, -S-S-, an atom or group capable of donating lone pair electrons is present in L, and thus, it can be complexed with a molecule of a hydrogen halide salt.

In some embodiments, p1 is a positive integer, further optionally, a positive integer from 1~10; optionally p1 is 1, 2, 3, 4 or 5; and optionally p1 is 1 or 2.

In some embodiments, p1 is 0, 1, or 2.

When p1 is a positive integer (i.e. ≥1), the first additive comprises at least one monovalent weakly reducing group.

In some embodiments, p1 is a positive integer, L is C₂₋₁₈ alkyl, C₂₋₁₈ heteroalkyl, C₃₋₂₀ cycloalkyl, C₃₋₂₀ heterocycloalkyl, C₆₋₂₀ aryl, C₃₋₂₀ heteroaryl, substituted C₂₋₁₈ alkyl, substituted C₂₋₁₈ heteroalkyl, substituted C₃₋₂₀ cycloalkyl, substituted C₃₋₂₀ heterocycloalkyl, substituted C₆₋₂₀ aryl or substituted C₃₋₂₀ heteroaryl having a valance of p1; optionally, L is C₂₋₁₂ alkyl, C₂₋₁₂ heteroalkyl, C₃₋₁₂ cycloalkyl, C₃₋₁₂ heterocycloalkyl, C₆₋₁₅ aryl, C₃₋₁₅ heteroaryl, substituted C₂₋₁₂ alkyl, substituted C₂₋₁₂ heteroalkyl, substituted C₃₋₁₂ cycloalkyl, substituted C₃₋₁₂ heterocycloalkyl, substituted C₆₋₁₅ aryl or substituted C₃₋₁₅ heteroaryl having a valance of pl; further optionally, L is C₂₋₁₀ alkyl, C₂₋₁₀ heteroalkyl, C₅₋₁₀ cycloalkyl, C₄₋₁₀ heterocycloalkyl, C₆₋₁₀ aryl, C₃₋₁₀ heteroaryl, substituted C₂₋₁₀ alkyl, substituted C₂₋₁₀ heteroalkyl, substituted C₅₋₁₀ cycloalkyl, substituted C₄₋₁₀ heterocycloalkyl, substituted C₆₋₁₀ aryl or substituted C₃₋₁₀ heteroaryl having a valance of p1; and even further optionally, L is a linear chain structure; wherein when L contains a heteroatom, any one heteroatom in L is independently a non-carbon or non-hydrogen atom, and further independently selected from any one of N, S, P, O, and B.

In some embodiments, L is phenyl, naphthyl, cyclopentyl, cyclohexyl, n-octyl, n-nonyl, n-decyl, n-undecyl, n-dodecyl, n-tridecyl, n-tetradecyl, n-pentadecyl, n-hexadecyl, heptadecyl, n-octadecyl, pyridyl, piperidinyl, piperazinyl, N-methylpiperidinyl, methylphenyl, dimethylphenyl, biphenyl, naphthyl-substituted phenyl, aminophenyl, C₂₋₁₈ alkyl with 1 or more sulfo substituents (optionally ethyl with a monosulfo substituent, and further optionally 2-sulfoethyl), C₂₋₁₈ alkyl substituted with 1 or more NH₂ (optionally C₂₋₁₈ alkyl substituted with one -NH₂, further optionally aminobutyl, and even further optionally 4-aminobutyl), C₂₋₁₈ alkyl substituted with 1 or more -(O=)P(OH)₂ (optionally C₂₋₁₂ alkyl substituted with 1 -(O=)P(OH)₂, further optionally phosphonopropyl, and even further optionally 3-phosphonopropyl), C₂₋₁₈ alkyl substituted by 1 or more - (O=)PR₀₂(OH) (optionally C₂₋₁₂ alkyl substituted with 1 -(O=)PR₀₂(OH), optionally C₂₋₈ alkyl substituted with 1 -(O=)PR₀₂(OH), even further optionally (CH₃)(OH)(R₀₂)P(=O)-C₂₋₈ alkylene -, even further optionally (CH₃)(OH)(R₀₂)P(=O)-(CH₂)_{2~8}-, and even further optionally (CH₃)(OH)(R₀₂)P(=O)-(CH₂)₃-), and R₀₂ is alkyl (optionally C₁₋₆ alkyl, further optionally C₁₋₃ alkyl, and even further optionally methyl), C₂₋₁₈ alkyl having 1 or more - (O=)PR₀₂(OH) and 1 or more NH₂- substituents (optionally C₂₋₁₈ alkyl having 1 -(O=)PR₀₂(OH) and 1 NH₂ substituent, further optionally C₂₋₁₂ alkyl having 1 -(O=)PR₀₂(OH) and 1 NH₂ substituent, even further optionally C₂₋₈ alkyl having 1 -(O=)PR₀₂(OH) and 1 NH₂ substituent, and even further optionally (CH₃)(OH)(R₀₂)P(=O)-CH₂CH₂CH(NH₂)-), sulfophenyl, carboxyphenyl, phosphonophenyl, and boratophenyl.

In some embodiments, L is an aliphatic structure and Z₁ is a chemical bond or carbonyl (Z₁ optionally is a chemical bond).

In some embodiments, L is an aromatic structure and Z₁ is not a chemical bond.

In some embodiments, when p1 is a positive integer, and R₁₁is -NHNH₂, at least one R₁₁ is linked to -CH₂-, -CH(Q₁)- or -(Q₂)C(Q₃)-; and Q₁, Q₂, and Q₃ may be defined with referred to structures in any of the embodiments herein, and for example, Q₁, Q₂, and Q₃ may each independently be the group selected from the group of: alkyl, heteroalkyl, cycloalkyl, heterocycloalkyl, aryl, heteroaryl, substituted alkyl, substituted heteroalkyl, substituted cycloalkyl, substituted heterocycloalkyl, and substituted aryl and substituted heteroaryl;
optionally, Q₁, Q₂, and Q₃ are each independently selected from the group of: alkyl, aralkyl, heteroaralkyl, substituted alkyl, substituted aralkyl, and substituted heteroaralkyl, wherein Q₁, Q₂, or Q₃, when is any one of the substituted forms described above, may be independently substituted with 1 or more groups selected from the group G1.

Improvement of the stability of the perovskite precursor solution is facilitated to prolong the useful period of the precursor in a case where a spacer group (e.g., alkylene or substituted alkylene, further such as -CH₂-, -CH(Q₁)- or -(Q₂)C(Q₃)-, and even further such as -CH₂-) is between hydrazino and a benzene ring with respect to a case where hydrazino is directly linked to an aromatic ring (e.g., a benzene ring). Taking a benzylhydrazine hydrochloride salt and a phenylhydrazine hydrochloride salt as examples, N in hydrazino has a valence of -1 and tends to lose an electron, and therefore, when N in hydrazino in the phenylhydrazine hydrochloride salt is directly linked to a benzene ring, a strong electron-withdrawing ability of the benzene ring aggravates a process of losing electrons from N in hydrazino, so that the phenylhydrazine hydrochloride salt exists as a sacrificial agent in the perovskite for a relatively short period of time; and in the benzylhydrazine hydrochloride salt, by separating hydrazino from a benzene ring by a methylene structure, the hydrazino is directly linked to a carbon atom of methylene, and the methylene tends to donate an electrons to transfer an electron to the hydrazino, so that the stability of the benzylhydrazine hydrochloride salt is increased, facilitating to increase the stability of the perovskite precursor solution to prolong the useful period of the precursor.

In addition, when a spacer group is present between hydrazino and an aromatic ring in L, it is facilitated to promote crystallization reaction, and lattice distortion can also be avoided. The spacer group may increase steric hindrance, so that it is difficult for the first additive to enter ABX₃ lattices in perovskite, and thus lattice distortion can also be avoided, and since the first additive is dissolved in the perovskite precursor solution due to the like-dissolves-like rule, it can be uniformly dispersed in the perovskite thin film to induce, to some extent, nucleus formation during crystallization.

When the spacer group is present between the hydrazino and the aromatic ring in L, a lifetime of a perovskite device can also be prolonged. Degradation of perovskite is mainly caused by degradation of A⁺ and BX₂ in a perovskite layer, a hydrochloride salt can suppress degradation of a site-A cation, and in this case, an aromatic hydrazino compound containing a spacer group can suppress degradation of a halogen anion, and also has a better stability.

L may contain an aromatic ring or be an aliphatic chain, and may produce different steric-hindrance and electron-donating abilities, leading to different levels of reaction. Given an identical number of carbon atoms, an aromatic ring-containing structure is preferred to an aliphatic chain, on one hand, when the aromatic ring-containing structure is contained, it may not be involved in perovskite crystal lattices, and on the other hand, the aliphatic chain is more hydrophobic than the aromatic ring, and possibly degrades crystal quality.

In some embodiments, when p1 is a positive integer, Z₁ is directly linked to the aromatic ring in L, and when corresponding R₁₁ is hydrazino, Z₁ is not a chemical bond, and further, R₁₁ is -CH₂-, -CH(Q₁)- or -(Q₂)C(Q₃)-. The definitions of Q₁, Q₂, and Q₃ may refer to the structures in any of the embodiments herein, for example, Q₁, Q₂, and Q₃ may be the group each independently selected from the group of: alkyl, heteroalkyl, cycloalkyl, heterocycloalkyl, aryl, heteroaryl, substituted alkyl, substituted heteroalkyl, substituted cycloalkyl, substituted heterocycloalkyl, substituted aryl, and substituted heteroaryl; and optionally, Q₁, Q₂, and Q₃ may be the group each independently selected from the group of: alkyl, aralkyl, heteroaralkyl, substituted alkyl, substituted aralkyl, and substituted heteroaralkyl, wherein Q₁, Q₂ or Q₃, when it is in any one of the above substituted forms, may be independently substituted with 1 or more groups selected from the group G1.

In some embodiments, in the first additive, p1 is 1, R₁₁ is hydrazino, and R₁₁ is not directly linked to phenyl. An example of R₁₁ directly linked to phenyl is a benzylhydrazine hydrochloride salt.

In some embodiments, L includes a sulfur-containing group such as -SH. In this case, the number of -SH may be ≦5, for example 1, 2, 3, 4 or 5. The complexing ability of the perovskite precursor solution may be promoted, the stability of B²⁺ during perovskite crystallization may be promoted, and the dissociation thereof in long-term use may be inhibited.

In some embodiments, p1 is a positive integer, R₁₁ is -NHNH₂, and optionally, at least one Z₁ is not a chemical bond.

In some embodiments, p1 is a positive integer, at least one R₁₁ is -NHNH₂, any one Z₁ linked to -NHNH₂ is independently -CH₂-, -CH(Q₁)- or -(Q₂)C(Q₃)-; and L is an aromatic group, and at least one -Z₁-R₁₁ is linked to the aromatic ring in L;

The definitions of Q₁, Q₂, and Q₃ may refer to the structures in any of the embodiments herein, for example, Q₁, Q₂, and Q₃ may be the group each independently selected from the group of: alkyl, heteroalkyl, cycloalkyl, heterocycloalkyl, aryl, heteroaryl, substituted alkyl, substituted heteroalkyl, substituted cycloalkyl, substituted heterocycloalkyl, substituted aryl, and substituted heteroaryl; and optionally, Q₁, Q₂, and Q₃ may be the group each independently selected from the group of: alkyl, aralkyl, heteroaralkyl, substituted alkyl, substituted aralkyl, and substituted heteroaralkyl, wherein Q₁, Q₂ or Q₃, when it is in any one of the above substituted forms, may be independently substituted with 1 or more groups selected from the group G1.

In some embodiments, L is aryl, heteroaryl, substituted aryl, or substituted heteroaryl; and
optionally, L is any one of C₆₋₂₀ aryl, C₄₋₂₀ heteroaryl, substituted C₆₋₂₀ aryl, and substituted C₄₋₂₀ heteroaryl; and further optionally, L is any one of C₆₋₁₀ aryl, C₄₋₁₀ heteroaryl, substituted C₆₋₁₀ aryl, and substituted C₄₋₁₀ heteroaryl;
wherein L, when it is any one of the above substituted forms, is independently substituted with 1 or more groups selected from the group G1.

When the first additive contains an aromatic ring, it may not be involved in perovskite lattices, and on the other hand, generally has hydrophobicity weaker than that of an aliphatic chain, so that crystal quality can be increased.

In some embodiments, the first additive has a structure represented by formula (11):

Ar(-Z₂₁-R₁₁)ₚ₁·nHCl (11)

in formula (11), p1 is a positive integer, and Z₂₁ is -CH₂-, -CH(Q₁)- or -(Q₂)C(Q₃)-.

In some embodiments, the first additive has a structure represented by formula (12):

Ar(-R₁₁)ₚ₁·nHCl (12)

in formula (12), p1 is a positive integer, and R₁₁ is sulfino, and phosphinico. In some embodiments, the first additive has a structure represented by formula (13): 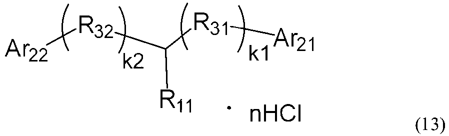
in formula (13), k1, and k2 are each independently 0 or 1; R₃₁, and R₃₂ are each independently alkylene; optionally, R₃₁, and R₃₂ are each independently C₂₋₂₀ alkylene, further optionally, R₃₁, and R₃₂ are each independently C₂₋₁₆ alkylene, even further optionally, R₃₁, and R₃₂ are each independently C₂₋₁₂ alkylene, even further optionally, R₃₁, and R₃₂ are each independently C₂₋₁₀ alkylene, even further optionally, R₃₁, and R₃₂ are each independently C₂₋₈ alkylene, even further optionally, R₃₁, and R₃₂ are each independently C₂₋₆ alkylene, and even further optionally, R₃₁, and R₃₂ are each independently C₂₋₄ alkylene.

In some embodiments, the first additive has a structure represented by formula (14):

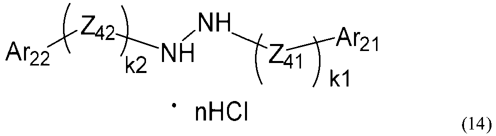

In some embodiments, the first additive has a structure represented by formula (15): 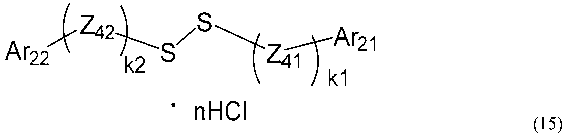 in formula (14) and formula (15), kl, and k2 are each independently 0 or 1, and Z₄₁, and Z₄₂ are each independently alkylene. Optionally, Z₄₁, and Z₄₂ are each independently C₂₋₁₅ alkylene, further optionally, Z₄₁, and Z₄₂ are each independently C₁₋₁₂ alkylene, even further optionally, Z₄₁, and Z₄₂ are each independently C₁₋₁₀ alkylene, even further optionally, Z₄₁, and Z₄₂ are each independently C₁₋₈ alkylene, even further optionally, Z₄₁, and Z₄₂ are each independently C₁₋₆ alkylene, even further optionally, Z₄₁, and Z₄₂ are each independently C₁₋₄ alkylene, and even further optionally, Z₄₁, and Z₄₂ are each independently methylene, ethylene, propylene or butylene.

In some embodiments, Ar, Ar₂₁, and Ar₂₂ are each independently an aromatic group. In some of the embodiments, the number of ring-membering atoms of Ar, Ar₂₁, and Ar₂₂ is each optionally 5~15, further optionally 5~12, further optionally 5~10 or 12, further optionally 5, 6, 10, or 12. In other embodiments, Ar, Ar₂₁, and Ar₂₂ are each independently aryl, heteroaryl, substituted aryl, or substituted heteroaryl, wherein aryl, and heteroaryl in the substituted aryl or the substituted heteroaryl are independently substituted with one or more groups selected from the group G1. When a hetero atom is contained, any hetero atom contained in any one of Ar, Ar₂₁ and Ar₂₂ is independently a non-carbon and non-hydrogen atom, and further independently any one selected from N, S, P, O and B.

In some embodiments, Ar, Ar₂₁, and Ar₂₂ each independently contain no more than 3 (e.g., 1, 2, or 3) rings.

In some embodiments, any one of "cycloalkyl", "heterocycloalkyl", "aryl" and "heteroaryl " related herein independently contains no more than 3 (e.g., 1, 2, or 3) rings.

In some embodiments, any one of "substituted cycloalkyl", "substituted heterocycloalkyl", "substituted aryl", and "substituted heteroaryl" related in the present applicaiton independently contains no more than 3 (e.g., 1, 2, or 3) rings.

In some embodiments, L contains a weakly acidic group such as -COOH, sulfo, phosphono, and boronate. Taking -COOH as an example, -COOH may be weakly dissociated into H⁺ and COO⁻, H⁺ may inhibit degradation of A⁺ cations, and COO⁻ has a strong ability to coordinate with B, thereby enhancing bulk stability of perovskite and promoting long-term stability of perovskite. As noted, introduction of a weakly acidic group does not substitute for salification of halogen acid molecules, salification of a halogen acid (for example, salification of hydrochloric acid) can significantly improve solubility; and salification with only a weakly acidic group and without halogen acid molecule may cause that the first additive can not achieve intended function or achieve a poor effect possibly because it can not be involved in the perovskite thin film due to a pre-filtration step or the like, or it is not unevenly distributed in the perovskite thin film.

In some embodiments, the first additive contains j second functional groups W₁₁, and W₁₁ is any one selected from the group of: -COOH, sulfo, phosphono, and boronate. In one molecule of the additive, the number j of the second functional group W₁₁ may be 1 or more; optionally j is 1, 2 or 3; and further optionally j is 1 or 2.

By introducing a weakly acidic group (such as, but not limited to, one or more of -COOH, sulfo, phosphono, and boronate, etc.) into the first additive, addition-elimination reaction of A (A is an organic cation) in ABX₃ can be suppressed, to prolong a service life of a perovskite device. In addition, when it is consumed in terms of acidity, it itself may be supplemented to a perovskite system to enhance a stability of a perovskite structure. Electron transport may also be enhanced when the weak acid group is coordinated with a B-site cation. A trace amount of an acid radical anion contained in W₁₁ may promote seed crystal formation and enhance seed crystal quantitative formation, and, when perovskite is annealed, such a substance is easy to exist from a lattice system thereof, so that perovskite is first subjected to a stable α-phase transition.

In some embodiments, the first additive has a structure represented by formula (2):

(W₁₁)ⱼL₀(R₀₂)ₘ₁·nHCl (2)

in formula (2), j is a positive integer, ml is a positive integer; any one R₀₂ is independently the weakly reducing group (optionally, any one R₀₂ is independently selected from the group of: a group containing -NHNH-, -NHNH₂, sulfino, and phosphinico); and Lo is hydrocarbyl or heterohydrocarbyl with a valence of m1+j, the heterohydrocarbyl containing 0, 1 or more substituents selected from the group of: -CN, -OH, halogen (optionally one or more of F, Cl, Br and I), -NH₂, -SH, -CF₃, and -COOH.

In some embodiments, the first additive has a structure represented by formula (21):

(W₁₁)ⱼ₁L₀₁(Z₁-R₁₁)ₚ₁·nHCl (21)

in formula (21), p1, and j1 are each independently a positive integer, any one Z₁, and any one R₁₁ are each independently as defined as above; L₀₁(Z₁₋)ₚ₁ contains p2 the divalent weakly reducing groups (R₀₁), and j2 W₁₁, wherein p2, and j2 are each independently 0 or a positive integer; and L₀₁ is hydrocarbyl or heterohydrocarbyl having a valence of p1+j1, the heterohydrocarbyl containing 0, 1 or more substituents selected from the group of: -CN, -OH, and halogen (optionally one or more of F, Cl, Br and I), - NH₂, -SH, -CF₃, and -COOH.

In some embodiments, the first additive has a structure represented by formula (22):

(W₁₁)ⱼ₁Ar₀₁(-Z₃₁-R₁₁)ₚ₁·nHCl (22)

in formula (22), p1, j1, any one R₁₁, and any one W_{1,} are each independently as defined in formula (21); and Ar_{0 1}is a divalent aromatic group; wherein the divalent aromatic group contains 0, 1, or more substituents selected from the group of: -CN, -OH, -NH₂, -SH, -CF₃, -COOH, and halogen; Z₃₁ is -CH₂-, -CH(Q₁)- or -(Q₂)C(Q₃)-; and Q₁, Q₂, and Q₃ are each independently a group selected from group G3 of: alkyl (optionally C₁₋₆ alkyl, further optionally C₁₋₃ alkyl, and even further optionally methyl), and heteroalkyl (optionally alkoxy or alkylamino, further optionally C₁₋₆ alkoxy or C₁₋₆ alkylamino, even further optionally C₁₋₃ alkoxy or C₁₋₃ alkylamino, even further optionally methoxy, ethoxy, -NHCH₃ or -NHCH₂CH₃, even further optionally -NHCH₃ or -NHCH₂CH₃, and even further optionally -NHCH₃); wherein the heterohydrocarbyl contains 0, 1 or more substituents selected from the group of: -CN, - OH, halogen (optionally one or more of F, Cl, Br and I), -NH₂, -SH, -CF₃, and -COOH.

In some embodiments, the first additive has a structure represented by formula (23):

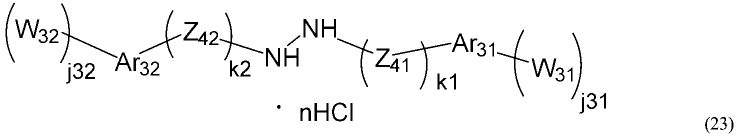

In some embodiments, the first additive has a structure represented by formula (24): 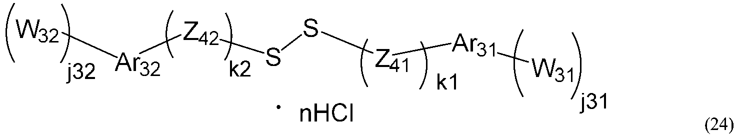
in formula (23), and formula (24), k1, k2, Z₄₁, and Z₄₂ are each independently as defined in formula (14); j31 and j32 are each independently 0 or a positive integer, with (j31 + j32) ≥1; and W₃₁, and W₃₂ are each independently any one group selected from the group of: -COOH, sulfo, phosphono, boronate, -NH ₂, and -SH;
and Ar₃₁, and A₃₂ are each independently a divalent aromatic group; wherein the divalent aromatic group contains 0, 1 or more substituents selected from the group of: -CN, -OH, amino, mercapto, and halogen (optionally one or more of F, Cl, Br, and I).

The aforementioned effect of the weakly acidic group can be more flexibly enabled by adjusting features, such as a type, a number, and a position of the weakly acidic group.

In some embodiments, the number of rings in the first additive is ≦ 3, and may be 1, 2, or 3.

In some embodiments, the first additive includes any any one or more of the following compounds: 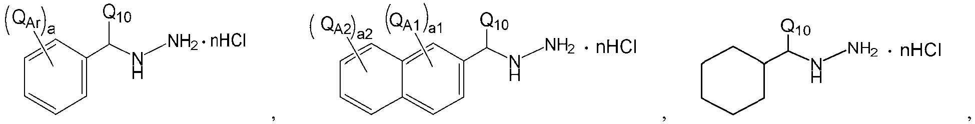 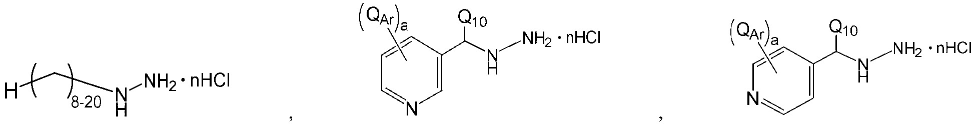 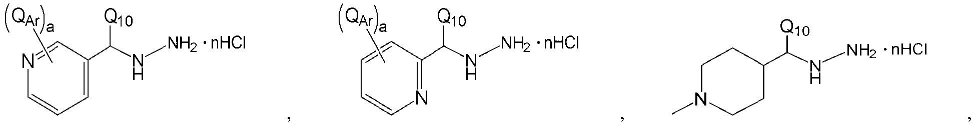 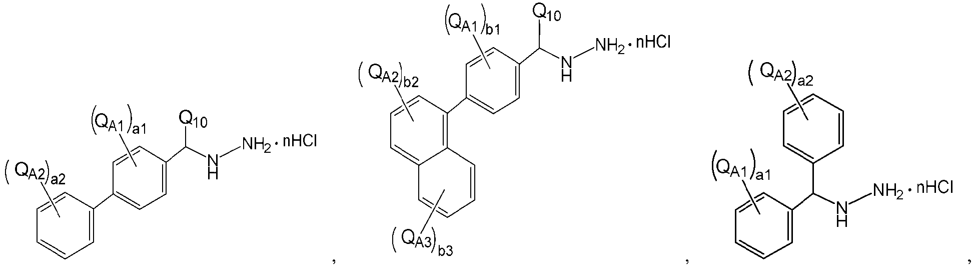 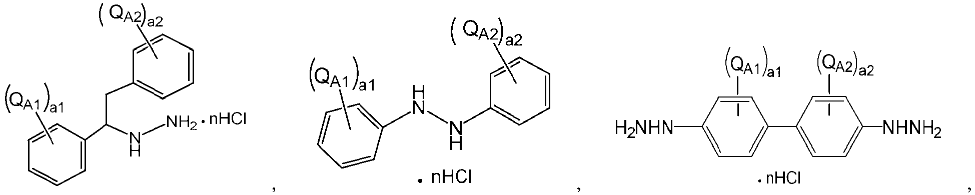 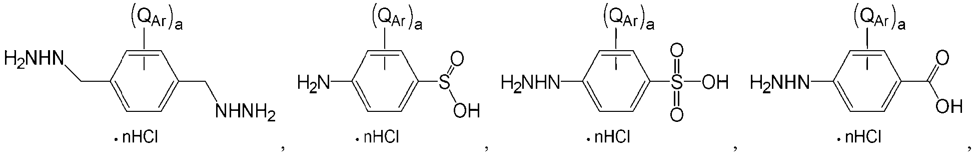 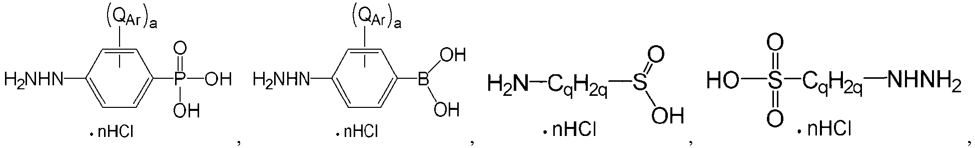 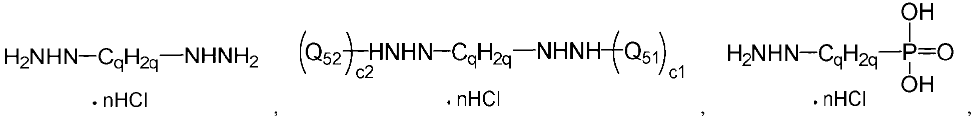 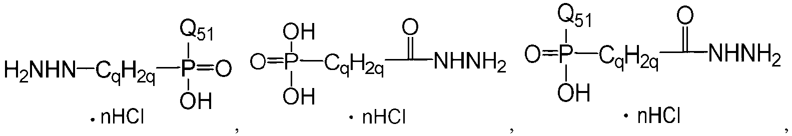 and a case where any one of the aforementioned compound salts is substituted with a substituent selected from group G1;
wherein n is as previously defined, n may be a positive integer, further, n may be an integer selected from 1~10; further optionally, n may be 1, 2, 3, 4 or 5; and further optionally, n may be 1 or 2;
Q_{Ar} is any one group selected from the group G1; and a is 0 or a positive integer (optionally is 0, 1 or 2);
Q₁₀ is H or Q₁; optionally, Q₁₀ is H; and further optionally, Q₁₀ is Qi;
al and a2 are each 0 or a positive integer, with a1+a2≥1;
b1, b2, and b3 are each 0 or a positive integer, with b1+b2+b2≥1, optionally, b2+b3≥1, and further optionally b1=0;
Q_{A1}, Q_{A2}, and Q_{A3}are each independently Q_{Ar};
Q is an integer selected from 2~20, optionally an integer of 2~18, further optionally an integer of 2~16, even further optionally an integer of 2~12, even further optionally an integer of 2~10, even further optionally an integer of 2~8, and even further optionally an integer of 2, 3, 4, 5, 6 or 7;
c1 and c2 are each independently 0 or 1, optionally c1+c2≥1; and
Q₅₁, and Q₅₂ are each independently alkyl (optionally C₁₋₆ alkyl, further optionally C₁₋₃ alkyl, and even further optionally methyl).

In some embodiments, the first additive includes any any one or more of the following compounds: 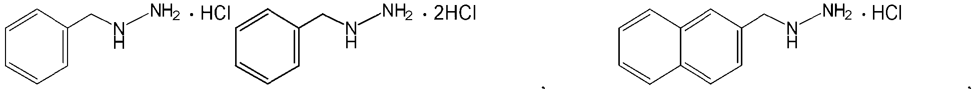 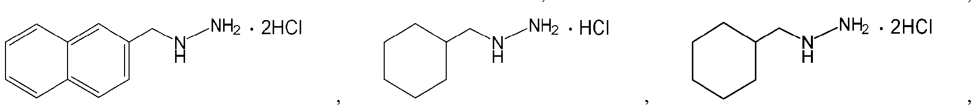 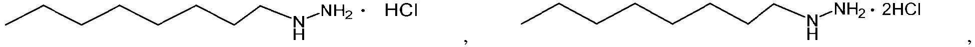 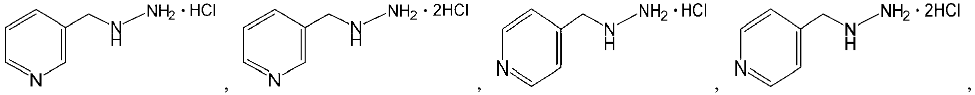 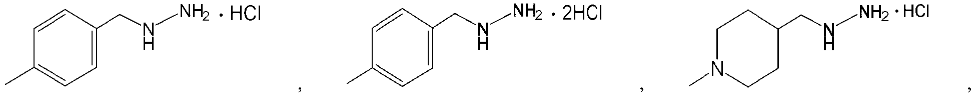 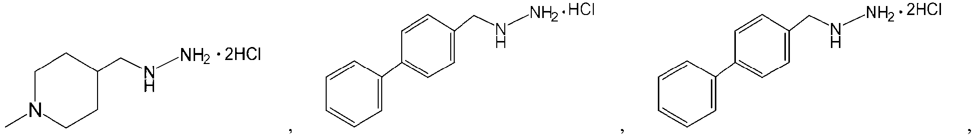 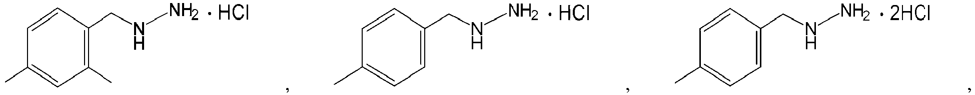 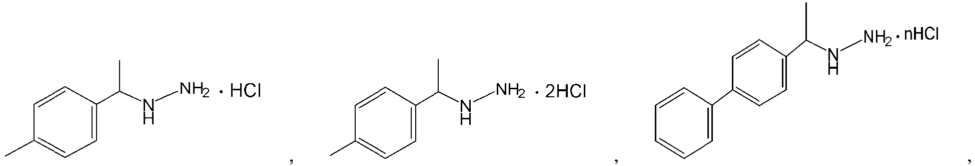 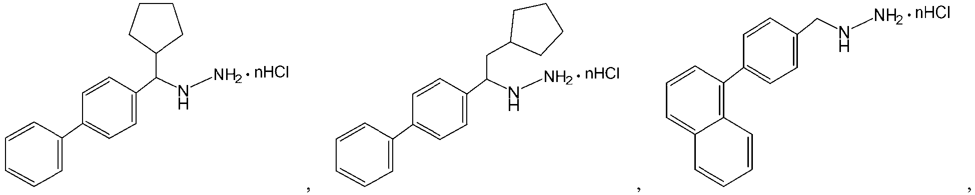 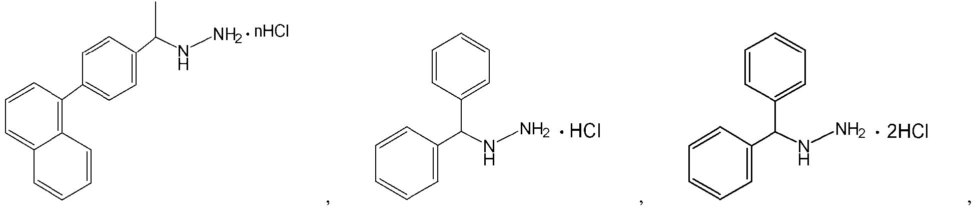 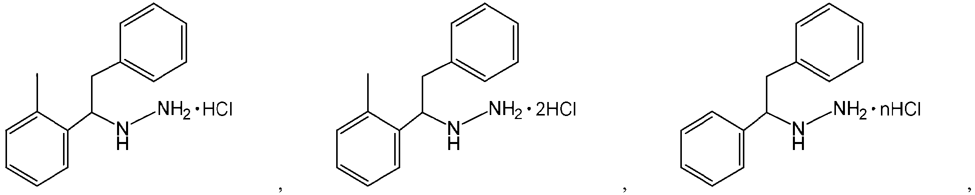 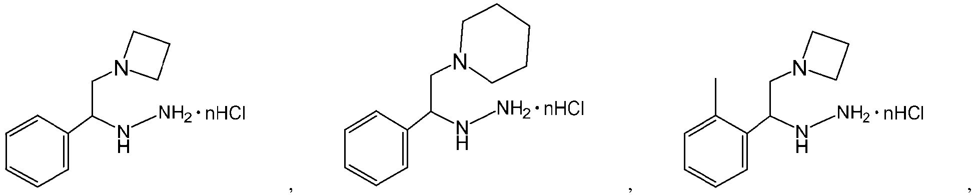 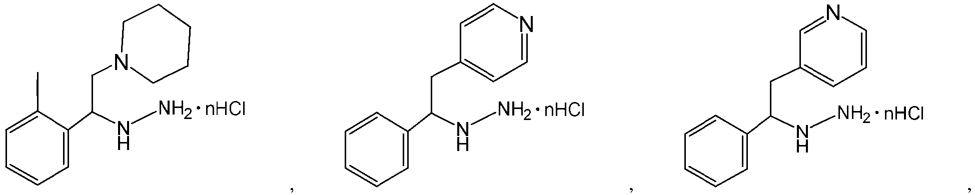 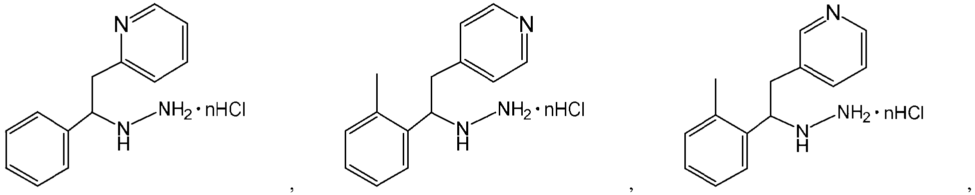 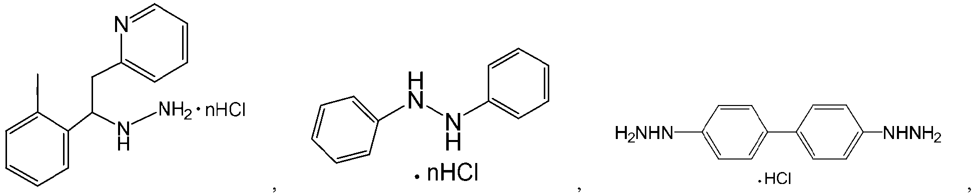 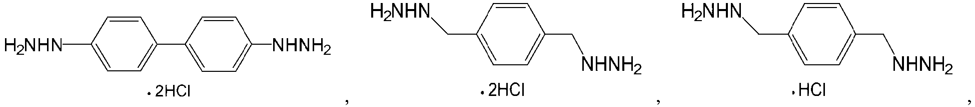 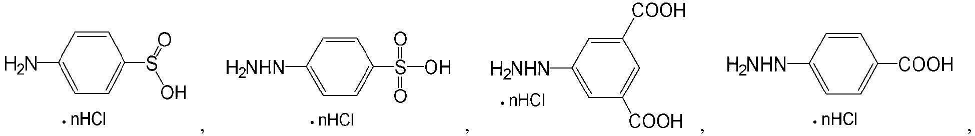 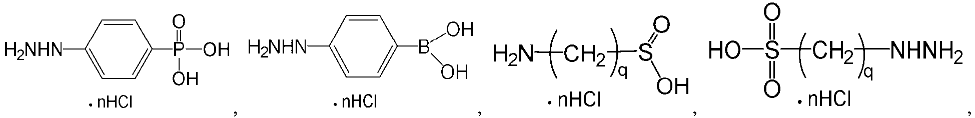 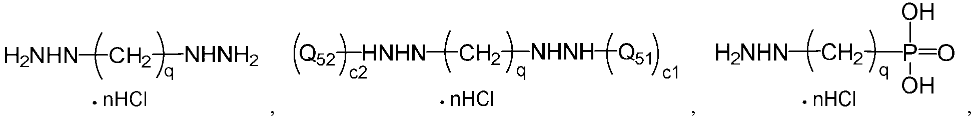 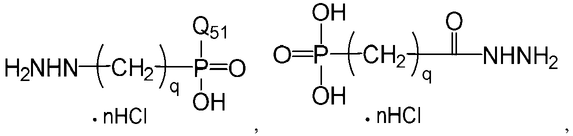 and a case where any one of the aforementioned compound salts is substituted with a substituent selected from the group G1. The definition of n in the above structure may be as described above. In some embodiments, n may be 1 or 2, wherein in some of the embodiments, n is 1, and in some other embodiments of the embodiments, n is 2. For example, 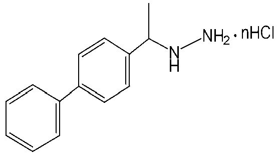 may be 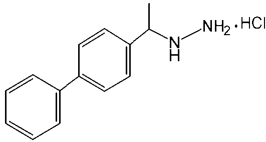 or

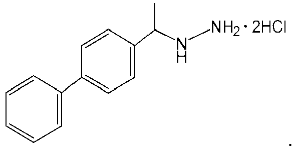

The first additive may include any any one or more of the compounds described above, thereby effectively improving the quality of the perovskite thin film to reduce defects, and thereby effectively increasing the energy conversion efficiency of the perovskite cell. In addition, the perovskite cell can maintain relatively good energy conversion efficiency for a long period of time, and the cell has good stability.

In some embodiments, the perovskite precursor material comprises a perovskite-type metal halide represented by chemical formula ABX₃, where A is a monovalent cation, B is a divalent cation, and X is a monovalent anion.

In some embodiments, A comprises one or more of Cs⁺, K⁺, Rb⁺, a monovalent amine cation, and a monovalent amidinate cation. Non-limiting examples of the monovalent amine cation are CH₃NH₃⁺ (methylamine, MA⁺), and ammonium (NH₄⁺). A non-limiting example of the monovalent amidinate cation is NH₂CH=NH₂⁺ (formamidine, which may be denoted as FA⁺).

In some embodiments, B comprises one or more of Pb²⁺, Sn²⁺, Fe²⁺, Mn²⁺, Ni²⁺, Ge²⁺, Co²⁺, and Sb²⁺.

In some embodiments, X comprises one or more of I⁻, Br⁻, and Cl⁻.

In some embodiments, X comprises one or both of I⁻, and Br⁻. X can be I⁻, Br⁻, or a combination thereof. In some embodiments, X is I⁻.

The first additive provided in the present application may improve different types of perovskite precursor material systems, increase quality of a corresponding perovskite thin film to reduce defects, and prolong a lifetime of a perovskite cell.

In some embodiments, the weight percent of the first additive relative to B element in the perovskite precursor material is 0.01%~15%, optionally 0.01%~10%, and optionally 0.1%~8%. The weight percent of the first additive relative to B element in the perovskite precursor material may also be any one selected from or within an interval consisting of any two selected from 0.01%, 0.05%, 0.1%, 0.2%, 0.4%, 0.5%, 0.6%, 0.8%, 1%, 1.5%, 2%, 2.5%, 3%, 3.5%, 4%, 4.5%, 5%, 6%, 7%, 8%, 9%, 10%, 11%, 12%, 13%, 14%, 15%, etc.

In some embodiments, the molar ratio of the first additive to the B element in the perovskite-type metal halide is 0.001%~15%, optionally 0.01%~15%, and optionally 2.0%~6.5%. The molar ratio of the first additive to the B element in the perovskite-type metal halide may also be any one selected from or within an interval consisting of any two selected from 0.001%, 0.005%, 0.01%, 0.05%, 0.1%, 0.2%, 0.4%, 0.5%, 0.6%, 0.8%, 1%, 1.5%, 2%, 2.5%, 3%, 3.5%, 4%, 4.5%, 5%, 6%, 7%, 8%, 9%, 10%, 11%, 12%, 13%, 14%, 15%, etc.

In some embodiments, the molar ratio of the R₀₁ to the B element in the perovskite-type metal halide is 0.01~15%, and optionally, the molar ratio of the R₀₁ to the B element in the perovskite-type metal halide is 1.0~8.0%. The molar ratio of the R₀₁ to the B element in the perovskite-type metal halide may also be any one selected from or within an interval consisting of any two selected from 0.001%, 0.005%, 0.01%, 0.05%, 0.1%, 0.2%, 0.4%, 0.5%, 0.6%, 0.8%, 1%, 1.5%, 2%, 2.5%, 3%, 3.5%, 4%, 4.5%, 5%, 6%, 7%, 8%, 9%, 10%, 11%, 12%, 13%, 14%, 15%, etc.

The first additive can function better by controlling its weak reducing capacity contributed to the perovskite precursor solution by adjusting one or more of the weight percentage of the first additive relative to the B element in the perovskite precursor material, the molar ratio of the first additive to the B element in the perovskite precursor material, the molar ratio of the weak reducing group to the B element in the perovskite-type metal halide, and the like.

In some embodiments, the perovskite precursor solution further contains an oxidation-state ion of a multi-valent metal element M.

In the present application, unless otherwise specified, "a multi-valent metal element" means that the metal element has a plurality of stably presentable valences.

In some embodiments, the multi-valent metallic element M comprises one or more elements of lanthanide elements, Fe, Co, Ni, Ti, Cr, Mn, Y, Rh, and Bi;
optionally, the lanthanide elements comprise one or more elements of Ce, Pr, Sm, Eu, Tb, and Yb;
optionally, the oxidation-state ion of the multi-valent metal element M comprises one or more of Ce⁴⁺, Pr⁴⁺, Sm³⁺, Eu³⁺ Tb⁴⁺, Yb³⁺, Fe³⁺, Co³⁺, Ni³⁺, Ti⁴⁺, Cr³⁺, Mn⁴⁺, Y³, and Rh⁴⁺; and
optionally, the oxidation-state ion of the multi-valent metal element M is derived from an organic salt of the metal element M, and further optionally, the organic salt includes one or more of an acetylacetonate salt, a sulfonate salt, and a sulfate salt.

In some embodiments, the perovskite precursor solution contains one or more ion pairs of Ce³⁺-Ce⁴⁺, Pr⁴⁺-Pr³⁺, Sm³⁺-Sm²⁺, Eu³⁺-Eu²⁺, Tb⁴⁺-Tb³⁺, Yb³⁺-Yb²⁺, Fe³⁺-Fe²⁺, Co³⁺-Co²⁺, Ni³⁺-Ni²⁺, Ti⁴⁺-Ti^{x+}, Cr³⁺-Cr²⁺, Mn⁴⁺-Mn²⁺, Y³⁺-Y²⁺, Rh⁴⁺-Rh²⁺, and Bi³⁺-Bi²⁺. Here, the positive-charge valence of Ti^{X} in the ion pair Ti⁴⁺-Ti^{X+} is less than 4.

By introducing the oxidation-state ion of the multi-valent metal cation into the perovskite precursor solution, the cation can react with B⁰ to form a reduction-state cation, and the oxidation-state ion of the multi-valent metal cation and the reduction-state cation can constitute a redox-balance pair which can cyclically inhibit degradation of BX₂ in the perovskite thin film, thereby prolonging the lifetime of the perovskite cell, and increasing energy conversion efficiency; and further, formation of B⁰, and X₂ can be inhibited to reduce defects at an interface, thereby further increasing performance of a perovskite device.

In some embodiments, the multi-valent metal element M may be added by a second additive. By adding Eu(acau)₃ (where acau is an abbreviation for acetylacetone), Eu³⁺ can be introduced and Eu³⁺ -Eu²⁺ ion pairs can be formed in the perovskite-precursor solution. As another example, by adding Fe(acau)₃, Fe³⁺ can be introduced and a Fe³⁺-Fe²⁺ ion pair can be formed in the perovskite-precursor solution.

In the present application, unless otherwise specified, "second additive" refers to a raw material that can form the aforementioned multi-valent metal element M or an ion pair thereof after being added to the perovskite precursor solution.

In some embodiments, the perovskite precursor material comprises the aforementioned perovskite-type metal halide; and the molar ratio of the multi-valent metal element M to B in the perovskite-type metal halide is 0.001%~15%, optionally 0.01%~15%. The molar ratio of the multi-valent metal element M to B in the perovskite-type metal halide may also be any one selected from or within an interval consisting of any two selected from 0.001%, 0.005%, 0.01%, 0.05%, 0.1%, 0.2%, 0.4%, 0.5%, 0.6%, 0.8%, 1%, 1.5%, 2%, 2.5%, 3%, 3.5%, 4%, 4.5%, 5%, 6%, 7%, 8%, 9%, 10%, 11%, 12%, 13%, 14%, 15%, etc.

The content of the multi-valent metal element M and the content of the corresponding ions of different valences can be detected by a method similar to the aforementioned method for detecting the concentration of B²⁺.

In a second aspect, the present application provides a perovskite thin film prepared by using the perovskite precursor solution described in the first aspect.

The perovskite thin film prepared by the perovskite precursor solution described in the first aspect of the present application has high quality with few defects, and the perovskite cell further prepared has high energy conversion efficiency, and also relatively good stability.

In some embodiments, the perovskite thin film is prepared by a method including steps of: applying the perovskite precursor solution described in the first aspect of the present application at a predetermined position and performing an annealing treatment to obtain the perovskite thin film.

The coating may be performed by slot die coating.

The temperature for the annealing treatment may be 100~180°C, for example, 100°C, 110°C, 120°C, 130°C, 140°C, 150°C, 160°C, 180°C, etc., or may be within a temperature range consisting of any two selected from the above temperatures, for example, 100~150°C.

In a third aspect, the present application provides a perovskite cell including the perovskite thin film described in the second aspect.

In some embodiments, the present application provides a perovskite cell comprising an electron transport layer, a hole transport layer, and the perovskite thin film described in the second aspect of the present disclosure, wherein the perovskite thin film is disposed between the electron transport layer and the hole transport layer.

In some embodiments, the present application provides a perovskite cell comprising a positive electrode, a negative electrode, and the perovskite thin film described in the second aspect of the present application, wherein the perovskite thin film is disposed between the positive electrode and the negative electrode.

In some embodiments, the present application provides a perovskite cell comprising a positive electrode, an electron transport layer, a perovskite thin film described in the second aspect of the present application, a hole transport layer, and a negative electrode which are sequentially disposed. Furthermore, it may be one of an inversed p-i-n cell or a normal n-i-p cell.

The perovskite thin film described in the second aspect of the present application, which may also be referred to as a perovskite layer, is also a light-absorbing layer in a perovskite cell.

During operation of the perovskite cell, after the light-absorbing layer is irradiated by light, electrons therein gain energy and escape from the light-absorbing layer to form negatively charged electron carriers, and also positively charged hole carriers, so as to obtain electron-hole pairs, and free electrons and free holes are transferred in opposite directions through corresponding transport layers, so that the electrons and the holes flow to constitute an external current to achieve conversion of light energy into electric energy. Further, after absorbing photons, the perovskite layer is excited to generate electron-hole pairs, they are further dissociated to form oppositely charged free carriers, wherein free electrons are transported to a positive electrode through the electron transport layer and free holes are transported to a negative electrode through the hole transport layer, and both free carriers are collected by corresponding electrodes, further forming photocurrent in a circuit of the perovskite cell.

The electron transport layer can extract and transport electron carriers, and can block passing of free holes.

The hole transport layer can extract and transport hole carriers, and can block passing of free electrons.

It will be appreciated that the perovskite cell further comprises two electrodes. One of the two electrodes is a positive electrode that can collect electron carriers transported via the electron transport layer, and the other one is a negative electrode that can collect hole carriers transported via the hole transport layer.

In some embodiments, for the electron-transporting layer, a material may be one or more of the following materials and derivatives thereof: an imide compound, a quinone compound, fullerene and a derivative thereof, methoxytriphenylamine-fluoroformamidine (OMeTPA-FA), calcium titanate (CaTiO₃), lithium fluoride (LiF), calcium fluoride (CaF₂), poly(3, 4-ethylenedioxythiophene): polystyrenesulfonic acid (PEDOT: PSS), poly-3-hexylthiophene (P3HT), triphenylamine with triptycene as a core (H101), 3, 4-ethylenedioxythiophene-methoxytriphenylamine (EDOT-OMeTPA), N-(4-aniline)carbazole-spirobifluorene (CzPAF-SBF), polythiophene, a metal oxide, silicon oxide (SiO₂), strontium titanate (SrTiO₃), cuprous thiocyanate (CuSCN), and the like, wherein metal elements may include one or more of Mg, Ni, Cd, Zn, In, Pb, Mo, W, Sb, Bi, Cu, Hg, Ti, Ag, Mn, Fe, V, Sn, Zr, Sr, Ga, and Cr.

In some embodiments, the hole transport layer may be at least one of the following materials and derivatives thereof: 2, 2', 7, 7'-tetra[N, N-bis(4-methoxyphenyl)amino]-9, 9'-spirobifluorene (Spiro-OMeTAD), polytriarylamine (PTAA), a nickel oxide (NiOₓ), poly-3, 4-ethylenedioxythiophene: polystyrene sulfonate (PEDOT: PSS), WO₃, etc., that can transport holes and block electrons.

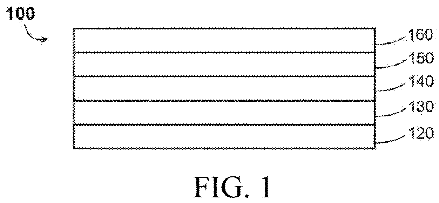

In some embodiments, the perovskite cell 100 includes the structure shown in FIG. 1, including a first electrode 120, a first transport layer 130, a perovskite layer 140, a second transport layer 150, and a second electrode 160 that are sequentially disposed. Furthermore, the shown respective structure layers are stacked sequentially.

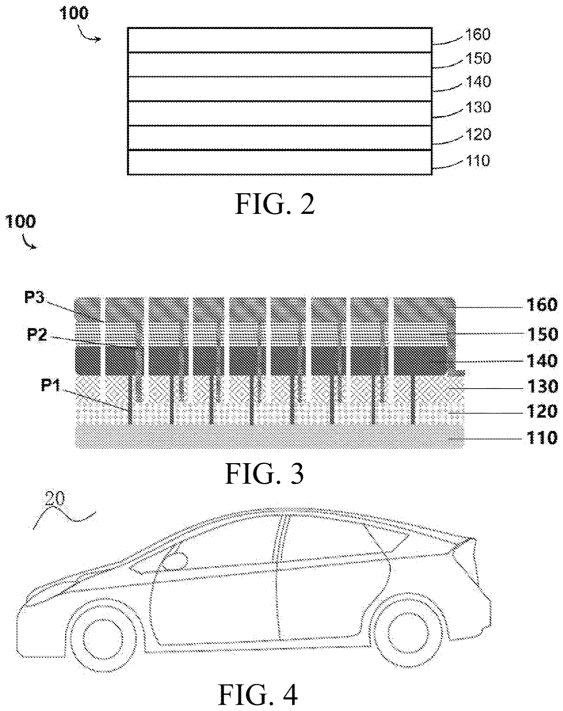

In some embodiments, the perovskite cell 100 includes the structure shown in FIG. 2, including a substrate layer 110, a first electrode 120, a first transport layer 130, a perovskite layer 140, a second transport layer 150, and a second electrode 160 that sequentially disposed. Furthermore, the shown respective structure layers are stacked sequentially.

In some embodiments, the perovskite cell comprises the structure shown in FIG. 3 (which is a vertical sectional structural view of the device) comprising a substrate layer 110, a first electrode 120, a first transport layer 130, a perovskite layer 140, a second transport layer 150, and a second electrode 160 disposed sequentially. Here, P1, P2, and P3 are etching regions disposed across layers, for dividing a film layer prepared with a large area into different components to enable the film layer to have a in-series cell structure, wherein P1, P2, and P3 are respectively used to connect structural layers disposed apart from each other, thus, the structural layers between the first electrode and the second electrode constitute a loop, and the perovskite cell is formed into a perovskite cell assembly. P1, P2 and P3 may each independently be a linear etched region, also referred to as an etched line. P1, P2 and P3 may each independently be a laser etched region. The numbers of P1, P2, and P3 may each independently be one or more. In FIG. 3, P1 is connected to the substrate layer by passing from the first transmission layer and the bottom of the first electrode from the surface of the first transmission layer, so that divided left and right sides of P1 are not connected to each other (to achieve insulation), the material in the etched region P1 is the same as that in the perovskite layer; P2 is connected to the surface of the first electrode by passing through the second transmission layer, the perovskite layer, and the first transmission layer from the surface of the second transmission layer, the material in the etched region P2 is the same as that in the second electrode; and P3 is connected to the surface of the first electrode by passing through the second transmission layer, the perovskite layer, and the first transmission layer from the surface of the second electrode, and the etched region P3 is not filled with a material.

In some embodiments, P1 has a width of 10~50 µm, for example 30 µm.

In some embodiments, P2 has a width of 10~200 µm, for example 150 µm. Further, the spacing between P2 and P1 may be 20~80 µm, for example, 20 µm.

In some embodiments, P3 has a width of 10~50 µm, for example 15 µm. Further, the spacing between P3 and P2 may be 20~40 µm, for example, 20 µm.

In some embodiments, P1 in the perovskite cell may run to the bottom of the first electrode from the surface of the first transport layer, with the material filled in P1 identical to that in the perovskite layer (as shown in FIG. 3). In other embodiments, P1 in the perovskite cell may also run to the bottom from the surface of the first electrode, with the material filled in P1 identical to that in the first transport layer.

In some embodiments, one of "the first transport layer" and "the second transport layer" is an electron transport layer and the other one is a hole transport layer. In some embodiments, the first transport layer is an electron transport layer. In some embodiments, the first transport layer is a hole transport layer.

In some embodiments, one of "the first electrode" and "the second electrode" is a transparent electrode for light incidence. In some implementations, the first electrode is a transparent electrode.

In some embodiments, the material of the transparent electrode may be exemplified by, but not limited to, one or more of: FTO (fluorine-doped tin oxide), ITO (tin-doped indium oxide), AZO (aluminum-doped zinc oxide transparent conductive glass), BZO (boron-doped zinc oxide), IZO (indium zinc oxide), IWO (tungsten-doped indium oxide), and the like.

In some embodiments, the second electrode comprises a conductive material, which may be an organic conductive material, an inorganic conductive material, or a combination thereof. A non-limiting example of the inorganic conductive material is a metallic conductive material, which is, for example, any one of gold (Au), silver (Ag), copper (Cu), aluminum (Al), nickel (Ni), chromium (Cr), bismuth (Bi), platinum (Pt), magnesium (Mg), and the like, or any suitable mixture of the aforementioned elements. The conductive material may be a conductive oxide, and further, a non-limiting example of the conductive oxide may include FTO, ITO, IWO, aluminum-doped zinc oxide (AZO), and the like.

In some embodiments, the perovskite cell is an inversed p-i-n cell or a normal n-i-p cell.

The perovskite cell provided in the present application may include both normal and inversed ones.

For a normal one, the perovskite cell includes a transparent electrode, and an electron transport layer, a perovskite layer, a hole transport layer, and a second electrode layer that are sequentially stacked on the transparent electrode.

For an inverted one, the perovskite cell includes a transparent electrode, and a hole transport layer, a perovskite layer, an electron transport layer, and a second electrode layer that are sequentially stacked on the transparent electrode. Here, the transparent electrode is used for light incidence.

In some embodiments, the perovskite cell includes a structure in which a substrate layer (which may be a glass substrate or a flexible substrate), a first electrode, a hole transport layer, a perovskite layer, an electron transport layer, and a second electrode are sequentially disposed. Here, the flexible substrate may include one or more materials selected from the group of polyethylene terephthalate, polyimide, polyethylene, polypropylene, polystyrene, polyethylene naphthalate, and the like. Optionally, the first electrode is a transparent electrode for light incidence.

In some embodiments, the perovskite cell includes a structure in which a substrate layer (a glass substrate or a flexible substrate), a first electrode, an electron transport layer, a perovskite layer, a hole transport layer, and a second electrode are sequentially disposed. Optionally, the first electrode is a transparent electrode for light incidence. Reference man be made to the above for the definition of the flexible substrate.

The substrate layer related in the embodiments or examples of the present application may be, but is not limited to, a glass substrate or a flexible substrate.

In some embodiments, the substrate layer is a flexible substrate layer. Further, the material of the substrate layer may be, for example, but not limited to, an organic polymer material, and further, a mixture of one or more in various ratios of the following materials including, but not limited to, polyvinyl alcohol (PVA), polyester (PET), polyimide (PI), polyethylene naphthalate (PEN), polydimethylsiloxane (PDMS), and the like.

In some embodiments, the substrate layer 110 in the structure shown in FIG. 3 is a light-incidence-surface glass substrate.

The size of the perovskite cell is not particularly limited, and may be, but not limited to, 300 mm × 300 mm.

It may be understood that the structure of the perovskite cell related in the present application may not be limited to the structural layers listed above. It is possible to introduce other functional layers, such as buffer layers, as desired. In some embodiments, the perovskite cell may be provided with a buffer layer having an appropriate energy level, which may function as one or more of reducing an energy level barrier, promoting energy level matching, increasing carrier extraction efficiency, and also, passivating an interface defect state, protecting a light-absorbing layer, inhibiting oxidative decomposition of a cell due to water molecules and oxygen, increasing photoelectric conversion efficiency, improving stability of a perovskite cell, and the like. According to the position of the buffer layer, the type of the buffer layer may include a buffer layer between a hole transport layer and an anode, a buffer layer between an electron transport layer and a cathode, a buffer layer between a hole transport layer and an absorption layer, and a buffer layer between an electron transport layer and an absorption layer. Materials that can be used for the buffer layer in the perovskite cell may include, but are not limited to, Cu₂O, NiO, AZO, TiO₂, etc. In a fourth aspect, the present application provides an electric device, comprising the perovskite cell described in the third aspect.

In some of the embodiments, the perovskite cell described above may be used as a power generation device for the electrical device. The type of the power generation device may include, but are not limited to, integrated power generation. The location of the power generation device may include, but is not limited to, a roof, a back panel, or the like of a vehicle.

Further, the electrical device described above may include, but is not limited thereto, a mobile apparatus such as a cell phone, a notebook computer, etc., or an electric vehicle, an electric train, a ship, a satellite, a power generation system, and the like.

FIG. 4 shows an electric device as an example. The electric device 20 is a vehicle, further, an electric vehicle, a hybrid electric vehicle, a plug-in hybrid electric vehicle, or the like.

As another example, the electric device may be a cell phone, a tablet computer, a notebook computer, a calculator, or the like.

As another example, the electrical device may be a wearable device, such as a watch.

Examples of the present application are described below. The example described below are illustrative only and are not to be construed as limiting the present application. In the example, specific techniques or conditions are not indicated, and they are performed according to techniques or conditions described above, in documents in the art or according to the specification of the product. The reagents or instruments used without specifying the manufacturer(s) are commercially available conventional products, or can be synthesized from commercially available products by a conventional mode.

In the following examples, "room temperature" refers to 20°C~30°C, further 25°C.

The types of the first additive related in the following examples can be seen in table 1. These first additives are commercially available, or can be obtained by salification reaction of a pure compound, which is not complexed with a hydrogen halide salt, and a haloid acid, or obtained by preparing a pure compound by a conventional method reported in documents and performing salification reaction via the pure compound and a haloid acid. Here, commercially available reagents can also be prepared according to established methods. Here, salification reaction with hydrohalic acid and preparation of some pure compounds can be achieved according to conventional synthetic methods in the field of organic chemistry. A pure compound for the first additive and a hydrogen halide salt thereof may be subjected to structural identification by one or more of detection methods including, but not limited to, Fourier infrared (FT-IR) spectroscopy, ultraviolet spectroscopy, hydrogen nuclear magnetic resonance (¹H NMR), X-ray diffraction (XRD), gel permeation chromatography (GPC), high performance liquid chromatography (HPLC), mass spectrometry, single crystal X-ray diffraction (SCXRD), inductively coupled plasma spectroscopy (ICP), and the like. These test methods have sample preparation methods and test methods which are known to those skilled in the art and can be adjusted depending on a specific compound structure and a material property.

It should be noted that, in the following examples, hydrochloride salt is used as a non-limiting example of a hydrogen halide salt. It may be understood that, in the present application, the hydrochloric acid molecule in the structure of the first additive may be replaced with another hydrogen halide salt acid molecule, that is, the hydrochloride salt may be replaced with another hydrogen halide salt For those skilled in the art, it may be easy to verify the effect of improving the perovskite precursor solution and perovskite thin film provided by the present application by a corresponding first additive structure with reference to the following non-limiting design examples.

First four numbers in a serial number of a first additive compound in table 1 correspond to a type of a pure compound part, the first four numbers being the same meaning that the same pure compound can be used for preparation; and the last number of the serial number represents the number of halogen acid molecules contained in one molecule, A, B, C,... Z representing 1, 2, 3,... 26, respectively. When the first four number are used to indicate the first additive, it means that the number of a hydrogen halide salt molecule contained in one molecule is not particularly limited. In table 1, when the number of HCl is denoted by n, a corresponding first additive corresponds to a combination of two salt forms with n=1 and n=2, unless otherwise specified.

**Table 1 Hydrogen Halide Salt Additive (First Additive) as Weakly Reducing Compound**

| No. | First Additive No. | Compound Structure | Acquisition Mode | Other Description |
|---|---|---|---|---|
| Example 1 | C001A | 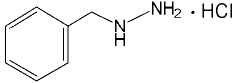 | Commercially available | |
| Example 2 | C001B | 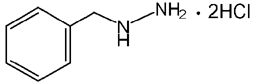 | Commercially available | |
| Example 3 | C002A | 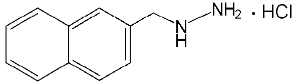 | Commercially available | |
| Example 4 | C002B | 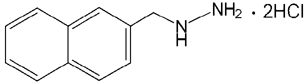 | Commercially available | |
| Example 5 | C003A | 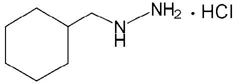 | Commercially available | |
| Example 6 | C003B | 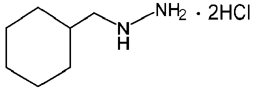 | Commercially available | |
| Example 7 | C004A | 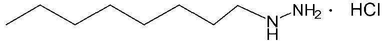 | Commercially available | |
| Example 8 | C004B | 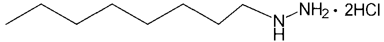 | Commercially available or prepared | |
| Example 9 | C005 | 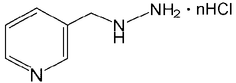 | Commercially available | n=1 and n=2 correspond to C005A and C005B, respectively. |
| Example 10 | C006 | 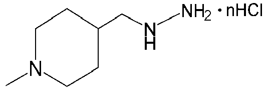 | Commercially available | n=1 and n=2 correspond to C006A and C006B, respectively. |
| Example 11 | C007 | 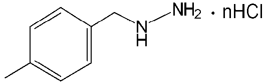 | Commercially available | n=1 and n=2 correspond to C007A and C007B, respectively. |
| Example 12 | C008A | 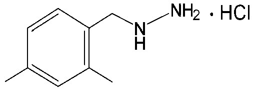 | Commercially available | |
| Example 13 | C009 | 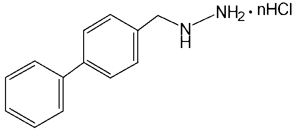 | Commercially available | n=1 and n=2 correspond to C009A and C009B, respectively. |
| Example 14 | C010 | 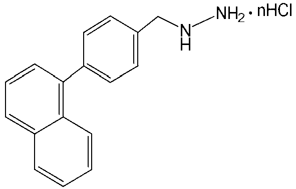 | 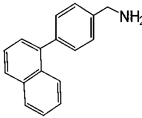 Prepared by | n=1 and n=2 correspond to CO10A and CO11B, respectively. |
| Example 15 | C011 | 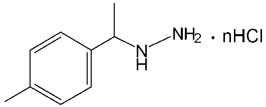 | Commercially available | n=1 and n=2 correspond to C011A and CO11B, respectively. |
| Example 16 | C012 | 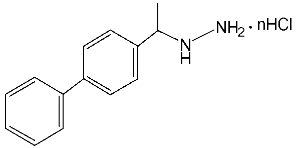 | Prepared | n=1 and n=2 correspond to C012A and C012B, respectively. |
| Example 17 | C013A | 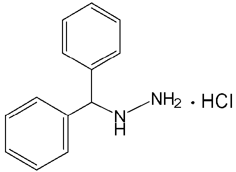 | Commercially available | |
| Example 18 | C014 | 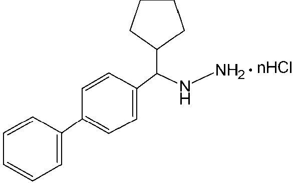 | Commercially available | n=1 and n=2 correspond to C014A and C014B, respectively. |
| Example 19 | C015 | 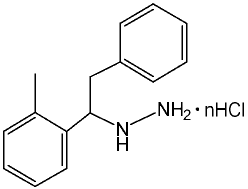 | Commercially available | n=1 and n=2 correspond to C015A and C015B, respectively. |
| Example 20 | C016 | 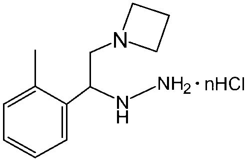 | Commercially available | n=1 and n=2 correspond to C016A and C016B, respectively. |
| Example 21 | C017 | 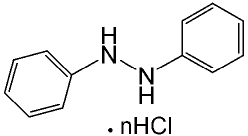 | Commercially available | n=1 and n=2 correspond to C017A and C017B, respectively. |
| Example 22 | C018 | 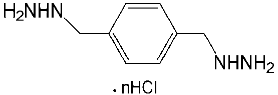 | Commercially available | n=1 and n=2 correspond to C018A and C018B, respectively. |
| Example 23 | C019 | 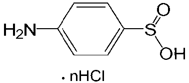 | Commercially available | |
| Example 24 | C020 | 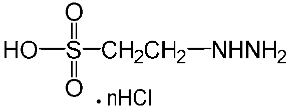 | Prepared | n=1 and n=2 correspond to C020A and C021B, respectively. |
| Example 25 | C021 | 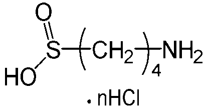 | Commercially available | n=1 and n=2 correspond to C021A and C021B, respectively. |
| Example 26 | C022B | 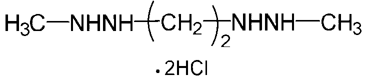 | Prepared | |
| Example 27 | C023 | 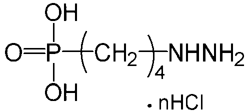 | Commercially available | |
| Example 28 | C024 | 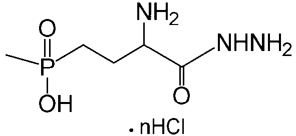 | Prepared | n=1 and n=2 correspond to C024A and C024B, respectively. |
| Example 29 | C025 | 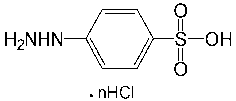 | Commercially available | n=1 and n=2 correspond to C025A and C025B, respectively. |
| Example 30 | C026A | 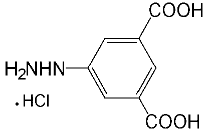 | Commercially available | |
| Example 31 | C027A | 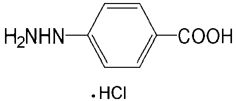 | Commercially available | |
| Example 32 | C028A | 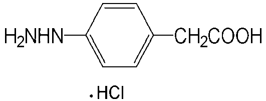 | Prepared | |
| Example 33 | C029 | 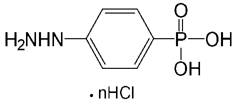 | Commercially available | n=1 and n=2 correspond to C029A and C029B, respectively. |
| Example 34 | C030 | 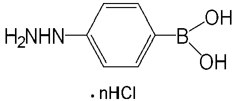 | Prepared | n=1 and n=2 correspond to C030A and C030B, respectively. |

In table 1, each first additive has a molecular weight less than 500 Da.

### Example 1. Preparation of Perovskite Precursor Solution, Perovskite Thin Film, and Perovskite Battery

For the perovskite cell prepared in example 1, an inversed p-i-n structure is used.

### 1.1. Preparation of First Electrode of Assembly

A group of FTO conductive glass having a size of 300 mm × 300 mm was etched using an infrared laser to form P1 having a width of about 30 µm, a piece of glass was divided into 44 sub-cells sequentially in a longitudinal direction, a series resistance of different sub-cells was greater than 10 MΩ, and a 10-mm upper portion and a 10-mm lower portion are regions to which an assembly was welded; and
the surface of the etched conductive glass was washed twice sequentially with acetone and isopropyl alcohol, immersed in deionized water for ultrasonic treatment for 10 min, dried in a blast drying oven, and then placed in a drying chamber (with a humidity (a relative humidity) less than 2%RH), and it was used as a first electrode.

### 1.2. Preparation of Hole Transport Layer of Assembly: Nickel Oxide Layer

The cleaned conductive glass was placed for magnetron sputtering to deposit a hole transporting layer of nickel oxide (noted as NiOₓ, where Ni may be Ni²⁺ or Ni³⁺), with a film thickness of a nickel oxide layer of 15 nm.

### 1.3. Preparation of Perovskite Main Layer of Assembly

A FAPbI₃ perovskite precursor solution with a concentration of 1.25 M in a solvent of dimethylformamide (DMF) was added with a first additive C001A with 0.001% (a molar concentration with respect to Pb), to form a perovskite precursor solution containing the first additive, which was sealed for later use;
the perovskite precursor solution containing the first additive was coated on the nickel oxide prepared above, by slot die coating, with a thickness of about 500 nm, and annealing at 100°C for 20 min.

### 1.4. Preparation of Electron Transport Layer of Assembly

A substrate having a prepared perovskite absorption layer was placed in a vacuum-thermal vapor deposition apparatus which was evacuated to 4 × 10⁻⁴ Pa, and 30 nm C₆₀, and 8 nm BCP (bathocuproine) were deposited as an electron-transporting layer of an assembly.

### 1.5. Preparation of Second Electrode of Assembly

After the electron transport layer was deposited in the vacuum-thermal vapor evaporation apparatus, 10 nm Ag was continuously deposited on the surface of the electron transport layer, after taking out after breaking vacuum, laser etching was made to form P2 with a width of 150 µm and a depth etched to a FTO layer, and the distance between P2 and P1 was 20 µm; and then the substrate was again placed in the vapor evaporation apparatus which is evacuated to 4 × 10⁻⁴ Pa, and then a layer of Ag was continuously deposited with a thickness of about 80 nm.

Followed by cooling and taking out after breaking vacuum, laser etching was made via green light for P s to form P3 with a width of 15 µm, and a depth etched to the FTO layer, and the distance between P3 and P2 was 20 µm, (positions of etching lines were P1/P2/P3);
and infrared edge trimming was then used for the assembly, i.e. both sides of the assembly were etched to 10 mm.

**Examples 2~26, and 31~34.** Substantially the same technical solution as that of example 1 was used, except for materials of structure layers of a perovskite cell (a first electrode, a first transport layer, a perovskite layer, a second transport layer, and a second electrode), types of a first additive and a second additive, and amounts of additives, referring to table 1, table 2, and table 3.

Example 27 An assembly with a normal n-i-p structure was prepared as follows.

### 1.1 Preparation of First Electrode of Assembly

A group of FTO conductive glass having a size of 300 mm × 300 mm was etched using an infrared laser to form P1 having a width of about 30 µm, a piece of glass was divided into 44 sub-cells sequentially in a longitudinal direction, a series resistance of different sub-cells was greater than 10 MΩ, and a 10-mm upper portion and a 10-mm lower portion are regions to which an assembly was welded; and
the surface of the etched conductive glass was washed twice sequentially with acetone and isopropyl alcohol, immersed in deionized water for ultrasonic treatment for 10 min, dried in a blast drying oven, and then placed in a drying chamber (with a humidity less than 2%RH), and it was used as a first electrode.

### 1.2 Preparation of Electron Transport Layer of Assembly: Stannic Oxide Layer

The cleaned conductive glass was placed into a chemical solution deposition apparatus for chemical depositioN(CBD deposition) to deposit an electron transport layer of SnO₂ with a tin oxide layer film having a thickness of about 50 nm.

### 1.3. Preparation of Perovskite Main Layer of Assembly

A FAPbI₃ perovskite precursor solution with a concentration of 1.25 M in a solvent of DMF was added with a first additive C001A with 0.01% (a molar concentration with respect to Pb), to form a perovskite precursor solution containing the first additive, which was sealed for later use; and
the perovskite precursor solution containing the first additive was coated on the nickel oxide prepared above, by slot die coating, with a thickness of about 500 nm, and annealing at 100°C for 20 min.

### 1.4. Preparation of Hole Transport Layer of Assembly

By slot die coating, the substrate with a prepared perovskite absorption layer was deposited with 20 nm Spiro-OOMeTAD as a hole transport layer of an assembly.

### 1.5. Preparation of Second Electrode of Assembly

After the electron transport layer was deposited, the assembly was placed in the vacuum-thermal vapor evaporation apparatus which was evacuated to 4 × 10⁻⁴ Pa, 10 nm Ag was continuously deposited on the surface of the electron transport layer, after taking out after breaking vacuum, laser etching was made to form P2 with a width of 150 µm and a depth etched to a FTO layer, and the distance between P2 and P1 was 20 µm; and then the substrate was again placed in the vapor evaporation apparatus which is evacuated to 4 × 10⁻⁴ Pa, and then a layer of Ag was continuously deposited with a thickness of about 80 nm.

Followed by cooling and taking out after breaking vacuum, laser etching was made via green light for P s to form P3 with a width of 15 µm, and a depth etched to the FTO layer, and the distance between P3 and P2 was 20 µm, (positions of etching lines were P1/P2/P3).

The normal n-i-p assemblies in examples 28-30 can be prepared in substantially the same mode as that in example 27, with the difference shown in tables 1-3.

**Comparative Examples 1~18.** Substantially the same technical solutions were used as that in examples 1, 16-18, 4, 20-24, 15, 25-27, 29-30, 6, and 5, except that the first additive in the present application was not added. See tables 2 and 3 for details.

**Comparative Examples 19~24** Substantially the same technical solutions were used as that in example 1, except for different types of additives, wherein no hydrogen halide salt was complexed in comparative example 19, no weak reducing group was in comparative example 20, the reducing group had too strong reducilibity in comparative example 21, the reducing group had too weak reducilibity in comparative example 22, and the additives in comparative examples 23-24 had no reducilibity.

**Comparative Example 25** Substantially the same technical solutions were used as that in example 27, and the amount of the first additive was substantially the same, except that the type of the additive was different. See Tables 1-3.

**Table 2.**

| No. | Additive Code | Molar Ratio of First Additive to B |
|---|---|---|
| Example 1 | C001A | 0.34% |
| Example 2 | C001B | 0.14% |
| Example 3 | C002A | 0.53% |
| Example 4 | C002B | 0.22% |
| Example 5 | C003A | 0.33% |
| Example 6 | C003B | 10.30% |
| Example 7 | C004A | 15.00% |
| Example 8 | C004B | 8.00% |
| Example 9 | C005 | 6.72% |
| Example 10 | C006 | 9.23% |
| Example 11 | C007 | 4.13% |
| Example 12 | C008A | 4.81% |
| Example 13 | C009 | 7.13% |
| Example 14 | C010 | 6.42% |
| Example 15 | C011 | 5.19% |
| Example 16 | C012 | 7.22% |
| Example 17 | C013A | 1.02% |
| Example 18 | C014 | 1.77% |
| Example 19 | C015 | 5.66% |
| Example 20 | C016 | 0.88% |
| Example 21 | C017 | 3.67% |
| Example 22 | C018 | 0.37% |
| Example 23 | C019 | 0.67% |
| Example 24 | C020 | 0.01% |
| Example 25 | C021 | 4.53% |
| Example 26 | C022B | 6.50% |
| Example 27 | C023 | 0.01% |
| Example 28 | C024 | 10.17% |
| Example 29 | C025 | 2.00% |
| Example 30 | C026A | 5.36% |
| Example 31 | C027A | 0.56% |
| Example 32 | C028A | 0.56% |
| Example 33 | C029 | 0.85% |
| Example 34 | C030 | 4.16% |
| Comparative example 1 | None | None |
| Comparative example 2 | None | None |
| Comparative example 3 | None | None |
| Comparative example 4 | None | None |
| Comparative example 5 | None | None |
| Comparative example 6 | None | None |
| Comparative example 7 | None | None |
| Comparative example 8 | None | None |
| Comparative example 9 | None | None |
| Comparative example 10 | None | None |
| Comparative example 11 | None | None |
| Comparative example 12 | None | None |
| Comparative example 13 | None | None |
| Comparative example 14 | None | None |
| Comparative example 15 | None | None |
| Comparative example 16 | None | None |
| Comparative example 17 | None | None |
| Comparative example 18 | None | None |
| Comparative example 19 | Pure Compound of C001 (unsalified) | 0.34% |
| | 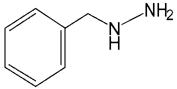 | |
| Comparative example 20 | 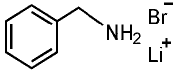 | 0.36% |
| Comparative example 21 | Nitrobenzene with strong reducilibity | 0.31% |
| Comparative example 22 | Phenol with weak reducilibity | 0.33% |
| Comparative example 23 | Benzoic acid with no reducilibity | 0.33% |
| Comparative example 24 | Oxalic acid with no reducilibity | 0.33% |
| Comparative example 25 | Benzene sulfonic acid with no reducilibity | 0.01% |

In Table 2, the additive in each of examples 1 to 35 is the first additive provided in the present application. Both the weight ratio of the first additive to B and the molar ratio of the weak reducing group to B can be calculated from the molar ratio of the first additive to B. Here, B is element corresponding to a divalent cation in perovskite-type metal halide in a perovskite layer.

**Table 3.**

| No. | First Electrode | First Transport Layer | Perovskite Main Layer Material | Second Transport Layer | Second Electrode | Normal/Inversed |
|---|---|---|---|---|---|---|
| Example 1 | FTO | Nickel oxide layer | FAPbI₃ | C60/BCP | Ag | Inversed p-i-n |
| Example 2 | FTO | Nickel oxide layer | FAPbI₃ | C60/BCP | Ag | Inversed p-i-n |
| Example 3 | FTO | Nickel oxide layer | FAPbI₃ | SiO₂ | Ag | Inversed p-i-n |
| Example 4 | ITO | Nickel oxide layer | FAPbI₃ | C60/BCP | Ag | Inversed p-i-n |
| Example 5 | FTO | Nickel oxide layer | FAPbI₃ | PCBM/BCP | Ag | Inversed p-i-n |
| Example 6 | FTO | Nickel oxide layer | FAPbI₃ | C60/BCP | Au | Inversed p-i-n |
| Example 7 | FTO | Nickel oxide layer | FAPbI₃ | C60/BCP | Cu | Inversed p-i-n |
| Example 8 | FTO | Nickel oxide layer | FAPbI₃ | C60/BCP | Ag | Inversed p-i-n |
| Example 9 | FTO | Nickel oxide layer | FAPbI₃ | C60/BCP | Ag | Inversed p-i-n |
| Example 10 | FTO | Nickel oxide layer | FAPbI₃ | C60/BCP | Ag | Inversed p-i-n |
| Example 11 | FTO | Nickel oxide layer | FAPbI₃ | C60/BCP | Ag | Inversed p-i-n |
| Example 12 | FTO | Nickel oxide layer | FAPbI₃ | C60/BCP | Ag | Inversed p-i-n |
| Example 13 | FTO | Nickel oxide layer | FAPbI₃ | C60/BCP | Ag | Inversed p-i-n |
| Example 14 | FTO | Nickel oxide layer | FAPbI₃ | C60/BCP | Ag | Inversed p-i-n |
| Example 15 | FTO | Nickel oxide layer | PbI₃ | C60/BCP | Ag | Inversed p-i-n |
| Example 16 | FTO | Nickel oxide layer | (FA_{0.95}Cs_{0.05})PbI₃ | C60/BCP | Ag | Inversed p-i-n |
| Example 17 | FTO | Nickel oxide layer | (FA_{0.90}MA_{0.05}Cs_{0.05})PbI₃ | C60/BCP | Ag | Inversed p-i-n |
| Example 18 | FTO | Nickel oxide layer | (FA_{0.95}Cs_{0.05})Pb(I_{0.9}Br_{0.1})₃ | C60/BCP | Ag | Inversed p-i-n |
| Example 19 | FTO | Nickel oxide layer | FAPbI₃ | C60/BCP | Ag | Inversed p-i-n |
| Example 20 | FTO | Nickel oxide layer | FAMnI₃ | C60/BCP | Ag | Inversed p-i-n |
| Example 21 | FTO | Nickel oxide layer | FASnI₃ | C60/BCP | Ag | Inversed p-i-n |
| Example 22 | FTO | Nickel oxide layer | FAFeI₃ | C60/BCP | Ag | Inversed p-i-n |
| Example 23 | FTO | Nickel oxide layer | FAGeI₃ | C60/BCP | Ag | Inversed p-i-n |
| Example 24 | FTO | Nickel oxide layer | FANiI₃ | C60/BCP | Ag | Inversed p-i-n |
| Example 25 | FTO | Nickel oxide layer | FACoI₃ | C60/BCP | Ag | Inversed p-i-n |
| Example 26 | FTO | Nickel oxide layer | FASbI₃ | C60/BCP | Ag | Inversed p-i-n |
| Example 27 | FTO | SiO₂ | FAPbI₃ | Spiro-OMeTAD | Ag | Normal n-i-p |
| Example 28 | ITO | SiO₂ | FAPbI₃ | Spiro-OMeTAD | Ag | Normal n-i-p |
| Example 29 | FTO | SiO₂ | (FA_{0.95}Cs_{0.05})Pb(I_{0.9}Br_{0.01})₃ | Spiro-OMeTAD | Ag | Normal n-i-p |
| Example 30 | FTO | SiO₂ | (FA_{0.95}Cs_{0.05})PbI₃ | Spiro-OMeTAD | Ag | Normal n-i-p |
| Example 31 | FTO | Nickel oxide layer | FAPbI₃ | C60/BCP | Ag | Inversed p-i-n |
| Example 32 | FTO | Nickel oxide layer | FAPbI₃ | C60/BCP | Ag | Inversed p-i-n |
| Example 33 | FTO | Nickel oxide layer | FAPbI₃ | C60/BCP | Ag | Inversed p-i-n |
| Example 34 | FTO | Nickel oxide layer | FAPbI₃ | C60/BCP | Ag | Inversed p-i-n |
| Comparative example 1 | FTO | Nickel oxide layer | FAPbI₃ | C60/BCP | Ag | Inversed p-i-n |
| Comparative example 2 | FTO | Nickel oxide layer | (FA_{0.95}CS_{0.05})PbI₃ | C60/BCP | Ag | Inversed p-i-n |
| Comparative example 3 | FTO | Nickel oxide layer | (FA_{0.90}MA_{0.05}Cs_{0.05})PbI₃ | C60/BCP | Ag | Inversed p-i-n |
| Comparative example 4 | FTO | Nickel oxide layer | (FA_{0.95}Cs_{0.05})Pb(I_{0.9}Br_{0.1})₃ | C60/BCP | Ag | Inversed p-i-n |
| Comparative example 5 | ITO | Nickel oxide layer | FAPbI₃ | C60/BCP | Ag | Inversed p-i-n |
| Comparative example 6 | FTO | Nickel oxide layer | FAMnI₃ | C60/BCP | Ag | Inversed p-i-n |
| Comparative example 7 | FTO | Nickel oxide layer | FASnI₃ | C60/BCP | Ag | Inversed p-i-n |
| Comparative example 8 | FTO | Nickel oxide layer | FAFeI₃ | C60/BCP | Ag | Inversed p-i-n |
| Comparative example 9 | FTO | Nickel oxide layer | FAGeI₃ | C60/BCP | Ag | Inversed p-i-n |
| Comparative example 10 | FTO | Nickel oxide layer | FANiI₃ | C60/BCP | Ag | Inversed p-i-n |
| Comparative example 11 | FTO | Nickel oxide layer | PbI₃ | C60/BCP | Ag | Inversed p-i-n |
| Comparative example 12 | FTO | Nickel oxide layer | FACoI₃ | C60/BCP | Ag | Inversed p-i-n |
| Comparative example 13 | FTO | Nickel oxide layer | FASbI₃ | C60/BCP | Ag | Inversed p-i-n |
| Comparative example 14 | FTO | SiO₂ | FAPbI₃ | Spiro-OMeTAD | Ag | Normal n-i-p |
| Comparative example 15 | FTO | SiO₂ | (FA_{0.95}Cs_{0.05})Pb(I_{0.9}Br_{0.01})₃ | Spiro-OMeTAD | Ag | Normal n-i-p |
| Comparative example 16 | FTO | SiO₂ | (FA_{0.95}Cs_{0.05})PbI₃ | Spiro-OMeTAD | Ag | Normal n-i-p |
| Comparative example 17 | FTO | Nickel oxide layer | FAPbI₃ | C60/BCP | Au | Inversed p-i-n |
| Comparative example 18 | FTO | Nickel oxide layer | FAPbI₃ | PCBM/BCP | Ag | Inversed p-i-n |
| Comparative example 19 | FTO | Nickel oxide layer | FAPbI₃ | C60/BCP | Ag | Inversed p-i-n |
| Comparative example 20 | FTO | Nickel oxide layer | FAPbI₃ | C60/BCP | Ag | Inversed p-i-n |
| Comparative example 21 | FTO | Nickel oxide layer | FAPbI₃ | C60/BCP | Ag | Inversed p-i-n |
| Comparative example 22 | FTO | Nickel oxide layer | FAPbI₃ | C60/BCP | Ag | Inversed p-i-n |
| Comparative example 23 | FTO | Nickel oxide layer | FAPbI₃ | C60/BCP | Ag | Inversed p-i-n |
| Comparative example 24 | FTO | Nickel oxide layer | FAPbI₃ | C60/BCP | Ag | Inversed p-i-n |
| Comparative example 25 | FTO | SiO₂ | FAPbI₃ | Spiro-OMeTAD | Ag | Normal n-i-p |

### Example 35. Ion Pair Containing Multivalent Metal Element M

### 35.1. Preparation of First Electrode of Assembly

A group of FTO conductive glass having a size of 300 mm × 300 mm was etched using an infrared laser to form P1 having a width of about 30 µm, a piece of glass was divided into 44 sub-cells sequentially in a longitudinal direction, a series resistance of different sub-cells was greater than 10 MΩ, and a 10-mm upper portion and a 10-mm lower portion are regions to which an assembly was welded; and

the surface of the etched conductive glass was washed twice sequentially with acetone and isopropyl alcohol, immersed in deionized water for ultrasonic treatment for 10 min, dried in a blast drying oven, and then placed in a drying chamber (with a humidity less than 2%RH), and it was used as a first electrode.

### 35.2. Preparation of Hole Transport Layer of Assembly: Nickel Oxide Layer

The cleaned conductive glass was placed for magnetron sputtering to deposit a hole transporting layer of nickel oxide (NiOₓ), with a film thickness of a nickel oxide layer of 15 nm.

### 35.3. Preparation of Perovskite Main Layer of Assembly

A FAPbI₃ perovskite precursor solution with a concentration of 1.25 M in a solvent of DMF (dimethylformamide) was added with a first additive **C001A** with 0.001% (a molar concentration with respect to Pb), and also a second additive Eu(acau)₃ (wherein, acau is short for diacetone)to form a perovskite precursor solution containing the first additive, which was sealed for later use; and
the perovskite precursor solution containing the additive was coated on the nickel oxide prepared above, by slot die coating, with a thickness of about 500 nm, and annealing at 100°C for 20 min.

### 35.4. Preparation of Electron Transport Layer of Assembly

A substrate having a prepared perovskite absorption layer was placed in a vacuum-thermal vapor deposition apparatus which was evacuated to 4 × 10⁻⁴ Pa, and 30 nm C₆₀, and 8 nm BCP were deposited as an electron-transporting layer of an assembly.

### 35.5. Preparation of Second Electrode of Assembly

After the electron transport layer was deposited in the vacuum-thermal vapor evaporation apparatus, 10 nm Ag was continuously deposited on the surface of the electron transport layer, after taking out after breaking vacuum, laser etching was made to form P2 with a width of 150 µm and a depth etched to a FTO layer, and the distance between P2 and P1 was 20 µm; and then the substrate was again placed in the vapor evaporation apparatus which is evacuated to 4 × 10⁻⁴ Pa, and then a layer of Ag was continuously deposited with a thickness of about 80 nm.

Followed by cooling and taking out after breaking vacuum, laser etching was made via green light for P s to form P3 with a width of 15 µm, and a depth etched to the FTO layer, and the distance between P3 and P2 was 20 µm, (positions of etching lines were P1/P2/P3);
and infrared edge trimming was then used for the assembly, i.e. both sides of the assembly were etched to 10 mm.

**Examples 36~41.** Substantially the same technical solutions as example 35 were used, except for a base system (a salt containing a weakly reducing compound), a type of an ion pair, and its raw material and additive, see Table 4.

**Table 4**

| No. | Base System | Ion Pair | Second Additive | Molar Ratio of Second Additive to B |
|---|---|---|---|---|
| Example 35 | Comparative example 1 | Eu³⁺-Eu²⁺ | Eu(acau)₃ | 0.001% |
| Example 36 | Comparative example 1 | Eu³⁺-Eu²⁺ | Eu(acau)₃ | 4.8% |
| Example 37 | Comparative example 1 | Eu³⁺-Eu²⁺ | Eu(acau)₃ | 15% |
| Example 38 | Example 1 | Eu³⁺-Eu²⁺ | Eu(acau)₃ | 0.350% |
| Example 39 | Comparative example 1 | Fe³⁺-Pe²⁺ | Fe(acau)₃ | 0.001% |
| Example 40 | Comparative example 1 | Fe³⁺-Pe²⁺ | Fe(acau)₃ | 0.488% |
| Example 41 | Comparative example 1 | Fe³⁺-Pe²⁺ | Fe(acau)₃ | 15% |

In table 4, the molar amount of the second additive was numerically equal to the molar amount of the metal element of the ion pair, and thus the molar ratio of the metal element of the ion pair to B was numerically equal to the molar ratio of the second additive to B.

### Test Method

### 1. Energy Conversion Efficiency Test

Energy conversion efficiency of an assembly is tested by a solar simulator at 1000W/m².

At normal pressure and temperature, using a standard light source of AM1.5G as a solar simulator light source, and a four-channel digital source meter (Keithley 2440), a voltammogram of an assembly under light irradiation from the light sourceis measured, to obtain an open circuit voltage Voc, a short circuit current density Jsc, and a fill factor FF (Fill Factor) of the assembly, so as to obtain the energy conversion efficiency Eff (Efficiency) of the assembly.

"Normal temperature and normal pressure" means a pressure of 1 atm at a temperature of 25°C; and "normal temperature" means 20°C~30°C, and further 25°C.

Measured values of initial energy conversion efficiency can be seen in Table 5.

### 2. Battery Stability Performance

After end of the test, the cell was placed in an atmospheric environment (a relative humidity 65%-85 RH, and ambient temperature about 15-40°C, specifically 25°C), and after being placed for at least 500 h without keeping out of the sun, the energy conversion efficiency was tested again, and the ratio of the assembly efficiency after placement for 500 h and 1000 h in the atmosphere to the initial efficiency value was calculated, and the relative efficiency was used as a performance parameter for assembly stability. The test results can be seen in Table 5.

### Test Result and Analysis

With respect to comparative examples 1-18 in which the first additive of the present application is not added, examples 1-34 in which the first additive of the present application is added, according to the test results of the perovskite cells prepared in the corresponding examples, have the initial energy conversion efficiency and the relative efficiency after placement for 500 h and 1000 h that are significantly increased, and it can be seen that the cell stability is significantly enhanced. The first additives in examples 1-34 all contain 1 or more weakly reducing groups, in addition, examples 1-4, 9, 11-23 and 29-34 have aromatic end groups or linking groups, examples 5-6 and 10 have alicyclic end groups or alicyclic linking groups, and examples 7-8 and 24-27 have aliphatic chain end groups or linking groups. In examples 1-6, 9, 12-20, 32, etc., a spacer group is provided between an aromatic end group or a linker group and the weakly reducing group -NHNH₂.

With respect to comparative examples 1-18 in which the first additive of the present application is not added, in all of examples 35-37 and 39-41 in which the second additive provided by the present application is added, and oxidation-state ions of multi-valent metal element M and corresponding ion pairs are introduced into the perovskite precursor solution, according to test results of perovskite cells prepared in corresponding examples, enables initial energy conversion efficiency and relative efficiency after placement for 500 h and 1000 h that are improved.

With respect to comparative examples 1-18 in which the first additive of the present application is not added, example 38 in which both the first additive and the second additive of the present application are added enables significantly improved initial energy conversion efficiency, and also significantly improved relative efficiency after placement for 500 h and 1000 h.

Comparative example 19 uses the additive which is is not formed into a salt and is a pure compound, and as found from the result, the comparative example enables the initial energy conversion efficiency and the relative efficiency after placement for 500 h and 1000 h that are all significantly reduced with respect to the technical solutions such as example 1 provided in the present application, so that cell stability is significantly deteriorated.

Comparative example 20 in which the additive has no weakly reducing group and a salt formation mode is different, as found from the result, enables the initial energy conversion efficiency and the relative efficiency after placement for 500 h and 1000 h that are all significantly reduced with respect to the technical solutions such as example 1 provided in the present application, so that cell stability is significantly deteriorated.

Compared with the first additive such as example 1 provided by the present application, the reducing group in comparative example 21 has too strong reducibility, the reducing group in comparative example 22 has too weak reducibility, and the additives in comparative examples 23-24 have no reducibility, and as found from the result, the initial energy conversion efficiency and the relative efficiency after placement for 500 h and 1000 h are reduced to some degrees.

Compared with the first additive provided in example 27, the additive in comparative example 25 has no reducibility, and as as found from the result, both the initial energy conversion efficiency and the relative efficiency after placement for 500 h and 1000 h are significantly reduced.

**Table 5.**

| No. | Initial Energy Conversion Efficiency | Stability Performance of Battery (Relative Efficiency after Placement for a Period of Time) (Based on Initial Energy Conversion Effect) | |
|---|---|---|---|
| | | Relative Efficiency after Placement of Assembly for 500 h | Relative Efficiency after Placement of Assembly for 1000 h |
| Example 1 | 23.91% | 23.61% | 23.01% |
| Example 2 | 23.04% | 21.23% | 21.04% |
| Example 3 | 23.54% | 23.10% | 22.26% |
| Example 4 | 23.19% | 22.15% | 21.54% |
| Example 5 | 22.87% | 20.51% | 20.04% |
| Example 6 | 20.94% | 20.01% | 19.45% |
| Example 7 | 20.11% | 19.64% | 19.13% |
| Example 8 | 20.05% | 19.32% | 19.04% |
| Example 9 | 21.69% | 20.58% | 19.98% |
| Example 10 | 19.99% | 19.13% | 18.94% |
| Example 11 | 22.64% | 22.03% | 21.43% |
| Example 12 | 22.87% | 22.29% | 21.16% |
| Example 13 | 22.85% | 22.11% | 21.59% |
| Example 14 | 23.66% | 22.10% | 21.31% |
| Example 15 | 19.68% | 19.05% | 18.44% |
| Example 16 | 22.15% | 20.97% | 19.83% |
| Example 17 | 23.12% | 21.34% | 20.57% |
| Example 18 | 21.97% | 20.63% | 20.07% |
| Example 19 | 21.02% | 20.42% | 19.75% |
| Example 20 | 16.07% | 15.47% | 15.01% |
| Example 21 | 18.33% | 17.14% | 16.32% |
| Example 22 | 15.44% | 14.16% | 13.55% |
| Example 23 | 16.54% | 15.12% | 14.62% |
| Example 24 | 15.31% | 13.20% | 11.94% |
| Example 25 | 15.64% | 14.16% | 14.01% |
| Example 26 | 15.01% | 14.24% | 13.08% |
| Example 27 | 22.51% | 19.15% | 17.02% |
| Example 28 | 21.08% | 20.11% | 18.98% |
| Example 29 | 21.43% | 19.88% | 17.15% |
| Example 30 | 20.83% | 19.02% | 18.22% |
| Example 31 | 20.34% | 18.86% | 17.25% |
| Example 32 | 20.87% | 20.01% | 19.62% |
| Example 33 | 21.64% | 20.03% | 19.19% |
| Example 34 | 20.03% | 18.73% | 18.01% |
| Example 35 | 18.21% | 15.11% | 10.46% |
| Example 36 | 20.08% | 19.23% | 18.21% |
| Example 37 | 17.23% | 16.99% | 16.01% |
| Example 38 | 23.92% | 23.79% | 23.34% |
| Example 39 | 18.08% | 14.95% | 9.36% |
| Example 40 | 19.11% | 18.37% | 17.25% |
| Example 41 | 17.29% | 16.87% | 15.97% |
| Comparative example 1 | 18.01% | 14.08% | 8.71% |
| Comparative example 2 | 18.94% | 15.15% | 10.23% |
| Comparative example 3 | 19.05% | 14.34% | 11.38% |
| Comparative example 4 | 18.77% | 13.42% | 9.87% |
| Comparative example 5 | 18.00% | 14.29% | 10.71% |
| Comparative example 6 | 12.37% | 9.37% | 6.21% |
| Comparative example 7 | 15.64% | 11.54% | 8.67% |
| Comparative example 8 | 10.84% | 6.89% | 5.21% |
| Comparative example 9 | 13.19% | 7.43% | 5.09% |
| Comparative example 10 | 11.68% | 8.35% | 4.37% |
| Comparative example 11 | 17.14% | 13.26% | 9.53% |
| Comparative example 12 | 12.52% | 9.53% | 6.32% |
| Comparative example 13 | 13.09% | 8.17% | 6.22% |
| Comparative example 14 | 20.07% | 13.63% | 5.87% |
| Comparative example 15 | 20.19% | 12.11% | 6.21% |
| Comparative example 16 | 20.21% | 12.45% | 7.63% |
| Comparative example 17 | 18.02% | 13.35% | 10.42% |
| Comparative example 18 | 18.17% | 12.59% | 10.32% |
| Comparative example 19 | 18.15% | 15.20% | 13.58% |
| Comparative example 20 | 18.14% | 15.13% | 10.21% |
| Comparative example 21 | 16.21% | 10.43% | 4.36% |
| Comparative example 22 | 18.16% | 11.32% | 6.49% |
| Comparative example 23 | 18.14% | 14.21% | 8.52% |
| Comparative example 24 | 18.17% | 14.36% | 8.66% |
| Comparative example 25 | 20.04% | 14.21% | 7.41% |

According to the research data made by the inventor of the present application, when the halogen acid molecule in the first additive of the present application is replaced with another organic acid molecule, in some cases, solubility may be significantly lowered, and in other cases, the effect of improving the energy conversion efficiency of the perovskite cell and the cell stabilizer is significantly deteriorated.

The technical features of the above-described example may be combined, and all possible combinations of the technical features in the above-described embodiments are not described for concise description, however, it should be regarded that the combinations of the technical features are with the range described in the present description as long as there is no inconsistency.

It should be noted that the present application is not limited to the above-described implementation. The above-described implementation is merely an example, and any implementation having substantially the same configuration as the technical concept and exhibiting the same operation and effect within the scope of the claims of the present application is included in the technical scope of the present application. The above examples merely illustrate several embodiments of the present application, which are described in detail, but are not to be construed as limiting the scope of the patent. In addition, various modifications that can be conceived by those skilled in the art may be made to the implementations without departing from the subject matter of the present application, and other implementations constructed by combining some of the constituent elements in the implementations are also included in the scope of the present application. It should be noted that, for those skilled in the art, several variations and modifications may be made without departing from the concept of the present disclosure, which are all within the protection scope of the present disclosure. Therefore, the protection scope of the present application should be defined by the appended claims, and the description and the drawings may be used to interpret the claims.

## Claims

1. A perovskite precursor solution comprising a perovskite precursor material, a solvent, and an additive, wherein the perovskite precursor material comprises a perovskite-type metal halide; the additive contains, in its structure, a weakly reducing group which has reducibility on a 0 valence corresponding to a monovalent anion in the perovskite-type metal halide and is inert to a divalent cation in the perovskite-type metal halide; and the additive is a hydrogen halide salt.

2. The perovskite precursor solution according to claim 1, wherein the weakly reducing group comprises one or more of the group of: -NHNH-, -S-S-, -NHNH₂, sulfino, phosphinico, hydroxyphenyl, and hydroxynaphthyl; and
optionally, the weakly reducing group is selected from the group of: -NHNH-, -S-S-, -NHNH₂, sulfino, phosphinico, hydroxyphenyl, and hydroxynaphthyl.

3. The perovskite precursor solution according to claim 1 or 2, wherein in one molecule of the additive, the number of the weak reducing group is 1 or more;
optionally, in one molecule of the additive, the number of the weak reducing group is an integer selected from 1~10; further optionally, the number of the weak reducing group is 1, 2, 3, 4 or 5; further optionally, the number of the weak reducing group is 1, 2 or 3; and further optionally, the number of the weak reducing group is 1 or 2; and
optionally, in one molecule of the additive, the type of the weak reducing group is one or more; and further optionally, the type of the weak reducing group is 1, 2, 3 or more.

4. The perovskite precursor solution according to any one of claims 1~ 3, wherein in one molecule of the additive, the number of halogen acid molecules is 1 or more; and
optionally, in one molecule of the additive, the number of halogen acid molecules is an integer selected from 1~10; further optionally, the number of halogen acid molecules is 1, 2, 3, 4, or 5; and even further optionally, the number of halogen acid molecules is 1 or 2.

5. The perovskite precursor solution according to any one of claims 1~4, wherein the halogen in the hydrogen halide salt comprises at least one of F, Cl, Br, and I;
optionally, any halogen in the hydrogen halide salt is independently F, Cl, Br or I; and
further optionally, the hydrogen halide salt is a hydrochloride salt.

6. The perovskite precursor solution according to any one of 1~5, wherein one or more of the following features are satisfied:
the molecular weight of the additive is less than 1000 Da; optionally, the molecular weight of the additive is less than or equal to 500 Da; further optionally, the molecular weight of the additive is less than or equal to 400 Da; further optionally, the molecular weight of the additive is less than or equal to 350 Da; further optionally, the molecular weight of the additive is less than or equal to 300 Da; and further optionally, the molecular weight of the additive is 150~300 Da;
the number of the carbon atoms in the additive is 2~40; further optionally, the number of the carbon atoms in the additive is 2~25; even further optionally, the number of the carbon atoms in the additive is 2~20; even further optionally, the number of the carbon atoms in the additive is 2~18; even further optionally, the number of the carbon atoms in the additive is 2~15; even further optionally, the number of the carbon atoms in the additive is 2~10; even further optionally, the number of the carbon atoms in the additive is 2~8; and even further optionally, the number of the carbon atoms in the additive is 2~6; and
the number of non-hydrogen atoms in the additive is 8~40; further optionally, the number of non-hydrogen atoms in the additive is 10~30; even further optionally, the number of non-hydrogen atoms in the additive is 10~25; even further optionally, the number of non-hydrogen atoms in the additive is 10~20; and even further optionally, the number of non-hydrogen atoms in the additive is 10~18.

7. The perovskite precursor solution according to any one of claims 1~6, wherein the additive has a structure represented by formula (1);
L(Z₁-R₁₁)ₚ₁·nHZ (1)
in formula (1), p1 is 0 or a positive integer; Z is halogen in the hydrogen halide salt; and n is the number of hydrohalic acid molecules in the hydrogen halide salt;
any one R₁₁ is independently the weakly reducing group and has a valence of 1, optionally any one R₁₁ is independently - NHNH₂, sulfino, phosphinico, hydroxyphenyl or hydroxynaphthyl, and further optionally any one R₁₁ is independently -NHNH₂, sulfino or phosphinico;
when p1 is 0, the additive is a hydrohaliden salt of an organic compound containing at least one of -NHNH-, and -S-S-;
when p1 is a positive integer, L is alkyl, heteroalkyl, cycloalkyl, heterocycloalkyl, aryl, heteroaryl, substituted alkyl, substituted heteroalkyl, substituted cycloalkyl, substituted heterocycloalkyl, substituted aryl, or substituted heteroaryl in a valence of p1; and when L contains a heteroatom, any one heteroatom in L is independently a non-carbon atom or a non-hydrogen atom, and is further independently any one selected from N, S, P, O, and B;
L and all Z₁ contain a total of p2 divalent groups selected from -NH-NH-, and -S-S-, p2 being 0 or a positive integer; and (p1+p2)≥1;
Z₁ is a chemical bond, carbonyl, -CH₂-, -CH(Q₁)- or -(Q₂)C(Q₃)-, wherein Q₁, Q₂, and Q₃ are each independently selected from groups in the group of: alkyl, heteroalkyl, cycloalkyl, heterocycloalkyl, aryl, heteroaryl, substituted alkyl, substituted heteroalkyl, substituted cycloalkyl, substituted heterocycloalkyl, substituted aryl, and substituted heteroaryl; and when Z₁ contains a heteroatom, any one heteroatom in Z₁ is independently a non-carbon, or non-hydrogen atom, and further independently selected from any of N, S, P, O, and B; and optionally, Z₁ is -CH₂-, -CH(Q₁)- or -(Q₂)C(Q₃)-;
any one R₁₁ is independently directly linked to a carbon atom in Z₁ or L; and
in formula (1), alkyl, heteroalkyl, cycloalkyl, heterocycloalkyl, aryl, or heteroaryl in any one of the substituted alkyl, the substituted heteroalkyl, the substituted heterocycloalkyl, the substituted aryl, or the substituted heteroaryl contained in L, Z₁, Q₁, Q₂, or Q₃ is independently substituted with 1 or more groups selected from group G1 of: alkyl, heteroalkyl, cycloalkyl, heterocycloalkyl, aryl, heteroaryl, -CN, hydrazino, sulfino, phosphinico, -OH, -NH₂, -COOH, sulfo, phosphono, boronate, and halogen.

8. The perovskite precursor solution according to claim 7, wherein the additive satisfies any one or more of the following features:
L is a chain structure; optionally, L is a C₂₋₂₀ chain structure; optionally, L is a C₂₋₁₈ chain structure; further optionally, L is a C₂₋₁₅ chain structure; even further optionally, L is a C₂₋₁₂ chain structure; even further optionally, L is a C₂₋₁₀ chain structure; and even further optionally, L is a C₂₋₈ chain structure;
L is a ring-containing structure, and the number of ring atoms in L is 3~25; optionally 3~20; further optionally 3~18; further optionally 5~18; even further optionally 5~15; even further optionally 5~12; even further optionally 5, 6, 10, 11 or 12; and even further optionally 6, 10, 11 or 12;
the number of carbon atoms in L is 2~30; optionally 2~25; further optionally 2~20; even further optionally 2~18; even further optionally 2~15; even further optionally 2~12; even further optionally 2~10; and even further optionally 2~8;
the number of non-hydrogen atoms in L is from 2~35; further optionally the number of non-hydrogen atoms in L is 2~30; even further optionally the number of non-hydrogen atoms in the additive is 2~25; even further optionally the number of non-hydrogen atoms in the additive is 2~20; and even further optionally the number of non-hydrogen atoms in the additive is 2~18;
L is a ring-containing structure, and the ring in L is an aromatic ring, an aliphatic ring, or a combination thereof; further optionally, the ring in L comprises an aromatic ring; even further optionally, the number of carbon atoms in L is 3~40; even further optionally, the number of carbon atoms in L is 3~30; even further optionally, the number of carbon atoms in L is 3~25; even further optionally 3~20; even further optionally 3~18; even further optionally 5~18; even further optionally 5~15; even further optionally 5~12; even further optionally 5, 6, 10, 11, or 12; and even further optionally 6, 10, 11 or 12;
L is a ring-containing structure containing 2~40 carbon atoms; and optionally, the number of carbon atoms in L is 3~30, further optionally 3~25, even further optionally 3~20, further optionally 3~18, and further optionally 4~18;
L is an aliphatic structure; and optionally, the number of carbon atoms in L is 2~2; further optionally 2~20, further optionally 2~18; even further optionally 2~15; further optionally 2~12; even further optionally 2~10, and even further optionally 2~8;
L is an aromatic structure; optionally, L comprises one or more of aryl, heteroaryl, substituted aryl and substituted heteroaryl; further optionally, L comprises one or more of C₆₋₂₀ aryl, C₄₋₂₀ heteroaryl, substituted C₆₋₂₀ aryl and substituted C₄₋₂₀ heteroaryl; and even further optionally, L comprises one or more of C₆₋₁₀ aryl, C₄₋₁₀ heteroaryl, substituted C₆₋₁₀ aryl, and substituted C₄₋₁₀ heteroaryl;
p1 is 0, and the additive is a hydrogen halide salt of a C₂₋₄₀ compound containing at least one of -NHNH-, and -S-S-;
p1 is a positive integer selected from 1~10; optionally p1 is 1, 2, 3, 4 or 5; and optionally p1 is 1 or 2;
p1 is a positive integer, L is C₂₋₁₈ alkyl, C₂₋₁₈ heteroalkyl, C₃₋₂₀ cycloalkyl, C₃₋₂₀ heterocycloalkyl, C₆₋₂₀ aryl, C₃₋₂₀ heteroaryl, substituted C₂₋₁₈ alkyl, substituted C₂₋₁₈ heteroalkyl, substituted C₃₋₂₀ cycloalkyl, substituted C₃₋₂₀ heterocycloalkyl, substituted C₆₋₂₀ aryl or substituted C₃₋₂₀ heteroaryl having a valance of p1; optionally, L is C₂₋₁₂ alkyl, C₂₋₁₂ heteroalkyl, C₃₋₁₂ cycloalkyl, C₃₋₁₂ heterocycloalkyl, C₆₋₁₅ aryl, C₃₋₁₅ heteroaryl, substituted C₂₋₁₂ alkyl, substituted C₂₋₁₂ heteroalkyl, substituted C₃₋₁₂ cycloalkyl, substituted C₃₋₁₂ heterocycloalkyl, substituted C₆₋₁₅ aryl or substituted C₃₋₁₅ heteroaryl having a valance of p1; further optionally, L is C₂₋₁₀ alkyl, C₂₋₁₀ heteroalkyl, C₅₋₁₀ cycloalkyl, C₄₋₁₀ heterocycloalkyl, C₆₋₁₀ aryl, C₃₋₁₀ heteroaryl, substituted C₂₋₁₀ alkyl, substituted C₂₋₁₀ heteroalkyl, substituted C₅₋₁₀ cycloalkyl, substituted C₄₋₁₀ heterocycloalkyl, substituted C₆₋₁₀ aryl or substituted C₃₋₁₀ heteroaryl having a valance of p1; and even further optionally, L is a linear chain structure; wherein when L contains a heteroatom, any one heteroatom in L is independently a non-carbon and non-hydrogen atom, and further independently selected from any one of N, S, P, O, and B;
Q₁, Q₂, and Q₃ are each independently selected from the group of C₁₋₆ alkyl, C₁₋₆ heteroalkyl, C₃₋₈ cycloalkyl, C₃₋₈ heterocycloalkyl, C₆₋₁₂ aryl, C₃₋₁₁ heteroaryl, substituted C₁₋₆ alkyl, substituted C₁-₆ heteroalkyl, substituted C₃₋₈ cycloalkyl, substituted C₃₋₈ heterocycloalkyl, substituted C₆₋₁₂ aryl, and substituted C₃₋₁₁ heteroaryl; optionally, Q₁, Q₂, and Q₃ are each independently selected from the group of: C₁₋₃ alkyl, C₁₋₃ heteroalkyl, C₃₋₆ cycloalkyl, C₃₋₆ heterocycloalkyl, C₆₋₁₀ aryl, C₃₋₉ heteroaryl, substituted C₁₋₃ alkyl, substituted C₁₋₃ heteroalkyl, substituted C₃₋₆ cycloalkyl, substituted C₃₋₆ heterocycloalkyl, substituted C₆₋₁₀ aryl, and substituted C₃₋₉ heteroaryl; and further optionally, Q₁, Q₂, and Q₃ are each independently selected from the group of: methyl, methoxy, cyclopropyl, cyclobutyl, cyclopentyl, cyclohexyl, piperidinyl (optionally 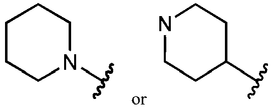 ), piperazinyl (optionally 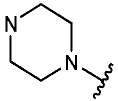 ), phenyl, naphthyl, pyridyl, pyrimidinyl, pyrazinyl, pyridazinyl, and N_{1~6} azanaphthyl, and a substituted version of any one of the aforementioned groups; wherein, n₁, Q₂ or Q₃ is a substituted version of any one of the aforementioned groups, it is independently substituted by 1 or more (optionally 1 or 2~5, further optionally 1, 2 or 3) groups selected from group G1^{a} of: methyl, cyclopentyl, phenyl, benzyl, methylphenyl, -Ph-Ph, N_{1~2} aza heterocycloalkyl (optionally monoaza C₃₋₈ cycloalkyl or diaza C₃₋₈ cycloalkyl, further optionally monoaza cyclobutyl, piperidinyl or piperazinyl, even further optionally monoaza cyclobutyl, and even further optionally 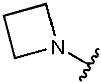 ), -CN, hydrazino, sulfino, phosphinico, -OH, -NH₂, -COOH, sulfo, phosphono, boronate, and halogen (optionally one or more of F, Cl, Br, and I);
the group G1 comprises alkyl (optionally C₁₋₈ alkyl, further optionally C₁₋₆ alkyl, even further optionally C₁₋₄ alkyl, even further optionally C₁₋₃ alkyl, and even further optionally methyl), heteroalkyl (optionally alkoxy or secondary amino, further optionally C₁₋₈ alkoxy or C₁₋₈ alkylamino, even further optionally C₁₋₆ alkoxy or C₁₋₆ alkylamino, even further optionally C₁₋₄ alkoxy or C₁₋₄ alkylamino, even further optionally C₁₋₃ alkoxy or C₁₋₃ alkylamino, even further optionally -NHCH₃ or -NHCH₂CH₃, and even further optionally -NHCH₃), cycloalkyl (optionally C₃₋₈ cycloalkyl, further optionally C₃₋₆ cycloalkyl, and even further optionally cyclopropyl, cyclobutyl, cyclopentyl or cyclohexyl), heterocycloalkyl (optionally 3~8-membered heterocycloalkyl having 1 or 2 N atoms as ring atoms, further optionally 3~6-membered heterocycloalkyl having 1 or 2 N atoms as ring atoms, further optionally 3~4-membered heterocycloalkyl having 1 or 2 N atoms as ring atoms, further optionally piperidinyl, piperazinyl or monoaza cyclobutyl, and further optionally 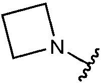 ), aryl (optionally C₆₋₁₂ aryl, further optionally C₆₋₁₀ aryl, and even further optionally phenyl, naphthyl or biphenyl), heteroaryl (6~12-membered aryl containing 1~6 ring nitrogen atoms, further optionally 6~12-membered aryl containing 1~4 ring nitrogen atoms, even further optionally 6~12-membered aryl containing 1~3 ring nitrogen atoms, further optionally 6~12-membered aryl containing 1 or 2 ring nitrogen atoms, even further optionally N₁₋₂ azaphenyl, even further optionally monoazaphenyl or diazazaphenyl, and even further optionally pyridyl, pyrimidyl, pyrazyl or pyridazyl), -CN, hydrazino, sulfino, phosphinico, -OH, - NH₂, -COOH, sulfo, phosphono, boronate, and halogen (optionally one or more of F, Cl, Br and I); and
Z₁ is a chemical bond, -CH₂-, -CH(Q₁)- or -(Q₂)C(Q₃)-.

9. The perovskite precursor solution according to claim 8, wherein the additive satisfies any one or more of the following feature:
L is an aliphatic structure and Z₁ is a chemical bond or carbonyl (Z₁ optionally is a chemical bond);
L is an aromatic structure and Z₁ is not a chemical bond;
L is phenyl, naphthyl, cyclopentyl, cyclohexyl, n-octyl, n-nonyl, n-decyl, n-undecyl, n-dodecyl, n-tridecyl, n-tetradecyl, n-pentadecyl, n-hexadecyl, heptadecyl, n-octadecyl, pyridyl, piperidinyl, piperazinyl, N-methylpiperidinyl, methylphenyl, dimethylphenyl, biphenyl, naphthyl-substituted phenyl, aminophenyl, C₂₋₁₈ alkyl with 1 or more sulfo substituents (optionally ethyl with a monosulfo substituent, and further optionally 2-sulfoethyl), C₂₋₁₈ alkyl substituted with 1 or more NH₂ (optionally C₂₋₁₈ alkyl substituted with one -NH₂, further optionally aminobutyl, and even further optionally 4-aminobutyl), C₂₋₁₈ alkyl substituted with 1 or more -(O=)P(OH)₂ (optionally C₂₋₁₂ alkyl substituted with 1 -(O=)P(OH)₂, further optionally phosphonopropyl, and even further optionally 3-phosphonopropyl), C₂₋₁₈ alkyl substituted by 1 or more -(O=)PR₀₂(OH) (optionally C₂₋₁₂ alkyl substituted with 1 - (O=)PR₀₂(OH), optionally C₂₋₈alkyl substituted with 1 -(O=)PR₀₂(OH), even further optionally (CH₃)(OH)(R₀₂)P(=O)-C_{2~8} alkylene -, even further optionally (CH₃)(OH)(R₀₂)P(=O)-(CH₂)_{2~8}-, and even further optionally (CH₃)(OH)(R₀₂)P(=O)-(CH₂)₃-), and R₀₂ is alkyl (optionally C₁₋₆ alkyl, further optionally C₁₋₃ alkyl, and even further optionally methyl), C₂₋₁₈ alkyl having 1 or more -(O=)PR₀₂(OH) and 1 or more NH₂- substituents (optionally C₂₋₁₈ alkyl having 1 -(O=)PR₀₂(OH) and 1 NH₂ substituent, further optionally C₂₋₁₂ alkyl having 1 -(O=)PR₀₂(OH) and 1 NH₂ substituent, even further optionally C₂₋₈ alkyl having 1 -(O=)PR₀₂(OH) and 1 NH₂ substituent, and even further optionally (CH₃)(OH)(R₀₂)P(=O)-CH₂CH₂CH(NH₂)-), sulfophenyl, carboxyphenyl, phosphonophenyl, and boratophenyl;
Z₁ is any one of divalent groups of: methylene, -CH(CH₃)-, -CH(benzyl)-, -CH(cyclopentyl)-, -CH(-Ph-Ph)-, -CH(phenyl), - CH(-Ph-CH₃)-, and 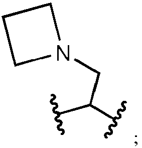
the group G1 is group G2 comprising groups of: alkyl (optionally C₁₋₈ alkyl, further optionally C₁₋₆ alkyl, even further optionally C₁₋₄ alkyl, even further optionally C₁₋₃ alkyl, and even further optionally methyl), heteroalkyl (optionally alkoxy or secondary amino, further optionally C₁₋₈ alkoxy or C₁₋₈ alkylamino, even further optionally C₁₋₆ alkoxy or C₁₋₆ alkylamino, even further optionally C₁₋₄ alkoxy or C₁₋₄ alkylamino, even further optionally C₁₋₃ alkoxy or C₁₋₃ alkylamino, even further optionally -NHCH₃ or -NHCH₂CH₃, and even further optionally -NHCH₃), cycloalkyl (optionally C₃₋₈ cycloalkyl, further optionally C₃₋₆ cycloalkyl, and even further optionally cyclopropyl, cyclobutyl, cyclopentyl or cyclohexyl), heterocycloalkyl (optionally 3~8-membered heterocycloalkyl having 1 or 2 N atoms as ring atoms, further optionally 3~6-membered heterocycloalkyl having 1 or 2 N atoms as ring atoms, even further optionally 3~4-membered heterocycloalkyl having 1 or 2 N atoms as ring atoms, still further optionally piperidinyl, piperazinyl or monoazetidinyl, and further optionally 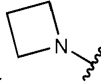 ), aryl (optionally C₆₋₁₂ aryl, further optionally C₆₋₁₀ aryl, and still optionally phenyl, naphthyl or biphenyl), and heteroaryl (6~12-membered aryl containing 1~6 ring nitrogen atoms, further optionally 6~12-membered aryl containing 1~4 ring nitrogen atoms, even further optionally 6~12-membered aryl containing 1~3 ring nitrogen atoms, even further optionally 6~12-membered aryl containing 1 or 2 ring nitrogen atoms, even further optionally N₁₋₂ azaphenyl, even further optionally monoazaphenyl or diazazaphenyl, and even further optionally pyridyl, pyrimidyl, pyrazyl or pyridazyl); optionally, the group G1 is the group G1^{b} comprising groups of: methyl, cyclopentyl, cyclopentylmethyl, phenyl, benzyl, biphenyl, methylphenyl, piperidinyl (optionally 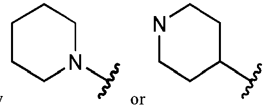 ), piperazinyl (optionally 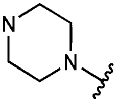 ), and monoaza cyclobutyl (optionally 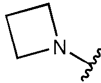 ); and optionally, the group G1 comprises methyl, cyclopentyl, phenyl, benzyl, biphenyl, methylphenyl, piperidinyl (optionally 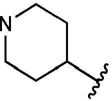 ), and monoaza cyclobutyl (optionally 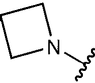 ).

10. The perovskite precursor solution according to any one of claims 7~9, wherein p1 is a positive integer, R₁₁ is -NHNH₂, and optionally, at least one Z₁ is not a chemical bond.

11. The perovskite precursor solution according to any one of claims 7~10, wherein p1 is a positive integer, L is an aromatic group, Z₁ is directly linked to the aromatic ring in L, and corresponding R₁₁ is -NHNH₂, and Z₁ is not a chemical bond; and optionally, R₁₁ is -CH₂-, -CH(Q₁)- or -(Q₂)C(Q₃)-; and
optionally, Q₁, Q₂, and Q₃ are each independently selected from the group of: alkyl, aralkyl, heteroaralkyl, substituted alkyl, substituted aralkyl, and substituted heteroaralkyl, wherein Q₁, Q₂, or Q₃, when is any one of the substituted forms described above, is independently substituted with 1 or more groups selected from the group G1.

12. The perovskite precursor solution according to claim 11, wherein L is aryl, heteroaryl, substituted aryl or substituted heteroaryl; and
optionally, L is any one of C₆₋₂₀ aryl, C₄₋₂₀ heteroaryl, substituted C₆₋₂₀ aryl, and substituted C₄₋₂₀ heteroaryl; and further optionally, L is any one of C₆₋₁₀ aryl, C₄₋₁₀ heteroaryl, substituted C₆₋₁₀ aryl, and substituted C₄₋₁₀ heteroaryl;
wherein L, when it is any one of the above substituted forms, is independently substituted with 1 or more groups selected from the group G1.

13. The perovskite precursor solution according to any one of claims 7~12, wherein the additive has a structure of any one of formula (11), formula (12), formula (13), formula (14), and formula (15):
Ar(-Z₂₁-R₁₁)ₚ₁·nHCl (11)
in formula (11), p1 is a positive integer, and Z₂₁ is -CH₂-, -CH(Q₁)- or -(Q₂)C(Q₃)-;
Ar(-R₁₁)ₚ₁·nHCl (12)
in formula (12), p1 is a positive integer, and R₁₁ is sulfino, and phosphinico; 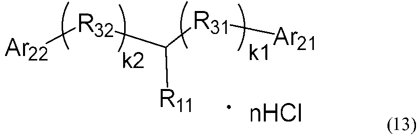
in formula (13), k1, and k2 are each independently 0 or 1; R₃₁, and R₃₂ are each independently alkylene; optionally, R₃₁, and R₃₂ are each independently C₂₋₂₀ alkylene, further optionally, R₃₁, and R₃₂ are each independently C₂₋₁₆ alkylene, even further optionally, R31, and R₃₂ are each independently C₂₋₁₂ alkylene, even further optionally, R₃₁, and R₃₂ are each independently C₂₋₁₀ alkylene, even further optionally, R₃₁, and R₃₂ are each independently C₂₋₈ alkylene, even further optionally, R₃₁, and R₃₂ are each independently C₂₋₆ alkylene, and even further optionally, R₃₁, and R₃₂ are each independently C₂₋₄ alkylene; 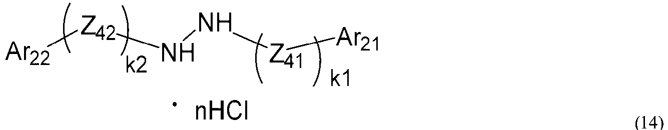 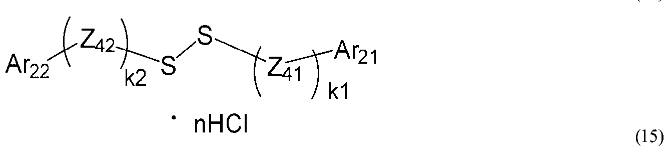
in formula (14) and formula (15), k1, and k2 are each independently 0 or 1; Z₄₁, and Z₄₂ are each independently alkylene; optionally, Z₄₁, and Z₄₂ are each independently C₂₋₁₅ alkylene, further optionally, Z₄₁, and Z₄₂ are each independently C₁₋₁₂ alkylene, even further optionally, Z₄₁, and Z₄₂ are each independently C₁₋₁₀ alkylene, even further optionally, Z₄₁, and Z₄₂ are each independently C₁₋₈ alkylene, even further optionally, Z₄₁, and Z₄₂ are each independently C₁₋₆ alkylene, even further optionally, Z₄₁, and Z₄₂ are each independently C₁₋₄ alkylene, and even further optionally, Z₄₁, and Z₄₂ are each independently methylene, ethylene, propylene or butylene;
wherein Ar, Ar₂₁, and Ar₂₂ are each independently an aromatic group; optionally the number of ring atoms of Ar, Ar₂₁, and Ar₂₂ are each independently 5~15, further optionally 5~12, further optionally 5~10 or 12, and further optionally 5, 6, 10 or 12; and further optionally Ar, Ar₂₁, and Ar₂₂ are each independently aryl, heteroaryl, substituted aryl or substituted heteroaryl, wherein aryl or heteroaryl in the substituted aryl or the substituted heteroaryl is independently substituted with 1 or more groups selected from the group G1; and when containing a heteroatom, any one heteroatom contained in any one of Ar, Ar₂₁, and Ar₂₂ is independently a non-carbon, or non-hydrogen atom, and further independently any one selected from N, S, P, O, and B.

14. The perovskite precursor solution according to any one of claims 1~13, wherein the additive contains a second functional group W₁₁, and W₁₁ is any one selected from the group of: -COOH, sulfo, phosphono, and boronate; and
in one molecule of the additive, the number j of the second functional group W₁₁ is 1 or more; optionally j is 1, 2 or 3; and further optionally j is 1 or 2.

15. The perovskite precursor solution according to claim 14, wherein the additive has any one of the following structures:
(W₁₁)ⱼL₀(R₀₂)ₘ₁·nHCl (2)
in formula (2), j is a positive integer, and m1 is a positive integer; any one R₀₂ is independently the weak reducing group; L₀ is hydrocarbyl or heterohydrocarbyl with a valence of m1+j, the heterohydrocarbyl containing 0, 1, or more substituents selected from the group of: -CN, -OH, halogen (optionally one or more of F, Cl, Br, and I), -NH₂, -SH, -CF₃, and -COOH; and optionally, any one R₀₂ is independently selected from the group of: a -NHNH-containing group, -NHNH₂, sulfino, and phosphinico;
(W₁₁)ⱼ₁L₀₁(Z₁-R₁₁)ₚ₁·nHCl (21)
in formula (21), p1, and j1 are each independently a positive integer, any one Z₁, and any one R₁₁ are each independently as defined in any one of claims 7~14; L₀₁(Z₁₋)ₚ₁ contains p2 the divalent weakly reducing groups, and j2 W₁₁, wherein p2, and j2 are each independently 0 or a positive integer; and L₀₁ is hydrocarbyl or heterohydrocarbyl having a valence of p1+j1, the heterohydrocarbyl containing 0, 1 or more substituents selected from the group of: -CN, -OH, and halogen (optionally one or more of F, Cl, Br and I), - NH₂, -SH, -CF₃, and -COOH;
(W₁₁)ⱼ₁Ar₀₁(-Z₃₁-R₁₁)ₚ₁·nHCl (22)
in formula (22), p1, j1, any one R₁₁, and any one W₁₁ are each independently as defined in formula (21); and Ar₀₁ is a divalent aromatic group; wherein the divalent aromatic group contains 0, 1, or more substituents selected from the group of: -CN, -OH, -NH₂, - SH, -CF₃, -COOH, and halogen; Z₃₁ is -CH₂-, -CH(Q₁)- or -(Q₂)C(Q₃)-; and Q₁, Q₂, and Q₃ are each independently a group selected from group G3 of: alkyl (optionally C₁₋₆ alkyl, further optionally C₁₋₃ alkyl, and even further optionally methyl), and heteroalkyl (optionally alkoxy or alkylamino, further optionally C₁₋₆ alkoxy or C₁₋₆ alkylamino, even further optionally C₁₋₃ alkoxy or C₁₋₃ alkylamino, even further optionally methoxy, ethoxy, -NHCH₃ or -NHCH₂CH₃, even further optionally -NHCH₃ or -NHCH₂CH₃, and even further optionally -NHCH₃); wherein the heterohydrocarbyl contains 0, 1 or more substituents selected from the group of: -CN, - OH, halogen (optionally one or more of F, Cl, Br and I), -NH₂, -SH, -CF₃, and -COOH; 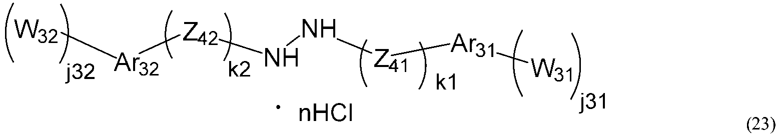 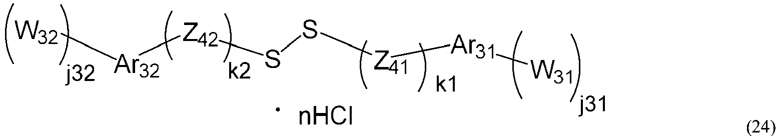
in formula (23), and formula (24), k1, k2, Z₄₁, and Z₄₂ are each independently as defined in formula (14); j31 and j32 are each independently 0 or a positive integer, with (j31 + j32) ≥1; and W₃₁, and W₃₂ are each independently any one group selected from the group of: -COOH, sulfo, phosphono, boronate, -NH₂, and -SH;
wherein Ar₃₁, and A₃₂ are each independently a divalent aromatic group; wherein the divalent aromatic group contains 0, 1 or more substituents selected from the group of: -CN, -OH, amino, mercapto, and halogen (optionally one or more of F, Cl, Br, and I).

16. The perovskite precursor solution according to any one of 1~15, wherein the additive comprises any one or more of the following compounds: 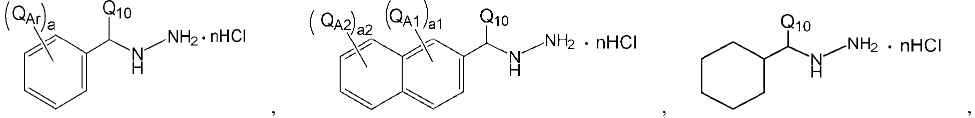 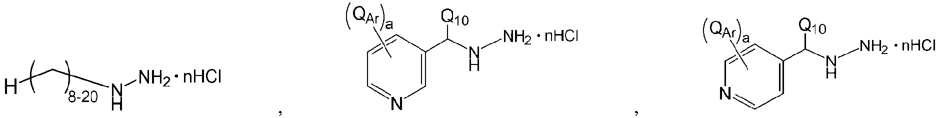 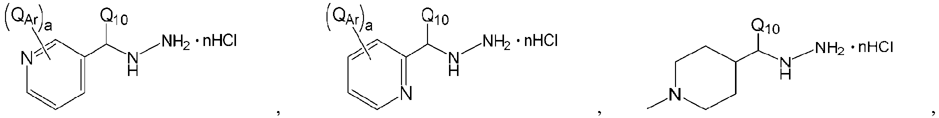 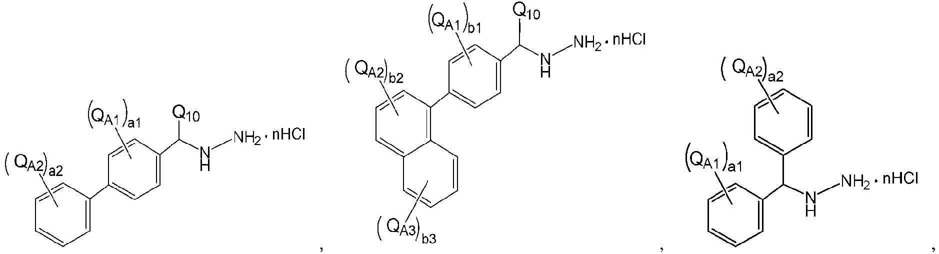 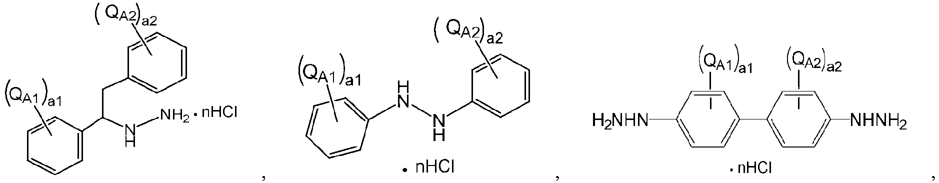 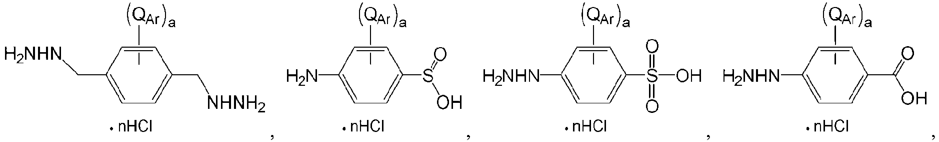 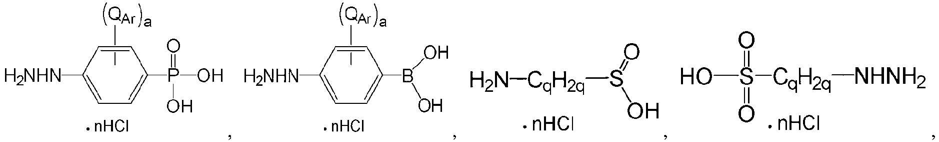 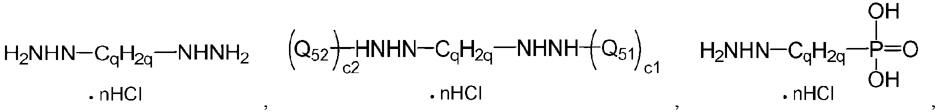 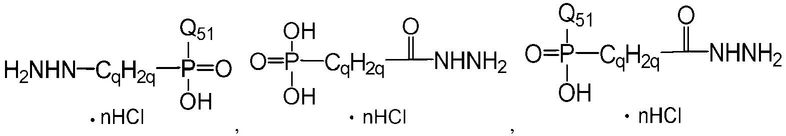 , and a case where any one of the aforementioned compound salts is substituted with a substituent selected from group G1 according to any one of claims 7~9;
wherein n is a positive integer, optionally, n is an integer selected from 1~10; further optionally, n is 1, 2, 3, 4 or 5; and further optionally, n is 1 or 2;
Q_{Ar} is any one group selected from the group G1; and a is 0 or a positive integer (optionally is 0, 1 or 2);
Q₁₀ is H or Q₁; optionally, Q₁₀ is H; and further optionally, Q₁₀ is Qi;
a1 and a2 are each 0 or a positive integer, with a1+a2≥1;
b1, b2, and b3 are each 0 or a positive integer, with b1 + b2 + b2 ≥ 1, optionally, b2 + b3 ≥1, and further optionally b1 = 0;
Q_{A1}, Q_{A2}, and Q_{A3} are each independently Q_{Ar};
q is an integer selected from 2~20, optionally an integer of 2~18, further optionally an integer of 2~16, even further optionally an integer of 2~12, even further optionally an integer of 2~10, even further optionally an integer of 2~8, and even further optionally an integer of 2, 3, 4, 5, 6 or 7;
c1 and c2 are each independently 0 or 1, optionally c1+c2≥1; and
Q₅₁ and Q₅₂ are each independently alkyl (optionally C₁₋₆ alkyl, further optionally C₁₋₃ alkyl, and even further optionally methyl).

17. The perovskite precursor solution according to 16, wherein the additive comprises any one or more of the following compounds: 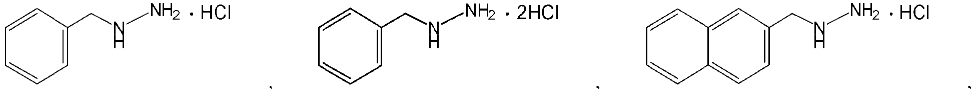 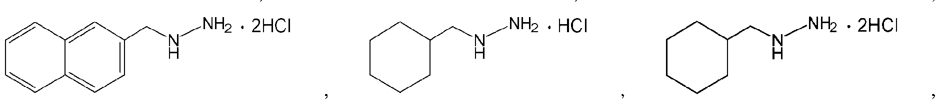 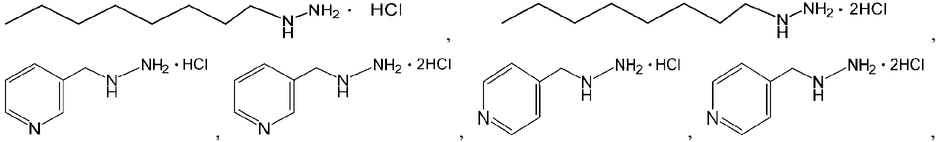 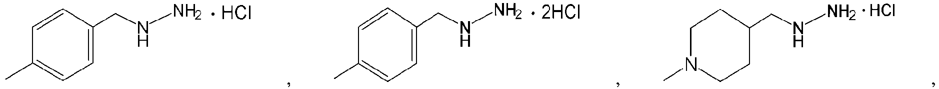 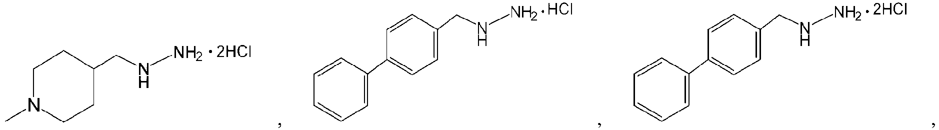 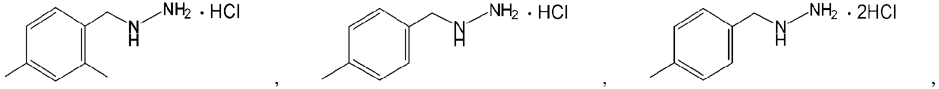 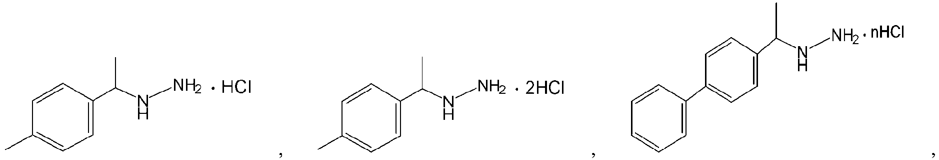 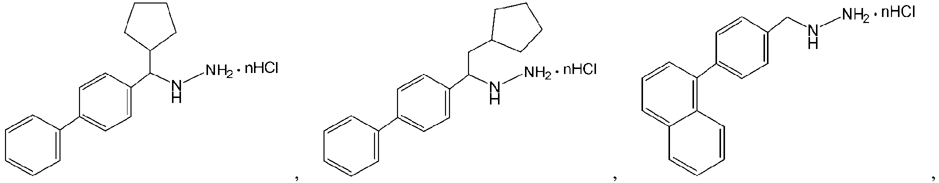 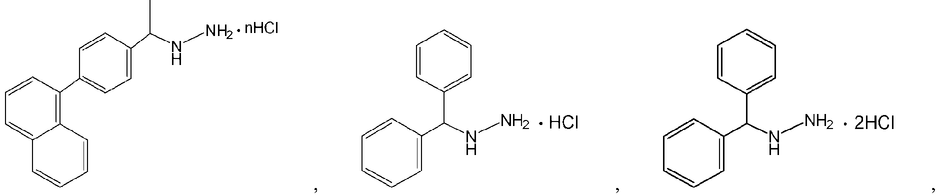 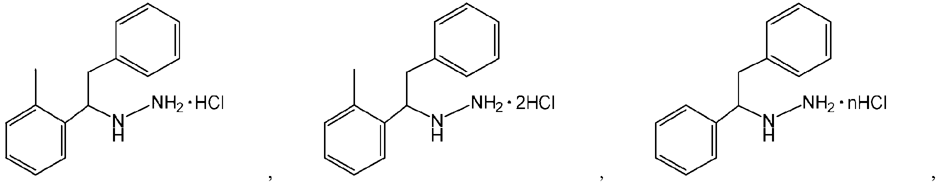 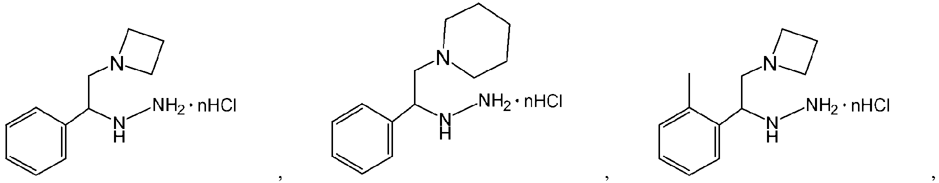 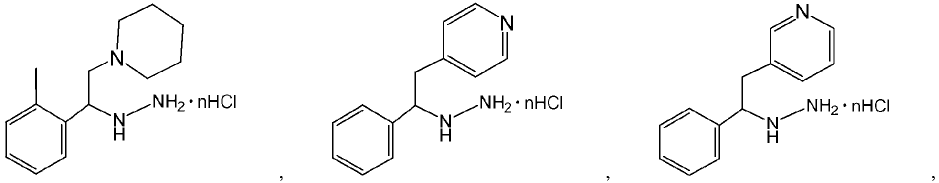 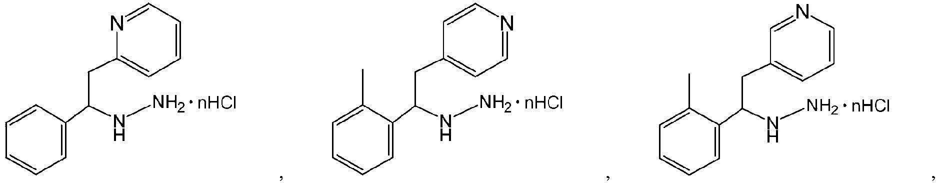 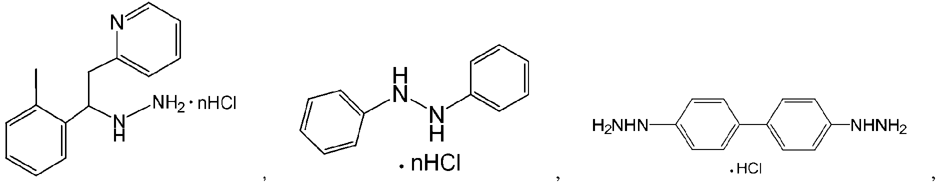 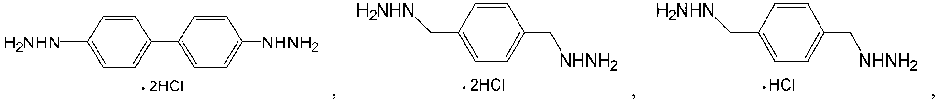 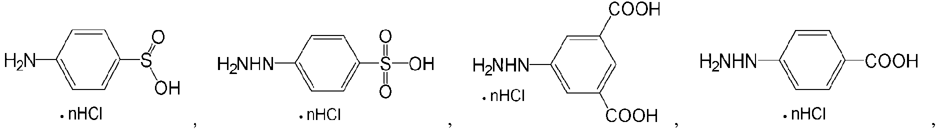 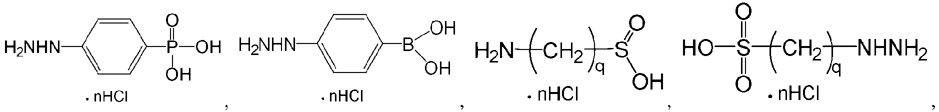 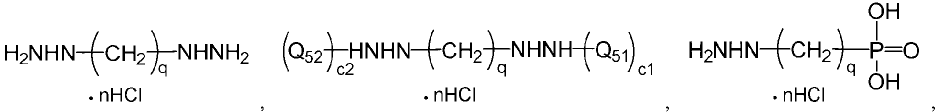 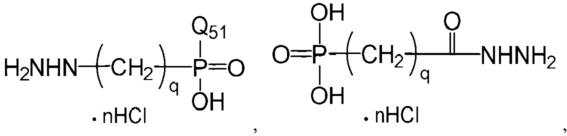 and a case where any one of the aforementioned compound salts is substituted with a substituent selected from the group G1;
optionally, n is 1, 2, or 2;
optionally, n is 1; and
optionally, n is 2.

18. The perovskite precursor solution according to any one of claims 1~17, wherein the perovskite precursor material comprises a perovskite-type metal halide represented by chemical formula ABX₃, where A is a monovalent cation, B is a divalent cation, and X is a monovalent anion;
optionally, A comprises one or more of Cs⁺, K⁺, Rb⁺, a monovalent amine cation, and a monovalent amidinate cation;
optionally, B comprises one or more of Pb²⁺, Sn²⁺, Fe²⁺, Mn²⁺, Ni²⁺, Ge²⁺, Co²⁺, and Sb²⁺; and
optionally, X comprises one or more of I⁻, Br⁻, and Cl⁻.

19. The perovskite precursor solution according to claim 18, wherein the weight percent of the additive relative to B element in the perovskite precursor material is 0.01%~15%;
optionally, the weight percent of the additive relative to B element in the perovskite precursor material is 0.01%~10%; and
optionally, the weight percent of the additive relative to B element in the perovskite precursor material is 0.1%~8%.

20. The perovskite precursor solution according to claim 18 or 19, wherein the molar ratio of the additive to B in the perovskite-type metal halide is 0.001%~15%;
optionally, the molar ratio of the additive to B in the perovskite-type metal halide is 0.01~15%; and
optionally, the molar ratio of the additive to B in the perovskite-type metal halide is 2~6.5%.

21. The perovskite precursor solution according to any one of claims 18~20, wherein the molar ratio of the weak reducing group to the B element in the perovskite-type metal halide is 0.01~15%; and
optionally, the molar ratio of the weak reducing group to the B element in the perovskite-type metal halide is 1~8%.

22. The perovskite precursor solution according to any one of 1~21, wherein the perovskite precursor solution further contains an oxidized ion of a multi-valent metal element M.

23. The perovskite precursor solution according to claim 22, wherein the multi-valent metal element M comprises one or more elements of lanthanoid elements, Fe, Co, Ni, Ti, Cr, Mn, Y, Rh, and Bi;
optionally, the lanthanide elements comprise one or more elements of Ce, Pr, Sm, Eu, Tb, and Yb;
optionally, the oxidation-state ion of the multi-valent metal element M comprises one or more of Ce⁴⁺, Pr⁴⁺, Sm³⁺, Eu³⁺, Tb⁴⁺, Yb³⁺, Fe³⁺, Co³⁺, Ni³⁺, Ti⁴⁺, Cr³⁺, Mn⁴⁺, Y³⁺and Rh⁴⁺; and
optionally, the oxidation-state ion of the multi-valent metal element M is derived from an organic salt of the metal element M, and further optionally, the organic salt includes one or more of an acetylacetonate salt, a sulfonate salt, and a sulfate salt.

24. The perovskite precursor solution according to claim 22 or 23, wherein the perovskite precursor solution contains one or more ion pairs of Ce³⁺-Ce⁴⁺, Pr⁴⁺-Pr³⁺, Sm³⁺-Sm²⁺, Eu³⁺-Eu²⁺, Tb⁴⁺-Tb³⁺, Yb³⁺-Yb²⁺, Fe³⁺-Fe²⁺, Co³⁺-Co²⁺, Ni³⁺-Ni²⁺, Ti⁴⁺-Ti^{X+}, Cr³⁺-Cr²⁺, Mn⁴⁺-Mn²⁺, Y³⁺-Y²⁺, Rh⁴⁺-Rh²⁺, and Bi³⁺-Bi²⁺; wherein Ti^{X+} in ion pair ⁴⁺-Ti^{X+} has a positive charge valence less than 4.

25. The perovskite precursor solution according to any one of claims 1~24, wherein the perovskite precursor material comprises the perovskite-type metal halide according to claim 15;
the molar ratio of the multi-valent metal element M to the B element in the perovskite-type metal halide is 0.001%~15%; and
optionally, the molar ratio of the multi-valent metal element M to the B element in the perovskite-type metal halide is 0.01%~15%.

26. A perovskite thin film prepared by using the perovskite precursor solution according to any one of claims 1~25.

27. A perovskite cell comprising the perovskite thin film according to claim 26.

28. The perovskite cell according to claim 27, wherein the perovskite cell is an inversed p-i-n cell or a normal n-i-p cell.

29. An electric device, comprising the perovskite cell according to claim 27 or 28.
